# EUROPÄISCHE PATENTSCHRIFT

(11) **EP 2 417 242 B1**
(45) Veröffentlichungstag und Bekanntmachung des Hinweises auf die Patenterteilung: **04.07.2018**
(21) Anmeldenummer: 10714611.0
(22) Anmeldetag: 08.04.2010
(51) Int. Cl.: C12M 1/00, A01G 9/18

(54) **VORRICHTUNG UND VERFAHREN ZUR BESEITIGUNG VON CO2, SOWIE VERWENDUNGEN HIERFÜR**
DEVICE AND METHOD FOR REMOVING CO2, AND USES THEREFOR
DISPOSITIF ET PROCÉDÉ D'ÉLIMINATION DE CO2, ET UTILISATIONS

(30) Priorität: 09.04.2009 DE 102009017046
(43) Veröffentlichungstag der Anmeldung: 15.02.2012
(73) Patentinhaber: Siemens Aktiengesellschaft, 80333 München (DE)
(72) Erfinder: HAJE, Detlef, 02828 Görlitz (DE)
(86) Internationale Anmeldenummer: PCT/EP2010/054618
(87) Internationale Veröffentlichungsnummer: WO 2010/115944

(56) Entgegenhaltungen:
- EP-A1- 1 559 311
- WO-A1-97/12511
- WO-A2-2007/143653
- WO-A2-2008/008262
- WO-A2-2008/134010
- US-A- 5 713 154
- US-A1- 2005 260 553
- DATABASE EPODOC EUROPEAN PATENT OFFICE, THE HAGUE, NL; 16. Dezember 1989 (1989-12-16), Gromas AB: "Plant conveyor for stocking greenhouse..." XP002598863 -& SE 8 802 245 A (GROMAS AB) 16. Dezember 1989 (1989-12-16)

## Beschreibung

Die vorliegende Erfindung betrifft eine Verbrennungsanlage umfassend einen Brennraum und eine Vorrichtung zur Beseitigung von CO2 (Kohlenstoffdioxid) nach dem Oberbegriff des Anspruchs 1.

Ferner betrifft die Erfindung ein Verfahren zum Betreiben einer derartigen Verbrennungsanlage nach dem Oberbegriff des Anspruchs 4.

Gegenwärtig werden weltweit große Anstrengungen zur Begrenzung der Konzentration von so genannten Treibhausgasen und insbesondere von CO2 in der Erdatmosphäre unternommen. Da die Verbrennung fossiler Brennstoffe wie Kohle oder Öl, beispielsweise in Kraftwerken, die CO2-Konzentration in der Atmosphäre erhöht, wird gegenwärtig an technischen Möglichkeiten für eine Beseitigung von CO2 geforscht.

Der Stand der Technik zur Beseitigung von CO2 besteht in der Speicherung von CO2 in großen unterirdischen Formationen wie Aquiferen oder Kavernen (so genanntes "Carbon Capture and Storage", CCS). Hierfür kommt in Betracht, im Betrieb von Kraftwerken entstehendes CO2 direkt aus dem Abgas abzuscheiden und einer langzeitigen unterirdischen Speicherung zuzuführen, um einer weiteren Erhöhung der atmosphärischen CO2-Konzentration entgegenzuwirken.

Alternativ zur Abscheidung und Speicherung von CO2 (CCS) können CO2-Emissionsrechte mit Hilfe des so genannten "Clean Development Mechanism" (CDM) durch Emissionsreduzierung in Entwicklungsländern und durch Transfer von umweltfreundlicher Technologie in Entwicklungsländer erworben werden. Weiterhin wird unter CDM die Schaffung von CO2-Senken (z. B. Aufforstung) durch Emissionsrechte vergütet.

Als natürliche CO2-Senke kommt heute Photosynthese der Pflanzen auf oder am Festland (65% der globalen CO2-Beseitigung, gemessen in Kohlenstoff in gebildeter Trockenmasse) sowie in den Ozeanen (35%) zum Tragen. Überlegungen, die Intensität der CO2-Umsetzung in den Ozeanen zu verbessern, weisen den Nachteil einer unbegrenzten Ausbreitungsmöglichkeit der eingesetzten Organismen und einer begrenzten Beeinflussbarkeit der photosyntheserelevanten Bedingungen auf.

Außerdem wurden im Stand der Technik bereits Überlegungen in Richtung einer technischen Beseitigung von CO2 mit Hilfe der Photosynthese angestellt. Nicht bekannt sind jedoch Vorrichtungen und Verfahren, die effizient für einen Einsatz in großem Maßstab ausgerichtet sind. Wünschenswert wären unkomplizierte bzw. kostenoptimierte Lösungen, etwa hinsichtlich der Konstruktion und Herstellbarkeit geeigneter Vorrichtungen.

Schließlich stehen der technischen CO2-Beseitigung im großen Maßstab bislang auch hohe Eigenbedarfs-Energieverbräuche einer Anwendung entgegen (z. B. signifikante Kompression von Gasen, signifikante Einbringung von Kühlleistung, massive Umwälzleistungen, aktive (technische) CO2-Abscheidung wie derzeit als "Post Combustion"-CO2-Abscheidung bekannt oder CO2-Einspeicherung unter Druckerhöhung oder Verflüssigung).

Aus der US 5,713,154 ist eine Vorrichtung zum Heizen eines Gewächshauses unter Einsatz einer mit Kohlenwasserstoff-Brennstoff betriebenen Heizeinrichtung bekannt. Von der Heizeinrichtung erzeugtes Abgas wird hierbei gesammelt, um sodann darin enthaltenes CO2 mittels einer Verteileinrichtung in gesteuerter Weise zu bestimmten Zeitperioden in eine Atmosphäre des Gewächshauses abzugeben.

Aus der WO 97/12511 ist ein System zur Aufrechterhaltung einer günstigen Atmosphäre eines Gewächshauses bekannt, wobei mittels eines mit Kohlenwasserstoff-Brennstoff betriebenen Brenners Abgas enthaltend Wasserdampf und CO2 erzeugt wird, das nach Kühlung desselben in das Gewächshaus geleitet wird.

Aus der EP 1 559 311 A1 ist es bekannt, bei der Kultivierung von Pflanzen in einem Gewächshaus ein Schienensystem mit verfahrbaren Trägern für die Pflanzen vorzusehen.

Aus der SE 8 802 245 A ist es bekannt, zum Einbringen und Ausbringen von Pflanzen in ein Gewächshaus hinein bzw. aus diesem heraus eine Transportmatte einzusetzen, welche mit darauf angeordneten Pflanzen in das Gewächshaus hinein bzw. aus diesem heraus gezogen werden kann.

Aus der WO 2008/134010 A2 ist ein umschlossener Photobioreaktor bekannt, der dazu ausgebildet ist, auf einem Gewässer zu schwimmen und der eine phototrope Organismen aufweisende Flüssigkeit beinhaltet.

Aus den Veröffentlichungen WO 2008/008262 A2 und US 2005/0260553 A1 sind Verbrennungsanlagen und jeweilige Betriebsverfahren hierfür bekannt, bei denen Abgas eines Verbrennungsprozesses einer auf Photosynthese basierenden CO2-Beseitigungsvorrichtung zugeführt wird, um den CO2-Anteil des Abgases zu verringern.

Eine Verbrennungsanlage nach dem Oberbegriff des Anspruchs 1 und ein Verfahren nach dem Oberbegriff des Anspruchs 4 sind aus der WO 2007/143653 A2 bekannt. Bei diesem Stand der Technik sind der Brennraum und der photosynthetisch aktive Bereich in einen Gaskreislauf eingebunden, wobei der Brennraum mit einem CO2-armen Gasgemisch bestehend aus Frischluft und Sauerstoff versorgt wird, wobei der Sauerstoff aus einem Abgas eines Bioreaktors gewonnen wird.

Nachteilig ist bei den bekannten Ansätzen zur technischen Beseitigung von CO2 insbesondere der damit einhergehende Kostenaufwand sowie dessen gravierender Einfluss auf die Effizienz.

Dies gilt insbesondere auch für die in der WO 2007/143653 A2 beschriebene Verbrennungsanlage, bei welcher ein Aufwand für die Gewinnung von Sauerstoff aus dem Bioreaktorabgas entsteht und die Effizienz des Bioreaktors aufgrund des vergleichsweise hohen Sauerstoffanteils in der Atmosphäre des Bioreaktors eher mäßig ist.

Es ist daher eine Aufgabe der vorliegenden Erfindung, eine Verbrennungsanlage der eingangs genannten Art sowie ein Betriebsverfahren hierfür anzugeben, mit denen in einfacher Weise eine hohe Effizienz erzielbar ist.

Diese Aufgabe wird gemäß der Erfindung durch eine Verbrennungsanlage nach Anspruch 1 bzw. ein Betriebsverfahren nach Anspruch 4 gelöst. Die abhängigen Ansprüche betreffen vorteilhafte Weiterbildungen der Erfindung.

Der Ausgestaltung der bei der Erfindung eingesetzten CO2-Beseitigungsvorrichtung liegt die Idee zugrunde, dass die Beseitigung von CO2 effektiv durch eine technisch, insbesondere großtechnisch unterstützte Anwendung der Photosynthese erfolgen kann. Hierbei können vorteilhaft die biologischen Prozesse der Photosynthese durch technische Einrichtungen positiv beeinflusst werden, so dass Effektivität und Ressourcenverbrauch (Landverbrauch) optimiert werden können.

Die CO2-Beseitigungsvorrichtung kann beispielsweise bevorzugt in geographischen Gegenden bzw. Breiten mit hoher Sonneneinstrahlung (geographische Breite z. B. im Bereich von -40° bis +40°, insbesondere -30° bis +30°) und/oder niedrigen Landkosten (z. B. Wüsten wie "Sahara" etc.) installiert und betrieben werden. Insbesondere für solche ausgewählten geographischen Gegenden kommt in Betracht, die Vorrichtung als CO2-Senke in großtechnischem Maßstab zu betreiben. Der Begriff "großtechnisch" soll hierbei insbesondere eine flächenmäßige Ausdehnung der Vorrichtung von mehr als 10¹ km², insbesondere mehr als 10² km², oder sogar mehr als 10³ km² bezeichnen. Die Höhe der Vorrichtung kann z. B. im Bereich zwischen 10 cm und einigen Metern liegen, z. B. 0,4 bis 1,5 m betragen.

Die Größe der erforderlichen photosynthetisch aktiven Fläche der Vorrichtung macht eine optimale Ausnutzung der Gesamtfläche der Vorrichtung notwendig. Vorteilhaft wird deshalb bevorzugt die weitgehend gesamte Fläche der Vorrichtung durch photosynthetisch aktive Bereiche bedeckt bzw. ausgebildet, z. B. mehr als 80% der Fläche, weiter bevorzugt mehr als 90% der Fläche.

Weiterhin kann z. B. durch eine im Wesentlichen flächig erstreckte Gestaltung der Vorrichtung eine signifikante Kostenreduzierung erreicht werden, etwa im Vergleich zu denkbaren Lösungen mit kompliziert geformten oder in der Herstellung kostenintensiven Vorrichtungskomponenten wie z. B. aufwändige Wandungen (wie etwa vielfache rohrförmige Begrenzungen von bekannten "Bioreaktoren").

Prinzipiell sind sämtliche Arten von photosynthesetreibenden Organismen für die Erfindung verwendbar, so dass im konkreten Anwendungsfall jeweils eine oder mehrere besonders geeignete Organismen ausgewählt werden können. Dies erfolgt zweckmäßigerweise in Abhängigkeit von den konstruktiven Details der betreffenden Vorrichtung und/oder den geographischen Gegebenheiten des betreffenden Verwendungsortes. Bei den Organismen kann es sich insbesondere um alle Arten von Landpflanzen (z. B. Bäume, Sträucher, Gräser, Bodenpflanzen, Rankpflanzen etc.), Wasserpflanzen (z. B. freischwimmende Wasserpflanzen, am Boden haftende Wasserpflanzen, Uferpflanzen etc.), Algen, Bakterien etc. handeln. Wesentlich ist, dass der betreffende Organismus eine photosynthetische Umsetzung von CO2 betreibt.

Für die Anwendung in einem wässrigen Milieu hat sich z. B. die Verwendung von einzelligen, im Wasser schwebenden Organismen als überaus vorteilhaft erwiesen, da diese einen intensiven Stoffaustausch mit dem Wasser besitzen und der technisch bereitgestellten Unterstützung der Photosynthese gut folgen können. Photosynthesetreibende Algen oder Cyanobakterien sind somit besonders gut für die Anwendung geeignet. Zum Beispiel können Algen der Art Chlorella minutissima verwendet werden, die unter mediterranen Bedingungen unter stark erhöhten CO2-Konzentrationen eine Vervielfachung ihres Wachstums / ihrer Vermehrung zeigen. In Versuchen ist die Pflanzenmasse unter stark erhöhten CO2-Konzentrationen um einen Faktor von bis zu 4,1 (!) schneller als bei üblichen CO2-Konzentrationen angewachsen. Bei einer weitergehenden Optimierung (optimale Ausgangskonzentration der eingesetzten Algen in g / Liter aktiven Einrichtungsvolumens) sind sogar Wachstumsraten in der Größenordnung des Neun- bis Zehnfachen des natürlichen Wachstums erreichbar.

In einer Ausführungsform ist vorgesehen, dass die Vorrichtung größtenteils, insbesondere im Wesentlichen ausschließlich mit einer Art von Organismus ("Monokultur") betrieben wird. Dies besitzt vor allem Vorteile hinsichtlich der Schaffung günstiger Umgebungsbedingungen für die in der Umhüllung untergebrachten Organismen sowie einer technisch einfachen Abfuhr von Produkten der CO2-Umsetzung.

In einer anderen Ausführungsform ist vorgesehen, dass wenigstens 2 (oder noch mehr) verschiedene Arten von Organismen verwendet werden. Hierbei können im Innenraum der Umhüllung z. B. mehrere Bereiche (z. B. Zellen) vorgesehen sein, welche jeweils größtenteils oder im Wesentlichen vollständig mit einer bestimmten Art von Organismus ausgestattet sind. In bestimmten Anwendungsfällen lässt sich damit z. B. die Effektivität der Photosynthese weiter erhöhen. Solche einzelnen, jeweils individuell mit Organismen ausgestattete Bereiche können z. B. in Form einzelner Innenraumabschnitte oder Zellen ausgeführt werden, um Störungen und Beeinträchtigungen auf einzelne Bereiche bzw. zwischen den einzelnen Bereichen begrenzen zu können und die Stoffumsetzung zu optimieren. Die Versorgung der einzelnen Bereiche mit CO2 oder einem CO2-haltigen Medium (z. B. Rauchgas aus einem Kraftwerk, CO2-angereichertes Wasser etc.) kann dabei z. B. in Form einer Reihen-, Parallel- oder Reihen/Parallelanordnung erfolgen.

Wenn z. B. bei einer Reihenanordnung das CO2 bzw. CO2-haltige Medium von einem Bereich (z. B. Zelle) in den nächsten Bereich geleitet wird, so nimmt die CO2-Konzentration von Bereich zu Bereich ab. In diesem Fall kann vorteilhaft eine Optimierung der Bereiche in Anpassung auf die jeweilige CO2-Konzentration vorgesehen sein, etwa durch spezielle Wahl der Organismen für die einzelnen Bereiche und/oder spezielle Einstellung sonstiger Umgebungsparameter der Organismen (z. B. Intensität der Sonnenlichtzufuhr, Luftfeuchtigkeit, pH-Wert, Temperatur etc.).

Demgegenüber werden bei einer Parallelanordnung alle Bereiche bevorzugt in gleichem Maße mit CO2 beaufschlagt und besitzen in etwa die gleiche CO2-Konzentration und die gleiche CO2-Beseitigungsaufgabe. In diesem Fall können durch geeignete Abtrennungen zwischen den Bereichen wieder Störungen bzw. Beeinträchtigungen (z. B. die Ausbreitung einer Krankheit) vermieden werden.

Mit der Umhüllung zur Unterbringung der Organismen in einem Innenraum der Umhüllung vereinfacht sich die Aufrechterhaltung technisch vorbestimmter, für die Photosynthese günstiger Umgebungsbedingungen für die einzelnen Organismen im Innenraum bzw. in einzelnen voneinander abgetrennten Innenraumbereichen. Je nach konkretem Anwendungsfall kann hierfür auch eine durch eine entsprechende technische Einrichtung nur teilweise geschaffene Umhüllung (z. B. Überdachung) ausreichend sein. Zu bedenken ist zum Beispiel, dass gegebenenfalls auch natürliche geographische Besonderheiten (z. B. Beschaffenheit des Bodens, Vorhandensein eines Sees etc.) vorteilhaft diesem Zweck dienen können bzw. somit einen Teil der bei der Erfindung vorgesehenen "Umhüllung" bilden können. So ist insbesondere denkbar, dass die zu errichtende Umhüllung im Wesentlichen von einer Abdeckung bzw. Überdachung gebildet wird. Hier kommt z. B. eine Leichtbauüberdachung in Frage, die z. B. nach Art einer Traglufthalle gestaltet sein kann. Beispielsweise kann eine Folie durch einen leicht erhöhten Innenraumdruck vom Untergrund abgehoben werden. Als alternative Bauweise sind auch übliche Hallen in einfacher (aber bevorzugt abgedichteter) Bauart denkbar, z. B. als Stahl- oder Stahlbetontragwerk mit einem ausreichenden Anteil lichtdurchlässiger Bereiche, z. B. aus Glas, Plexiglas oder anderen lichtdurchlässigen Materialien, insbesondere Kunststoffen.

In einer bevorzugten Ausführungsform umfasst die Umhüllung wenigstens eine transparente Folie oder Schicht. Gemäß einer "optimierten wasserpflanzenbasierten Lösung" kann z. B. vorgesehen sein, dass die Umhüllung in Form einer transparenten Folie oder Schicht mittelbar oder unmittelbar und im Wesentlichen vollflächig auf dem Wasser aufliegt. Damit entfallen vorteilhaft kostenintensive Tragwerke für die Umhüllung, weiterhin wird eine etwaige Windbeanspruchung minimiert. In einer Weiterbildung liegen einzelne Bereiche der Umhüllung nicht vollständig an dem photosynthetisch aktiven Bereich an, sondern bilden einen Hohlraum zur Zu- und/oder Abfuhr von CO2 bzw. O2.

Im Innenraum der Umhüllung sind die photosynthesetreibenden Organismen bevorzugt in hoher räumlicher Dichte angeordnet.

Gemäß einer "optimierten wasserpflanzenbasierten Lösung" werden einzellige, im Wasser treibende oder schwebende Organismen, insbesondere photosynthesetreibende Algen oder Cyanobakterien, verwendet. Zum Beispiel können Algen der oben bereits erwähnten Art "Chlorella minutissima" verwendet werden. Somit kann die Effektivität der Vorrichtung optimiert werden.

Es hat sich als vorteilhaft erwiesen, die Ausgangskonzentration oder die mittlere Konzentration der eingesetzten Organismen (z. B. Algen) so einzustellen, dass diese je nach vorliegender CO2-Konzentration hinsichtlich der CO2-Umsetzung und des Wachstums / der Vermehrung optimiert wird. Wie oben aufgeführt, sind bei einer optimalen Anpassung der Konzentration der Organismen (optimale Ausgangskonzentration der eingesetzten Algen in g / Liter aktiven Einrichtungsvolumens) hohe Wachstumsraten erreichbar.

In einer Ausführungsform umfasst die Vorrichtung eine Steuereinrichtung (z. B. programmgesteuerte, elektronische Steuereinrichtung) zur Steuerung wenigstens einer, insbesondere im Wesentlichen sämtlicher steuerbaren Einrichtungen der CO2-Beseitigungsvorrichtung. Mit einer derartigen Steuereinrichtung können vorteilhaft die Umgebungsbedingungen für die Organismen sowie sämtliche Zufuhr- und Abfuhrmaßnahmen in den Innenraum hinein und aus dem Innenraum heraus optimal eingestellt bzw. gesteuert werden.

In einer Ausführungsform umfasst die Vorrichtung ferner eine Sensorik zur Erfassung von Parametern der Organismen (z. B. Alter, Größe, Gesundheitszustand etc.) und/oder der Umgebung der Organismen (z. B. Sonnenlichtintensität, Temperatur etc.). Hierfür geeignete Sensoren sind aus dem Stand der Technik hinreichend bekannt und bedürfen daher keiner näheren Erläuterung. Die mittels der Sensorik erzeugten Sensorsignale bzw. Sensordaten können vorteilhaft als Eingangssignale bzw. Eingangsdaten der vorstehend erwähnten Steuereinrichtung zugeführt werden, welche die Steuerung der betreffenden technischen Einrichtungen dann in Abhängigkeit von den sensorisch erfassten Parametern vornehmen kann.

Mittels einer Steuerung können die biologischen Prozesse der Photosynthese in Richtung einer Effektivitätssteigerung optimiert werden, wobei je nach Art der verwendeten Organismen jeweils verschiedene Parameter (z. B. Temperatur(en), Luftfeuchtigkeit(en), pH-Wert(e), CO2-Konzentration(en), Sonnenlichtintensität(en) etc.) eine Rolle spielen können bzw. verschiedene Zielvorgaben mit sich bringen können.

In einer Ausführungsform umfasst die Vorrichtung Mittel zur gesteuerten oder passiven (ungesteuerten) Temperierung des Innenraumes, d. h. zum Heizen und/oder Kühlen des Innenraumes und/oder von Teilen davon (z. B. zur individuellen Temperierung der oben erwähnten Innenraumbereiche bzw- -zellen).

Die Temperierungsmittel bzw. Heiz/Kühleinrichtungen können einen Wärmespeicher (thermischen Speicher) umfassen, der z. B. von einer größeren Wassermasse oder Bodenmasse oder dergleichen (mit hoher Wärmekapazität) gebildet sein kann. Der Wärmespeicher kann z. B. eine so große Wärmekapazität besitzen, dass damit die (gemittelte) Innenraumtemperatur um wenigstens 5°C veränderbar ist (im Vergleich zu einer gleichartigen Vorrichtungskonstruktion ohne diesen Wärmespeicher). In Verbindung mit einer geeigneten Wärmemanagementeinrichtung, die z. B. aktiv durch die erwähnte Steuereinrichtung betrieben werden kann, lassen sich vorteilhaft gezielte Wärmetransfervorgänge aus dem Wärmespeicher heraus oder in den Wärmespeicher hinein realisieren (z. B. mittels Wärmemittelpumpen und Wärmetauschern).

In einer bevorzugten Ausführungsform steht der Wärmespeicher in unmittelbarer, wärmeübertragender Verbindung mit einem photosynthetisch aktiven (d.h. photosynthesetreibende Organismen enthaltenden) Bereich. Insbesondere kann der photosynthetisch aktive Bereich auf dem Wärmespeicher aufliegen, auf ihm schwimmen oder in Kontakt mit dessen Oberfläche oder Berandung angeordnet sein. Der Wärmespeicher und dessen Verbindung zum photosynthetisch aktiven Bereich können so gestaltet sein, dass Temperaturschwankungen im Tageszyklus (Tag-Nacht-Zyklus) in erheblichem Maße gedämpft werden (z. B. um mehr als 40%).

Mittels der Temperierungsmittel können vorteilhaft insbesondere Temperaturschwankungen in der Umgebung der Vorrichtung wenigstens teilweise ausgeglichen werden, seien es z. B. durch Tag und Nacht bedingte Temperaturschwankungen oder saisonale Temperaturschwankungen (Sommer/Winter).

Wie oben bereits erwähnt erfolgt die Errichtung und der Betrieb der Vorrichtung bevorzugt z. B. in Wüsten oder klimatisch ähnlichen Gegenden. Aufgrund der dort vorherrschenden hohen Tagestemperaturen und starken täglichen Temperaturschwankungen (z. B. Temperatur in Sebha/Libyen im Sommer: tagsüber 38-42°C, nachts 20-26°C) ist zur Erreichung einer optimalen Temperatur für die Photosynthese (z. B. 20-35°C) eine Stabilisierung der Temperatur durch eine aktive und/oder passive Temperaturführung, insbesondere auch Kühlung zweckmäßig.

Bei einer "landpflanzenbasierten Lösung", bei welcher ein Großteil oder im Wesentlichen sämtliche eingesetzten Organismen weitgehend von Luft umgeben und als Landpflanzen ausgebildet sind, kann dies z. B. durch Integration eines Wärmespeichers von ausreichend hoher Wärmekapazität in die Vorrichtung erreicht bzw. unterstützt werden. Ein solcher Wärmespeicher kann z. B. durch eine große Wassermasse gebildet sein, wobei gegebenenfalls zugeordnete Wärmetransfereinrichtungen (z. B. Wärmetauscher etc.) für jeweils bedarfsgerechte Wärmetransfervorgänge eingesetzt werden können. Außerdem kann eine Verdunstungskühlung eingesetzt werden (z. B. an einer großen Wasseroberfläche und/oder durch eine kontinuierliche oder zeitweise Verdüsung von Wassertropfen). Auch kommt eine bedarfsgerecht gesteuerte Abdeckung von Teilflächen der Vorrichtung durch Blenden, Spiegel oder dergleichen, sowie eine aktive Kühlung (Klimaanlagen, Kühlanlagen, Rückkühlanlagen etc.) in Betracht. Weiterhin kann eine Beregnung und/oder eine Bewässerung der Organismen vorgesehen werden. Es ist auch denkbar, die Organismen oder Teile davon zeitweise oder dauerhaft unter Wasser zu bringen. Letztere Maßnahmen können vorteilhaft auch zur Sicherstellung eines ausreichenden Feuchtigkeitsniveaus für die Photosynthese genutzt werden (z. B. zur Aufrechterhaltung einer ausreichenden Luftfeuchtigkeit). Zur Innenraumkühlung eingedüste Wassertröpfchen können somit auch zur Befeuchtung der Organismen und/oder deren Umgebung eingesetzt werden.

Bei einer "wasserpflanzenbasierten Lösung", bei welcher ein Großteil oder im Wesentlichen sämtliche Organismen wenigstens teilweise unter Wasser angeordnet sind (z. B. Wasserpflanzen), trägt bereits die Wärmekapazität des Wassers zum Ausgleich von Temperaturschwankungen bei (Nutzung der mittleren Tagestemperatur). Weiterhin kann die Größe eines Wasserreservoirs auf die Erzielung ausreichend kleiner Temperaturschwankungen bemessen werden. Alternativ oder zusätzlich können die für die landpflanzenbasierte Lösung vorstehend beschriebenen Maßnahmen zur Temperierung (und gegebenenfalls Befeuchtung) verwendet werden.

In einer bevorzugten Ausführungsform sind die Temperierungsmittel dazu ausgebildet, den Innenraum oder Teile davon bedarfsweise zu kühlen. Technische Möglichkeiten hierzu wurden vorstehend beispielhaft aufgeführt. Diese oder ähnliche technische Maßnahmen können steuerbar ausgeführt werden, so dass deren Betrieb bzw. deren Heiz/Kühlleistung durch eine Steuereinrichtung jeweils optimal eingestellt werden kann.

Weiter bevorzugt ist eine Ausführung, bei der der oder ein Wärmespeicher in unmittelbarer, wärmeübertragender Verbindung zum Innenraum und/oder zu einer Umgebung der Organismen steht. Der Wärmespeicher kann sich hierbei z. B. innerhalb des Innenraumes oder auch außerhalb des Innenraumes der Vorrichtung befinden. Insbesondere kann z. B. ein photosynthetisch aktiver Bereich auf dem Wärmespeicher aufliegen, auf ihm schwimmen oder in Kontakt mit dessen Oberfläche oder Berandung angeordnet sein. Als wärmeübertragender Kontakt wird dabei z. B. eine Anordnung verstanden, bei der Wärme im Wesentlichen durch Vorgänge der Wärmeleitung und/oder der natürlichen oder erzwungenen Konvektion übertragen wird. Somit kann eine ausreichende Temperaturstabilisierung auf im Wesentlichen passive Weise, also ohne signifikanten Energieeinsatz erreicht werden.

Die Größe des/der Wärmespeicher wird bevorzugt so bemessen, dass (unter Berücksichtigung der wärmeleitungsbedingten Temperaturdifferenzen zwischen Wärmespeicher und aktivem Bereich der Einrichtung) Temperaturschwankungen im Tageszyklus (Tag-Nacht-Zyklus) in ausreichendem Maße gedämpft werden (z. B. im Mittel um mindestens 40%). Insbesondere können der/die Wärmespeicher und die Verbindung zum aktiven Bereich der Einrichtung so bemessen sein, dass biologisch ertragbare Maximaltemperaturen nicht überschritten werden, insbesondere dass biologisch günstige Temperaturbereiche eingehalten werden.

In einer Ausführungsform ist vorgesehen, dass die Mittel zur Einleitung des Sonnenlichts steuerbar sind.

Im einfachsten Fall erfolgt die Einleitung des Sonnenlichts über eine wenigstens bereichsweise lichtdurchlässige Ausbildung der Umhüllung bzw. Abdeckung der Vorrichtung. In diesem Fall kann das Ausmaß der Sonnenlichteinleitung z. B. durch geeignete Blenden gesteuert werden. Mit einer Blendeneinrichtung (z. B. mit bevorzugt stufenlos verstellbaren Abschattungskörpern und/oder Spiegeln) kann eine bedarfsweise Begrenzung der Sonnenlichtleinleitung in den Innenraum realisiert werden. Derartige Blendeneinrichtungen können somit sowohl als Teil von steuerbaren Lichteinleitungsmitteln als auch als Teil der Temperierungsmittel angesehen werden. Bevorzugt gestattet die Blendeneinrichtung eine Maximal-Verminderung der einfallenden Strahlungsleistung um mindestens 50%.

Zur Verbesserung der Effektivität können photolumineszente Stoffe zur Wandlung von Bestandteilen der Sonnenstrahlung mit schlechterer Nutzbarkeit für die betreffende Photosynthese in Bestandteile von besserer Nutzbarkeit vorgesehen werden.

Die Integration der photolumineszenten Stoffe in die Vorrichtung kann so erfolgen, dass das emittierte (gewandelte) Licht den photosynthesetreibenden Organismen zufließen kann (z. B. Einbringung im als Trägermedium für die Organismen dienenden Wasser, an einer Berandung des Innenraumes oder im Strahlengang des in den Innenraum einfallenden Sonnenlichts.

In einer Weiterbildung der Erfindung umfasst die Vorrichtung photolumineszente Stoffe zur Wandlung von kurzwelligen Bestandteilen der Sonnenstrahlung (z. B. UV-Bestandteile) in langwelligere Strahlung (z. B. im sichtbaren Spektralbereich). Damit kann eine erhebliche Effizienzsteigerung hinsichtlich der Nutzung der Sonnenstrahlung für die Photosynthese erzielt werden.

Hierfür verwendbare Stoffe sind aus dem Stand der Technik bekannt (Beispiel: photolumineszente Beschichtung in Leuchtstoffröhren zur Umwandlung von UV-Licht einer Gasentladung in Licht im sichtbaren Spektrum).

In einer Ausführungsform ist die Umhüllung zumindest teilweise durchlässig für das Sonnenlicht, und insbesondere mit einem größtenteils lichtdurchlässigen Überdachungsbereich ausgebildet. Bevorzugt sind mehr als 90% des Überdachungsbereiches aus einem lichtdurchlässigen Material gebildet (Lichtdurchlässigkeit für den Bereich von 360 bis 700 nm bevorzugt größer als 90%).

In einer Weiterbildung umfassen die Mittel zur Einleitung des Sonnenlichts eine Anordnung von Spiegelflächen (innerhalb und/oder außerhalb des Innenraumes), z. B. zur Sammlung, Bündelung oder Umlenkung der Sonnenstrahlung.

Etwa können zur weiteren Erhöhung der Menge eingestrahlten Sonnenlichtes Spiegelflächen (bevorzugt gesteuert verstellbar) vorgesehen werden, die Sonnenlicht, welches außerhalb der photosynthese-wirksamen Oberflächen der Vorrichtung einfällt, so umlenken, dass es zur Photosynthese beiträgt.

In einer Ausführungsform wird die CO2-Zufuhr zur Erzielung einer vorbestimmten CO2-Konzentration in der Umgebung (Wasser und/oder Luft) der Organismen geeignet gesteuert.

In einer bevorzugten Ausführungsform ist die CO2-Zufuhreinrichtung dazu ausgebildet, die CO2-Konzentration in der Umgebung der Organismen so einzustellen, dass sich eine gegenüber natürlichen Bedingungen nennenswert erhöhte CO2-Umsetzungsrate ergibt (z. B. wenigstens 2-fache, insbesondere wenigstens 5-fache Rate).

Insbesondere kann für den Luftanteil im Innenraum eine im Vergleich zur atmosphärischen CO2-Konzentration (etwa 0,04%) höhere CO2-Konzentration (z. B. 0,1 bis 1,0%) eingestellt werden, was in der Regel eine beträchtliche Steigerung der Umsetzungseffizienz mit sich bringt. In einer Ausführungsform ist eine CO2-Konzentration in der (Luft-)Umgebung der Organismen von mehr als 0,1%, insbesondere mehr als 1% vorgesehen.

In einer Ausführungsform liegt die CO2-Konzentration in einer gasförmigen Umgebung der Organismen im Bereich von 1% bis 40%.

Bei den in dieser Anmeldung für eine gasförmige Umgebung der Organismen angegebenen Prozentangaben von CO2-Konzentrationen sind Volumenprozent gemeint.

Bei einer wässrigen Umgebung der Organismen kann die CO2-Konzentratrion im Bereich der Konzentrationen liegen, die sich bei Lösungsvorgängen von CO2 im Wasser bei Kontakt mit einem Gasgemisch mit den genannten CO2-Anteilen einstellen.

Der optimale Einsatzbereich für die Einrichtung liegt im Allgemeinen bei CO2-Konzentrationen von z. B. 35% bis hinunter zu 5% (für Chlorella minutissima), schlechter z. B. 35% bis 40% oder 1% bis 5%. Vorteilhafterweise wird im Verlauf der CO2-Beseitigung (mit abnehmender CO2-Konzentration) eine auf die jeweilige CO2-Konzentration hin optimierte Ausgangskonzentration oder mittlere Konzentration der eingesetzten Organismen (z. B. Algen) eingestellt, so dass sich je nach vorliegender CO2-Konzentration hinsichtlich der CO2-Umsetzung und des Wachstums und/oder der Vermehrung ein Optimum oder ein diesem naher Wert einstellt.

Eine CO2-Zufuhreinrichtung kann z. B. zur Zufuhr von gasförmigem CO2 in den Innenraum oder Bereiche davon ausgebildet sein. Alternativ oder zusätzlich kann eine Zufuhr von in wässriger Lösung vorliegendem CO2 vorgesehen sein (z. B. mittels Eindüsung). Gasförmiges komprimiertes CO2, flüssiges CO2 oder festes CO2 ("Trockeneis") kann z. B. über eine Expansions- bzw. Drosselungseinrichtung in gesteuerter Weise in den Innenraum der Vorrichtung bzw. einzelne Bereiche davon eingebracht werden.

Es versteht sich, dass das "CO2" bei der CO2-Beseitigungsvorrichtung nicht in reiner Form in den Innenraum zugeführt werden muss, sondern vielmehr auch als Bestandteil eines Gemisches wie z. B. als Bestandteil der atmosphärischen Luft oder eines Abgases aus einer Verbrennungsanlage wie insbesondere einem Kraftwerk oder dergleichen zugeführt werden kann.

In einer bevorzugten Ausführungsform umfasst die Vorrichtung (wenigstens) einen Austauschbereich (z. B. Austauschkanal), in dem gasförmig eingeleitetes CO2 (z. B. Gasgemisch) durch fein verteilte Einbringung (Eindüsung) von Wasser oder durch Führung über einer Wasseroberfläche in Lösung gehen kann. Das Wasser, und damit das CO2, kann sodann dem photosynthetisch aktiven Bereich zugeführt werden. Insbesondere kann die Vorrichtung einen Austauschbereich, welcher sich longi-tudinal erstreckt, insbesondere einen sich entlang oder durch die Vorrichtung hindurch erstreckenden Austauschbereich umfassen.

In einer vorteilhaften Ausgestaltung, welche sich besonders für eine wasserpflanzenbasierte Lösung, insbesondere für eine "optimierte wasserpflanzenbasierte Lösung" eignet, wird das CO2 oder ein CO2-haltiges Gasgemisch entlang wenigstens eines Austauschkanals geführt. Der Kanal kann z. B. ein langgestreckter, insbesondere sich longitudinal erstreckender, eine Gasströmung führende und einen über seine Erstreckung erfolgenden Gasaustausch ermöglichender Hohlraum sein.

Vorteilhaft wird der Austauschkanal entlang der Vorrichtung oder durch sie hindurch geführt, wodurch der Kanal den Transport des CO2 in die Einrichtung hinein übernehmen kann. Weiterhin kann der Austauschkanal den Abtransport des erzeugten Sauerstoffes übernehmen, welcher im Gegenzug zum entnommenen CO2 eingebracht werden kann.

Der Austausch kann vorteilhaft und energieaufwandsarm erfolgen, indem man die natürliche Löslichkeit von CO2 und/oder Sauerstoff in einer Flüssigkeit, insbesondere z. B. Wasser ausnutzt. Zur Erreichung eines möglichst effektiven Gasüberganges kann z. B. Wasser aus der Einrichtung in den Kanal eingedüst und dieses Wasser zur Einrichtung zurückgeführt werden, wodurch Lösungs- und Rücklösungsvorgänge von zu beseitigendem CO2 und produziertem Sauerstoff erreicht werden. Durch eine Einstellung des pH-Wertes des eingedüsten Wassers kann die Löslichkeit von CO2 und damit dessen Stoffaustausch und Transport begünstigt werden. Als vorteilhaft haben sich z. B. pH-Werte von 7 bis 9 erwiesen, bevorzugt 7,5 bis 8,5. Durch die Aufnahme von CO2 sinkt der pH-Wert ab (Bildung von Kohlensäure in wässriger Lösung). Als praktikable Untergrenze des pH-Wertes nach Aufnahme von CO2 hat sich ein pH-Wert von 5,5, besser 5,8 erwiesen. Durch eine Temperaturerhöhung des Austauschkanals kann auch die O2-Rücklösung verbessert werden (Erhöhung der Temperatur z. B. durch eingestrahltes Sonnenlicht, ggf. durch Spiegel verstärkt).

Bei optimaler Sättigung des Wassers mit CO2 kann der bei der CO2-Beseitigung entstehende Sauerstoff aufgrund seiner geringeren Löslichkeit nicht mehr mit der gleichen Menge an Wasser zurücktransportiert werden. Somit wird Sauerstoff in der Einrichtung außer Lösung gehen und an die Oberfläche steigen. Zur Sammlung/Abfuhr können in geeigneten Abständen O2-Sammelleitungen an der Oberfläche positioniert werden. Je nach O2- und CO2-Konzentration kann das Gas aus diesen Sammelleitungen dem Austauschkanal oder einer anderen Stelle der Einrichtung (z. B. auch dem Auslass der Einrichtung) zugeleitet werden.

In einer Ausführungsform stammt zugeführtes CO2 aus der Atmosphäre und wird unmittelbar (z. B. nach Extraktion aus der atmosphärischen Luft, oder als Bestandteil der atmosphärischen Luft) aus der Umgebung der Vorrichtung in den Innenraum zugeführt.

Erfindungsgemäß wird jedoch (auch) aus einer anthropogenen Quelle (Brennraum der Verbrennungsanlage) stammendes CO2 zwecks gesteuerter Zufuhr in die Vorrichtung verwendet (z. B. aus einer Großfeuerungsanlage, einem Kraftwerk oder dergleichen) .

Ein Beispiel hierfür ist die Erzeugung von CO2 im Betrieb eines Kraftwerkes zur Energieerzeugung durch einen CO2-erzeugenden Verbrennungsprozess.

Erfindungsgemäß ist eine Kombination der CO2-Beseitigungsvorrichtung mit einer Verbrennungsanlage, insbesondere z. B. einem Kraftwerk vorgesehen.

Ein besonderer Vorteil dieser Kombination besteht z. B. darin, dass das im Betrieb der Verbrennungsanlage als Bestandteil des Abgases (z. B. so genanntes Rauchgas) erzeugte CO2 bereits eine sehr hohe Konzentration (z. B. etwa 20%) besitzt, was eine hocheffiziente photosynthetische Umsetzung erlaubt. Falls, etwa bedingt durch die konkrete Art der verwendeten Organismen, eine niedrigere CO2-Konzentration in der Umgebung der Organismen von Vorteil ist, so kann dies problemlos durch einen geeignet gesteuerten Verdünnungsprozess (z. B. Beimischung von atmosphärischer Luft) bewerkstelligt werden.

Eine Verwendung von Abgas mit einem erhöhten CO2-Anteil ermöglicht eine Verbesserung der Umsetzung von CO2 in der Vorrichtung. Wie oben erwähnt, kann z. B. eine CO2-Konzentration im Bereich von 35% bis 5% besonders günstig für eine effektive Umsetzung sein. In der Kombination mit einer Verbrennungsanlage kann dies besonders einfach dadurch ausgenutzt werden, dass diese anstelle von Frischluft bereits ein Gasgemisch mit einem erhöhten CO2-Anteil für die Verbrennung verwendet. Dabei kann dem Verbrennungsprozess ein Gasgemisch zugeführt werden, welches sich (neben Spurengasen) im Wesentlichen aus Sauerstoff (z. B. 20%), CO2 (z. B. 15%) und Stickstoff (Rest) zusammensetzt. In der Brennkammer wird der Sauerstoff mit Brennstoff nahezu vollständig in CO2 umgesetzt, so dass sich eine Abgaszusammensetzung aus Stickstoff und einem hohen Anteil von CO2 (z. B. 35%) ergibt. Hinsichtlich der Verbrennung wird dabei Stickstoff (als in diesem Kontext weitestgehend inertes Gas) durch das ebenfalls nicht brennbare und nicht oxidierend wirkende CO2 ersetzt.

In einer besonders bevorzugten Ausführung wird dabei ein im Wesentlichen geschlossener Gaskreislauf geschaffen, in dem eine Menge von Stickstoff und CO2 von der Verbrennungsanlage zur Beseitigungsvorrichtung und zurück umläuft, ergänzt durch eine Menge von Sauerstoff bzw. CO2, welches in der Verbrennung und in der CO2-Beseitigung umgesetzt wird. Der umlaufende Anteil des CO2 dient dabei der vorteilhaften Erhöhung der CO2-Konzentration in der Einrichtung und erlaubt einen Betrieb der Einrichtung im Bereich des biologischen bzw. des photosynthetischen Optimums.

Ein noch weiterer Vorteil dieser Kombination besteht darin, dass aus dem Betrieb der CO2-Beseitigungsvorrichtung anfallende "Biomasse" als Brennstoff (gegebenenfalls zusätzlicher Brennstoff) dem Verbrennungsanlagen- bzw. Kraftwerksbetrieb zugeführt werden kann.

In einer Ausführungsform umfasst die Vorrichtung eine Einrichtung zur Abfuhr von O2 (Sauerstoff), wobei diese Einrichtung einen Teil bzw. eine Teilfunktionalität der steuerbaren Produkt-Abfuhreinrichtung zur gesteuerten Abfuhr von Produkten der CO2-Umsetzung darstellen kann.

Die O2-Abfuhreinrichtung kann z. B. dazu ausgebildet sein, gasförmige Produkte der CO2-Umsetzung aus dem Innenraum zu extrahieren.

In diesem Zusammenhang versteht sich, dass das "O2" nicht in reiner Form abgeführt werden muss, sondern z. B. auch als Bestandteil eines Gemisches wie der im Innenraum (bzw. Bereichen davon) befindlichen Luft ("Innenraumatmosphäre") und/oder gegebenenfalls im Innenraum vorgesehenen Wassers abgeführt werden kann.

Die O2-Abfuhreinrichtung kann insbesondere zur Abfuhr von Luft mit einem erhöhten O2-Anteil aus der Innenraumatmosphäre verwendet werden. In einer Weiterbildung wird wenigstens ein Teil dieser Luft einer Weiterverarbeitung und/oder Wiederverwendung zugeführt, wofür nachfolgend noch Beispiele erläutert werden.

Die O2-Abfuhreinrichtung kann einen so genannten CO2-Abscheider umfassen, um aus dem Abfuhrstrom CO2 bzw. CO2-reiche Luft abzuscheiden und bevorzugt über die CO2-Zufuhreinrichtung wenigstens einen Teil der CO2-reichen Luft wieder in den Innenraum zuzuführen.

Die O2-Abfuhreinrichtung, welche gegebenenfalls auch zur Extraktion und Rückführung von CO2 dient, kann Teil einer für die Vorrichtung vorgesehenen Luftaustauscheinrichtung sein, mittels derer ein bevorzugt gesteuerter Luftaustausch zwischen dem Innenraum (oder Teilen davon) und der Atmosphäre bewerkstelligt wird. Eine derartige Luftaustauscheinrichtung kann auch im Rahmen der gesteuerten Temperierung des Innenraumes der Vorrichtung verwendet werden.

In der weiter oben genannten Ausführung mit einem Austauschkanal und einem über dessen z. B. longitudinale Erstreckung durchgeführten Gasaustausch wird O2 im Verlauf der Durchströmung des Austauschkanals durch Rücklösungsvorgänge aus eingedüstem Wasser sowie aus optional angeschlossenen Sauerstoffsammelleitungen aufkonzentriert, so dass der Austritt des Kanals als Sauerstoff-Abfuhreinrichtung fungieren kann. Alternativ dazu können die vorgenannten Sauerstoffsammelleitungen separat zum Auslass der Einrichtung führen.

Es ist bekannt, dass bestimmte Mikroorganismen (z. B. Mikroalgen der Art "Dunaliella parva") in der Lage sind, Stickoxide (NOx) in unschädliche Substanzen umzusetzen. Dies kann vorteilhaft genutzt werden, indem derartige Organismen mit in die ersten bzw. die zuerst beströmten Bereiche der CO2-Beseitigungsvorrichtung eingebracht werden. Bei Verwendung eines Austauschkanals wird NOx in den ersten Bereichen des Kanals ausgetragen und im Wasser gelöst, da es nur in kleinen Konzentrationen vorliegt. Damit kann (je nach Schadstoffanfall) eine separate Anlage zur Rauchgasentstickung entfallen. Prinzipiell können Organismen mit der Fähigkeit zur NOx-Umsetzung auch in der gesamten Anlage eingesetzt/zugefügt werden. Ähnliches gilt optional und in analoger Weise auch für andere Schadstoffe im Verbrennungsabgas, etwa SO2.

In einer Ausführungsform umfasst die Vorrichtung eine Biomasse-Abfuhreinrichtung. Damit können die Organismen oder ein Teil davon (z. B. ältere oder abgestorbene Organismen) und deren im Betrieb der Vorrichtung entstandenen Stoffwechselprodukte aus dem Innenraum heraus abgeführt werden. Diese Abfuhr kann kontinuierlich oder diskontinuierlich (z. B. in vorbestimmten zeitlichen Abständen) vorgenommen werden, etwa um vorbestimmte Bedingungen des "Ökosystems" im Innenraum der Vorrichtung aufrechtzuerhalten bzw. einzustellen.

Der Biomasse-Abfuhreinrichtung kann eine Biomasse-Verarbeitungseinrichtung (mit Komponenten innerhalb und/oder außerhalb des Innenraumes) nachgeordnet sein, mittels welcher je nach Anwendungsfall verschiedene Weiterverarbeitungsschritte (z. B. Wasserentzug, Verdichtung etc.) durchgeführt werden können.

Die Biomasse-Abfuhreinrichtung sowie die gegebenenfalls nachgeordnete Biomasse-Verarbeitungseinrichtung können vorteilhaft steuerbar ausgebildet sein, um deren Betrieb in die durch eine Steuereinrichtung bewirkte Steuerung der Vorrichtung einzubeziehen.

In einer bevorzugten Weiterbildung wird bei der Abfuhr der Biomasse Wasser entzogen und in den Innenraum der Vorrichtung zurückgeführt. Das zurückgeführte Wasser kann insbesondere als Trägermedium für die Organismen (z. B. im Falle von Wasserpflanzen), zur Versorgung der Organismen, zu Kühlungszwecken (z. B. durch Verdüsung) und/oder zur Ergänzung eines Wasserreservoirs genutzt werden, welches z. B. einen Wärmespeicher bildet.

In einer Ausführungsform ist ein im Wesentlichen kontinuierlicher Abzug der im Innenraum erzeugten Biomasse durch entsprechende technische Einrichtungen zur Entnahme, Trocknung und Weiterverarbeitung vorgesehen. Damit kann vorteilhaft ein Gleichgewicht der Erzeugung von Biomasse (z. B. Pflanzenmasse) und ihrem Abzug erreicht werden.

Für den Wasserhaushalt der Gesamtvorrichtung ist es außerdem als wichtig zu erachten, dass bei etwaigen Trocknungsvorgängen der erzeugten Biomasse anfallendes Wasser aufgefangen und der Vorrichtung, gewissermaßen in einem Kreislauf, wieder zugeführt wird. Das gleiche gilt für anfallenden Wasserdampf, welcher kondensiert und ebenfalls wieder zugeführt werden kann. Die Trocknung der Biomasse wird vorzugsweise, soweit erforderlich, auf mechanische Weise (z. B. Filtration, Sedimentation, Zentrifugieren) und/oder thermische Weise (z. B. Einwirkung von Sonnenstrahlung oder einer anderen Wärmequelle) bewirkt. Die entnommene und gegebenenfalls weiterverarbeitete Biomasse kann sodann verschiedenen Nutzungen zugeführt werden, beispielsweise: Nahrungs- oder Futtermittel, Düngemittel, Grundstoff für die Produktion von Kraftstoffen, Brennstoff für Verbrennungsanlagen wie z. B. Kraftwerken.

In einer bevorzugten Ausführungsform umfasst die Biomasse-Abfuhreinrichtung eine umschlossene Trocknungseinrichtung zur Trocknung von aus dem Innenraum entnommener Biomasse.

In einer Weiterbildung ist die Trocknungseinrichtung zur Trocknung der Biomasse durch Wärme aus Sonneneinstrahlung ausgebildet.

In einer Weiterbildung ist die Trocknungseinrichtung bei einer erhöhten Temperatur gegenüber dem photosynthetisch aktiven Bereich gehalten, insbesondere auch gegenüber den sonstigen Bereichen der Vorrichtung.

In einer bevorzugten Ausführungsform wird der Trocknungseinrichtung im Wesentlichen abgekühltes Rauchgas (bevorzugt als nicht gesättigtes Gasgemisch) aus einem Verbrennungsprozess (z. B. Kraftwerksbetrieb) zugeführt.

In einer bevorzugten Ausführungsform umfasst die Trocknungseinrichtung eine Wasserdampf-Abfuhreinrichtung, um bei der Trocknung entstehenden Wasserdampf abzuführen. Diese Abfuhr kann z. B. durch ein Gas/Gasgemisch erfolgen. In einer Weiterbildung wird das wasserdampfhaltige Gas/Gasgemisch direkt oder indirekt wieder in den Innenraum der Vorrichtung zugeführt.

In einer bevorzugten Weiterbildung umfasst die Biomasse-Abfuhreinrichtung eine umschlossene Trocknungseinrichtung, bei der stückige oder feste, dem photosynthetisch aktiven Bereich entnommene Biomasse einer Zerkleinerung und/oder Entwässerung bzw. Trocknung zugeführt wird. Die Zerkleinerung kann auch bei oder nach der Entnahme erfolgen. In einer anderen bevorzugten Weiterbildung der Vorrichtung mit einer umschlossenen Trocknungsvorrichtung wird biomassehaltiges Wasser aus dem photosynthetisch aktiven Bereich abgezogen und einer Eindickung und/oder Entwässerung zugeführt. Hierbei kann vorgesehen sein, dass Biomasse oder Gasgemisch aus späteren Prozessschritten zurückgeführt (rezirkuliert) und beigemischt wird.

In einer Ausführungsform umfasst die Vorrichtung ferner eine steuerbare Hilfsstoff-Zufuhreinrichtung zur Zufuhr von Hilfsstoffen (z. B. Stoffe zur Aufrechterhaltung bzw. Verbesserung eines Gesundheitszustandes der Organismen bzw. deren Photosyntheseeffizienz) in den Innenraum bzw. zu den Organismen.

Bei den Hilfsstoffen kann es sich insbesondere um Nährstoffe, Dünger, Pflanzenschutzmittel (z. B. gegen Krankheitsbefall der Organismen) etc. handeln.

In einer bevorzugten Ausführungsform werden Nährstoffe und Düngemittel in gesteuerter Weise in geeigneter Art und Menge zugeführt, um die Durchführung der Photosynthese und gegebenenfalls des Wachstums zu verbessern. Die jeweils geeigneten Nährstoffe und Düngemittel werden durch die Art der eingesetzten Organismen bestimmt. Zur Bekämpfung etwaiger Krankheiten (z. B. Pflanzenkrankheiten) oder von Schädlingsbefall können über die Hilfsstoff-Zufuhreinrichtung jeweils geeignete Schutzmittel eingebracht werden.

In einer bevorzugten Ausführungsform ist die Vorrichtung mit einem weitgehend deren gesamte Fläche beanspruchenden photosynthetisch aktiven (mit den Organismen besetzten) Bereich ausgebildet, z. B. mehr als 80% der Fläche, bevorzugt mehr als 90% der Fläche. Bevorzugt besitzt die Vorrichtung eine im Wesentlichen flächig erstreckte Gestaltung (im Gegensatz z. B. zu Rohren/Kanälen), z. B. mit einer Gesamtfläche der Vorrichtung, deren Quadratwurzel größer als das 10²-fache, insbesondere 10³-fache, der Höhe der Vorrichtung (oder der Höhe der photosynthetisch aktiven Bereiche der Vorrichtung) ist.

In einer Ausführungsform umfasst die Vorrichtung einen mit Wasser befüllten Bereich, beispielsweise ein mit Wasser befülltes Becken, zur Aufnahme wenigstens eines Teils der Organismen im Wasser. Dies ist insbesondere für die bereits erwähnte "wasserpflanzenbasierte Lösung" von Vorteil.

Das Wasserbecken bzw. Wasserreservoir stellt eine Einrichtung zur Aufnahme eines Trägermediums (Wasser, gegebenenfalls mit Zusätzen) für die hierfür geeigneten Organismen dar. Bevorzugt wird der mit Wasser befüllte Bereich (z. B. Wasserbecken) als Einrichtung vorwiegend zur Aufnahme, Speicherung und zum Verweilen mit einer hohen Dichte photosynthesetreibender Organismen versetzt, so dass einfallendes Licht zu einem möglichst großen Teil für die Photosynthese genutzt werden kann. Das Wasser kann beispielsweise so dicht mit den Organismen besetzt sein, dass die Intensität des auf die Organismen fallenden Sonnenlichtes pro Meter (an "Eindringtiefe") um mindestens 50% abnimmt. Bevorzugt beträgt diese Intensität bei maximaler Eindringtiefe noch höchstens 10% der vorkommenden Maximalintensität.

Bevorzugt ist für eine bereits genannte "optimierte wasserpflanzenbasierte Lösung" eine Ausführung mit einem auf einer Wasserfläche / einem Wärmespeicher aufliegenden / schwimmenden / positionierbaren photosynthetisch aktiven Bereich. Bevorzugte Abmessungen wie z. B. eine Dicke des aktiven Bereiches und eine Dicke der Gesamteinrichtung können z. B. wie unten anhand der Ausführungsbeispiele beschrieben gewählt werden.

In einer Ausführungsform wird als Wasserreservoir eine natürliche Wasseransammlung wie z. B. ein größerer See genutzt. Auch ist es denkbar, ein "natürliches Becken", gegebenenfalls nach technischen Abdichtungsmaßnahmen, mit Wasser zu befüllen.

In einer Ausführungsform sind die Organismen im Wasser schwebende Organismen.

In einer Ausführungsform sind die Organismen einzellige Algen oder Cyanobakterien, beispielsweise Algen der Art Chlorella minutissima.

In einer Weiterbildung erfolgt die CO2-Zufuhr direkt in das Wasser hinein (z. B. durch unter Wasser angeordnete Düsen/Einlassöffnungen für CO2 bzw. ein CO2-haltiges Medium ( z. B. CO2-angereichertes Wasser). Alternativ oder zusätzlich kann die CO2-Zufuhr an die Wasseroberfläche heran erfolgen.

In einer Ausführungsform ist beispielsweise vorgesehen, dass CO2, welches aus einem Kraftwerksbetrieb oder aus einer anderen anthropogenen Quelle stammt, der Vorrichtung zugeführt und in einer für den Betrieb bzw. die Photosynthese optimalen Konzentration in das Wasser eingebracht wird. Die CO2-Zufuhr kann hierbei vorteilhaft in gesteuerter Weise unter Berücksichtigung der beispielsweise von einer Sensorik erfassten Parameter in der Umgebung der Organismen erfolgen. Damit kann z. B. eine Übersäuerung des Wassers vermieden werden und insbesondere die Überschreitung einer vorgegebenen Maximalkonzentration an CO2 im Wasser vermieden werden. Diese Maximalkonzentration kann unter Berücksichtigung der Art der eingesetzten Organismen vorgegeben werden.

In einer Weiterbildung sind ferner Mittel zur Verwirbelung und/oder Umwälzung des Wassers vorgesehen. Diese Mittel können z. B. am Wasserbecken angeordnete Wasserauslässe und Wassereinlässe, Wasserleitungsanordnungen mit Filtern, Pumpen etc., Rühreinrichtungen im Wasserbecken etc. umfassen und sind bevorzugt steuerbar ausgebildet.

Um eine gute Exposition aller Organismen zu erreichen, ist in einer Ausführungsform ein kontinuierliches oder diskontinuierliches (z. B. in vorbestimmten Zeitabschnitten erfolgendes) Umwälzen und/oder Verwirbeln des Wasserbeckeninhalts vorgesehen. Damit kann der Stoffaustausch der Photosynthese (Photosynthese bei Lichtexposition, Stoffaustausch auch außerhalb der Exposition; dadurch wird der Einfluss der Stoffaustauschgeschwindigkeit reduziert) unterstützt werden. Damit kann insbesondere eine Photosynthese-Inaktivierung der Organismen verhindert werden, die bei einer ununterbrochenen Lichteinstrahlung auftreten kann.

Die Umwälz- bzw. Verwirbelungsgeschwindigkeit ist dabei zweckmäßigerweise so zu begrenzen, dass die eingesetzten Organismen nicht zu großen mechanischen Beanspruchungen (z. B. Schub bzw. Scherung) ausgesetzt werden.

In einer Ausführungsform der "wasserpflanzenbasierten Lösung" erfolgt die CO2-Zufuhr bevorzugterweise in gasförmiger Form durch Einleitung in den Innenraum, z. B. in die Innenatmosphäre der Vorrichtung (Gaspolster über der Wasseroberfläche). Optional kann hierbei eine Verdüsung von Wassertröpfchen in dieser Innenatmosphäre vorgesehen sein, um eine größere Eintrittsoberfläche für den Eintritt des CO2 in das Wasser zu schaffen. Alternativ oder zusätzlich kann CO2 auch durch Verdüsung unterhalb des Wasserspiegels in das Wasser eingebracht werden. Weiterhin kann alternativ oder zusätzlich die Einbringung des CO2 in Form einer wässrigen Lösung (enthaltend CO2) erfolgen, welche z. B. dadurch erzeugt werden kann, dass Wasser in einen intensiven Kontakt mit CO2-haltigem Gas gebracht wird (z. B. Eindüsung feiner Wassertröpfchen in das betreffende Gas). Außerdem kann CO2 auch eingebracht werden durch Expansion bzw. Drosselung von komprimiert an die Vorrichtung herangeführtem CO2 (gasförmig oder flüssig) oder durch Verdampfung von flüssig oder fest angeliefertem CO2, gegebenenfalls wieder mittels einer bevorzugt gesteuerten Expansion bzw. Drosselung.

Besonders bevorzugt für die "wasserpflanzenbasierte Lösung" und deren Optimierung ist eine Zuführung mittels eines Austauschkanals, wobei Wasser in einer CO2-haltigen Gasströmung fein verteilt, insbesondere verdüst wird. Beim Austauschkanal werden vorteilhaft Abschnitte mit hoher vorgesehener CO2-Konzentration zu Beginn einer longitudinalen Erstreckung angeschlossen, und Abschnitte mit niedriger vorgesehener Konzentration zum Ende hin. Somit werden die Vorgänge des Stoffaustausches und Stofftransportes optimiert.

Bevorzugterweise ist über dem Wasserbecken eine Abdeckung bzw. Überdachung zur Reduzierung von Verdampfungsverlusten (Wasser) und Ausgasungsverlusten (CO2) vorgesehen. Im einfachsten Fall wird diese Funktion von der Umhüllung der Vorrichtung bzw. einem Teil dieser Umhüllung realisiert. Eine solche Abdeckung kann auch eine geeignete Durchlasscharakteristik für Sonnenlicht aufweisen.

Vorteilhafterweise wird (in der Gestaltung als "optimierte wasserpflanzenbasierte Lösung") eine Abdeckung bzw. Überdachung (also die Trennung der erhöhten CO2-Konzentration von der Atmosphäre) durch eine auf der Wasseroberfläche (weitgehend) aufliegende, für Sonnenlicht in den relevanten Wellenlängenbereichen durchflutbare Folie oder Scheibe bzw. Anordnung einer Vielzahl solcher Folien bzw. Scheiben erzielt. Durch die direkte Auflage wird eine Windbeanspruchung (Differenzdruck, Staudruck) vermieden.

In einer Ausführungsform, die insbesondere für die erwähnte "landpflanzenbasierte Lösung" geeignet ist, umfasst die Vorrichtung eine Trägereinrichtung für eine vorbestimme räumliche Anordnung der Organismen.

In einer Ausführungsform wird als Trägereinrichtung ein zur Besiedlung mit Organismen (z. B. Bepflanzung mit Bäumen, Sträuchern, Gräsern etc.) geeigneter natürlicher Boden genutzt.

Alternativ oder zusätzlich wird die Trägereinrichtung durch eine eigens hierfür vorgesehene technische Einrichtung wie z. B. Pflanzkästen etc. realisiert. Auch können die mit Landpflanzen versehenen Flächen der Trägereinrichtung mit einem von Pflanzen bewachsbaren Material beschichtet sein oder aus einem solchen Material bestehen (z. B. Pflanzmatten etc.).

In einer bevorzugten Weiterbildung ist die Trägereinrichtung in gesteuerter Weise bewegbar, so dass damit die räumliche Anordnung der Organismen auch veränderbar ist. Die Trägereinrichtung kann z. B. flächig ausgedehnte und durch Ansteuerung bewegbare Pflanzenträger umfassen, die mit den betreffenden Organismen versehen sind.

Die Bewegbarkeit der Trägereinrichtung bzw. von Teilen davon ermöglicht es z. B. vorteilhaft, die Anordnung der Organismen hinsichtlich einer momentanen Sonneneinstrahlungsrichtung zu optimieren bzw. einer solchen Einstrahlungsrichtung nachzuführen. Auch ist es denkbar, einen Teil der Organismen zeitweise aus Bereichen mit Sonnenlichtexposition herauszubewegen.

In einer Ausführungsform ist vorgesehen, dass die Trägereinrichtung statische oder gesteuert bewegbare Flächen oder Bauteile aufweist, die mit den photosynthesetreibenden Organismen versehen sind.

Die Organismen werden im Innenraum der Vorrichtung bevorzugt unter einer vorbestimmten Feuchtigkeit und einer vorbestimmten CO2-Konzentration gehalten. Für die Photosynthese bei vielen Arten von Landpflanzen ist beispielsweise eine CO2-Konzentration im Bereich von 0,1 - 5% sehr vorteilhaft. Eine möglichst nicht zu überschreitende Maximalkonzentration von CO2 wird durch die Art der eingesetzten Organismen bestimmt. Die Flächen oder Bauteile, an welchen die Organismen gehalten sind, können mit einer Anordnung biologisch wirksamen Bodens versehen sein.

Die mit Organismen versehenen Oberflächenbereiche der Trägereinrichtung werden bevorzugt so angeordnet, dass einfallendes Sonnenlicht zu einem möglichst großen Teil für die Photosynthese genutzt werden kann. Um eine gute Lichtexposition möglichst vieler Organismen zu erreichen, können die Oberflächen beispielsweise geneigt, gekrümmt oder wellenförmig verlaufen. Bewegbare Oberflächen können vorteilhaft der Sonneneinstrahlung nachgeführt werden. Der Innenraum kann beispielsweise so dicht mit den Organismen besetzt sein, dass die Intensität des auf die Organismen fallenden Sonnenlichtes pro Meter (an "Eindringtiefe") um mindestens 30% abnimmt. Bevorzugt beträgt diese Intensität bei maximaler Eindringtiefe noch höchstens 10% der vorkommenden Maximalintensität.

Um eine gute Lichtexposition möglichst vieler Organismen zu erreichen, können bewegbare Oberflächen samt daran angeordneten Organismen kontinuierlich sukzessive in eine Einstrahlungsposition und wieder hinaus bewegt werden. Damit kann z. B. der Stoffaustausch der Photosynthese unterstützt werden. Auch kann damit eine Photosynthese-Inaktivierung der Organismen vermieden werden, die bei einer ununterbrochenen Lichteinstrahlung auftreten kann.

Das Feuchtigkeitsniveau wird bevorzugt durch Eindüsen von Wassertropfen, Beregnen, Bewässern etc. aufrechterhalten bzw. auf ein gewünschtes Ausmaß eingestellt. Außerdem ist denkbar, die Organismen oder Teile davon zeitweise oder dauerhaft unter Wasser zu bringen.

Für die gesteuerte Einbringung von CO2 in die Innenraumatmosphäre der Vorrichtung können technische Einrichtungen vorgesehen sein, wie sie oben bereits für die wasserpflanzenbasierte Lösung beschrieben wurden.

Ein kontrollierter Luftaustausch zwischen dem Innenraum und der Atmosphäre kann im Rahmen einer Aufrechterhaltung bzw. Einstellung einer vorbestimmten Zusammensetzung der Innenraumatmosphäre nützlich sein. Damit kann z. B. bei der Photosynthese erzeugtes O2 abgeführt werden. Bei einer entsprechenden Luftaustauscheinrichtung sollte eine CO2-Abscheidung vorgesehen sein, um einen unkontrollierten Austritt von CO2 in die Umgebung zu vermeiden. Zu bedenken ist hierbei, dass im Allgemeinen eine im Innenraum erhöhte CO2-Konzentration (im Vergleich zur atmosphärischen CO2-Konzentration) empfehlenswert ist und ein unkontrollierter CO2-Austritt daher oftmals mit einem Effizienzverlust der CO2-Umsetzung einhergehen würde. CO2-Verlust ist weiterhin unerwünscht, da die Vorrichtung zur CO2-Beseitigung dient bzw. betrieben wird. Außerdem ergäbe sich eine Umweltbeeinträchtigung.

Außerdem ist es vorteilhaft, wenn die Luftaustauscheinrichtung mit einer Kühlung versehen ist, um durch Kondensation Wasser zu gewinnen, welches zur Versorgung der Organismen oder zu anderen Zwecken in den Innenraum zurückgeführt werden kann.

In einer bevorzugten Ausführungsform umfasst die Vorrichtung photosynthetisch aktive Bereiche mit verschiedenartiger Besetzung von Organismen. Die Organismen in den einzelnen Bereichen können z. B. in ihrer Art und/oder Konzentration der im betreffenden Bereich vorgesehenen CO2-Konzentration angepasst werden.

In einer bevorzugten Ausführungsform sind die Organismen derart gewählt und/oder sind zusätzlich weitere Organismen einer anderen Art vorgesehen, so dass eine wenigstens teilweise Entstickung eines das CO2 enthaltenden Mediums mittels der Organismen erfolgt. Für die Entstickung können z. B. Mikroorganismen wie z. B. Mikroalgen der Art Dunaliella parva vorgesehen sein.

Ein im Rahmen der vorliegenden Erfindung einsetzbares Verfahren zur Beseitigung von CO2 kann umfassen:
- Photosynthese zur Umsetzung von CO2 mittels einer Vielzahl von photosynthesetreibenden Organismen,
- Aufrechterhaltung vorbestimmter Umgebungsbedingungen für die Organismen mittels einer Umhüllung zur Unterbringung der Organismen in einem Innenraum der Umhüllung,
- Einleitung von Sonnenlicht in den Innenraum,
- gesteuerte Zufuhr von CO2 in den Innenraum,
- gesteuerte Abfuhr von Produkten der CO2-Umsetzung.

Bevorzugt wird dieses CO2-Beseitigungsverfahren unter Verwendung einer Vorrichtung der oben bereits beschriebenen Art durchgeführt.

Sämtliche der oben bereits für die CO2-Beseitigungsvorrichtung beschriebenen Besonderheiten bzw. Weiterbildungen können in analoger Weise, einzeln oder in beliebiger Kombination, auch für das CO2-Beseitigungsverfahren vorgesehen werden.

Zusammenfassend können auf Basis der im Rahmen der Erfindung eingesetzten Vorrichtungen und Verfahren insbesondere großtechnische Einrichtungen realisiert und gezielt eingesetzt werden, um Photosynthese mit einer großen Effektivität und kleinem Ressourcenverbrauch durchführen zu können. Insbesondere können z. B. folgende Vorteile erreicht werden:
- Ein Einsatz in Regionen hoher Sonneneinstrahlung (z. B. Wüsten) führt zu einer hohen Strahlungsintensität mit geringer Schwankung über das Jahr. Dies bedeutet eine Maximierung der Einstrahlung.
- Bei der "Wasserpflanzenlösung" kann eine Einrichtung wie zuvor beschrieben(natürlich und/oder technisch realisiert) mit hoher Dichte an photosynthesetreibenden Organismen ein hohes Nutzungspotenzial durch Anwesenheit einer großen Menge von Organismen liefern. Dies bedeutet eine Maximierung der möglichen Rezipienten. Eine Umwälzung und/oder Verwirbelung ermöglicht einen Ausgleich bzw. eine Kompensation der Stoffaustauschzeiten. Gemeinsam mit vorherigem Punkt bedeutet die Umwälzung eine Maximierung der Exposition der Rezipienten (Entspräche einer Maximierung der Blattoberfläche bei Landpflanzen).
- Bei der "Landpflanzenlösung" können statische oder bewegbare Flächen oder Bauteile mit photosynthesetreibenden Organismen in einer Anordnung zur Unterbringung möglichst vieler Organismen vorgesehen werden. Daraus ergibt sich ein hohes Nutzungspotenzial durch Anwesenheit einer großen Menge von Organismen. Wenn eine kontinuierliche oder sukzessive Bewegung der Organismen in eine Einstrahlungsposition und wieder heraus vorgesehen ist, so ergibt sich ein Ausgleich bzw. eine Kompensation der Stoffaustauschzeiten. Bei Einsatz in Regionen hoher Sonneneinstrahlung bedeutet dies eine Maximierung der Exposition.
- Eine hohe CO2-Konzentration, eine Temperaturführung (insbesondere mit Temperaturbegrenzung) und der Einsatz der Organismen im Wasserbecken ("Wasserpflanzenlösung") bzw. unter Feuchtigkeit ("Landpflanzenlösung") schafft optimale Bedingungen für die Photosynthese. Dies bedeutet eine Maximierung der Photosynthesegeschwindigkeit.
- Eine geeignete Umhüllung (z. B. zumindest Abdeckung/Überdachung), ein Luftaustausch und regelmäßige oder kontinuierliche Abfuhr von Biomasse grenzt die geschaffene "Biosphäre" vorteilhaft nach außen ab. Es kann ein Gleichgewicht von Produktion und Abfuhr der Biomasse geschaffen werden. Damit kann in der Biosphäre eine günstige, insbesondere optimale Zusammensetzung des Innenraummediums(z. B. Konzentrationen von CO2, O2 etc. in Luft bzw. Wasser) aufrechterhalten werden.
- Ein Einsatz photolumineszenter Stoffe zur Wandlung ineffektiverer (z. B. kurzwelliger) Anteile des Sonnenlichtes ermöglicht eine Maximierung des effektiven Anteils des eingestrahlten Lichtes.
- Mit einem Einsatz von Spiegelflächen oder anderen Lichtleiteinrichtungen zur Zuleitung weiteren Sonnenlichts kann eine Erhöhung der Einstrahlung erzielt werden. Bei der "Landpflanzenlösung" kann eine zusätzliche Erhöhung durch eine Nachführung der Organismen (in Abhängigkeit von der momentanen Richtung der Lichteinstrahlung) erzielt werden.
- Eine Entnahme/Trocknung/Weiterverarbeitung der Biomasse macht diese vielfältig nutzbar, z. B. als Rohstoff für Nahrungsmittel oder Futtermittel, mögliches Düngemittel für landwirtschaftliche Zwecke, Grundstoff für Kraftstoffe (ohne Einfluss auf Verfügbarkeit von Nahrungsmitteln wie bei Bioalkohol etc.), Brennstoff für Kraftwerke oder dergleichen mit hoher Homogenität und einstellbarem Trocknungsgrad.

Die Erfindung bietet somit interessante Möglichkeiten für eine nachhaltige Lösung des CO2-Problems, ohne CO2 unterirdisch in geologische Formationen verpressen zu müssen.

Die "Wasserpflanzenlösung" bietet neben der Einstellung eines erhöhten bzw. optimierbaren CO2-Anteils gegenüber einer Bepflanzung mit Landpflanzen den Vorteil, dass massive Verdunstung und damit Austrocknung bei intensivem Lichteinfall in einfacher Weise vermieden werden kann.

Die "Landpflanzenlösung" bietet insbesondere den Vorteil, dass neben der Einstellung eines erhöhten bzw. optimierbaren CO2-Anteils in einfacher Weise Vorkehrungen gegen zu hohe Temperaturen und gegen übermäßige Verdunstung bei intensivem Lichteinfall getroffen werden können.

Weiterhin schafft die Erfindung einfach zu realisierende Möglichkeiten dafür, den Wasserverlust insgesamt zu minimieren. Diesbezüglich wurden vorstehend (und werden nachfolgend) spezifische Ausführungen erläutert. Somit ermöglicht die Erfindung insbesondere einen wirtschaftlichen Einsatz von Photosynthese in Wüstengebieten wie z. B. der Sahara.

Vorteilhaft können mit der Erfindung Flächen genutzt werden, die ansonsten im Wesentlichen als unfruchtbare und somit im Wesentlichen nutzlose Flächen zu betrachten wären. Bei der "Wasserpflanzenlösung" besteht z. B. der besondere Vorteil, dass im Vergleich zu Landpflanzen bei optimalen Verhältnissen ein Mehrfaches an CO2 bei gleichem Flächeneinsatz umgesetzt werden kann. Bei der "Landpflanzenlösung" besteht der besondere Vorteil, dass durch die hierfür beschriebenen Optimierungsmaßnahmen eine höhere Effizienz (in CO2-Umsetzung und/oder Biomasseerzeugung) bei gleichem Flächeneinsatz erreicht werden kann.

Gemäß eines Aspekts der vorliegenden Erfindung wird die eingangs gestellte Aufgabe durch eine Verbrennungsanlage, insbesondere Kraftwerk, umfassend einen Brennraum, insbesondere zur Energieerzeugung durch einen CO2-erzeugenden Verbrennungsprozess, gelöst, welche eine Vorrichtung der oben beschriebenen Art zur wenigstens teilweisen Beseitigung von CO2 aus dem Abgas umfasst (einschließlich damit in Verbindung steht).

In einer bevorzugten Ausführungsform umfasst die Verbrennungsanlage eine steuerbare Luftzufuhreinrichtung zur wahlweisen Einstellung eines Betriebes mit Frischluft und/oder Abluft aus einem photosynthetisch aktiven Bereich.

In einer bevorzugten Ausführungsform umfasst die Verbrennungsanlage eine Luftzufuhreinrichtung, welche einen Betrieb mit zugeführter Luft vorsieht, welche einen gegenüber der Atmosphäre erhöhten CO2-Gehalt aufweist.

Erfindungsgemäß sind der Brennraum und ein photosynthetisch aktiver Bereich in einen im Wesentlichen geschlossenen Gaskreislauf eingebunden.

In einer bevorzugten Ausführungsform wird die aus dem Innenraum abgeführte Biomasse mittelbar oder unmittelbar als Brennstoff oder Brennstoffanteil für den Betrieb der Verbrennungsanlage verwendet.

In einer bevorzugten Ausführungsform sind die abgeführte Biomasse und der dem Verbrennungsprozess zugeführte Brennstoff bzw. Brennstoffanteil Stofferscheinungsformen in einem im Wesentlichen geschlossenen Stoffkreislauf(Biomasse = Brennstoff) .

Gemäß eines weiteren Aspekts der vorliegenden Erfindung wird die eingangs gestellte Aufgabe durch ein Verfahren zum Betreiben einer Verbrennungsanlage, insbesondere eines Kraftwerkes zur Energieerzeugung durch einen CO2-erzeugenden Verbrennungsprozess, gelöst, welches den Schritt einer wenigstens teilweisen Beseitigung von CO2 aus dem Abgas unter Verwendung einer CO2-Beseitigungsvorrichtung der oben beschriebenen Art und/oder eines CO2-Beseitigungsverfahrens der oben beschriebenen Art umfasst.

Die Erfindung ermöglicht somit auch ein im Wesentlichen "CO2-freies Kraftwerk" (insbesondere Großkraftwerk) bzw. einen im Wesentlichen CO2-freien Betrieb eines Kraftwerkes.

Durch die Verwendung einer CO2-Beseitigungsvorrichtung und/oder eines CO2-Beseitigungsverfahrens der hier beschriebenen Art kann insbesondere ein wettbewerbsfähiges Großkraftwerk mit geringem oder nahezu keinem Ausstoß von CO2 in die Atmosphäre geschaffen werden. Der Begriff "Großkraftwerk" soll insbesondere eine Kraftwerkseinrichtung mit einer (maximalen) Energieerzeugungsleistung von mehr als 100 MW, insbesondere mehr als 1000 MW, umfassen.

Zur Begrenzung der CO2-Emissionen wurden bislang Regulierungs- und Begrenzungsmechanismen wie die Ausgabe von Emissionszertifikaten installiert. Insbesondere für Großkraftwerke stellte sich bereits im Stand der Technik die Forderung, entstehendes CO2 abzuscheiden und beispielsweise einer langzeitigen Speicherung zuzuführen ("CCS"), um einer weiteren Erhöhung der atmosphärischen CO2-Konzentration entgegenzuwirken.

Für die Abscheidung von CO2 in Kraftwerken sind zurzeit drei Technologiezweige in der großtechnischen Entwicklung bzw. Erprobung: "Pre-Combustion", "Oxy-Fuel", "Post-Combustion".

Gravierender Nachteil aller bekannten Technologien ist, dass sie mit erheblichen Wirkungsgradeinbußen in der Größenordnung von ca. 10%-Punkten einhergehen. Bei einem modernen Kraftwerksblock mit einem ursprünglichen Wirkungsgrad von beispielsweise 45% und einer Wirkungsgradeinbuße von 9% ergäbe sich ein neuer Wirkungsgrad von 36% - eine Einbuße in der Stromerzeugung von 20%. Dies entspricht bei einem Großkraftwerk mit einer ursprünglichen Leistung von 1000 MW einer Leistungseinbuße von 200 MW - bei gleichem Brennstoffbedarf.

Somit ist festzustellen, dass für das technische Problem der Abscheidung und Speicherung von CO2 aus Kraftwerksprozessen zwar Lösungsansätze bestehen, aber alle bekannten großtechnischen Lösungen mit dem Nachteil massiver Leistungs- und Einnahmeeinbußen verbunden sind.

Demgegenüber vermeidet die vorliegende Erfindung den immensen Energieaufwand für die CO2-Abscheidung. Dies mit Hilfe einer mehr oder weniger unmittelbaren Einleitung des (bevorzugt gereinigten und abgekühlten) Abgases (z. B. Rauchgases) in eine von der Atmosphäre getrennte, photosynthesebasierte CO2-Senke der hier beschriebenen Art.

Mögliche Ausführungsformen in Frage kommender CO2-Senken bzw. deren Funktion wurden oben bereits beschrieben. Sie beruhen auf dem biologischen Prozess der Photosynthese, mittels welchem das CO2 des Abgases in O2 umwandelt werden kann.

Im Vergleich zu bestehenden CO2-freien Kraftwerken oder anderen Verbrennungsanlagen, bei denen hoher Energiebedarf für die großtechnische Abscheidung, Kompression und Verflüssigung des CO2 aufzubringen ist, wird hier die Abscheidung innerhalb der CO2-Beseitigungsvorrichtung durch photosynthesetreibende Organismen vorgenommen. An die Stelle der Einlagerung in geologische Formationen tritt die Beseitigung des CO2 durch Umwandlung in O2 und Biomasse (z. B. Pflanzenmasse).

Wesentliche Energieaufwände wie beim Stand der Technik, etwa zur Kompression des CO2 und zu seiner Aufbereitung, fallen nicht mehr an. Somit lässt sich mit der Erfindung ein CO2-freies Kraftwerk ohne wesentliche Leistungseinbußen realisieren.

Funktionsbedingt herrschen im Abgas typischer Kraftwerke überhöhte CO2-Konzentrationen (im Vergleich zur Atmosphäre). Dies kommt durch die Erfindung dem Prozess der Photosynthese zugute, der in der Regel bei erhöhten CO2-Konzentration sein Optimum besitzt (jedoch abhängig von der eingesetzten Art der photosynthesetreibenden Organismen, z. B. Chlorella minutissima) .

Eine jeweils günstige wie auch jeweils maximal zulässige CO2-Konzentration richtet sich nach der Art der photosynthesetreibenden Organismen und ihren Umgebungsbedingungen. Eine Schädigung der Organismen kann durch geeignete Wahl der CO2-Konzentration und der Randbedingungen verhindert werden (auch: Optimierung der Photosynthese-Aktivität).

Als mit dem betreffenden Kraftwerk zu koppelnde CO2-Senke kommt beispielsweise eine großflächig überdachte Wald-, Agrar- oder Grünfläche in Frage, die eine gegenüber der Umgebung erhöhte CO2-Konzentration enthalten bzw. aufnehmen kann.

Weiterhin kommt eine große Wasserfläche bzw. ein großes Wasserreservoir in Frage, welches photosynthesetreibende Organismen enthält, und in welches in gesteuerter Weise eine bestimmte (relativ hohe) Konzentration von CO2 eingebracht werden kann.

Hier könnte sogar die begrenzte Übertrittsgeschwindigkeit des CO2 aus dem Wasser in die Luft eine gewisse Abtrennung von der Atmosphäre gewährleisten, so dass die Wasseroberfläche selbst als "Umhüllung" der oben erwähnten Art genutzt werden kann.

Es versteht sich, dass hinsichtlich der konkreten Ausgestaltung der von dem betreffenden Kraftwerk (oder anderen Verbrennungsanlage) umfassten (einschließlich "damit gekoppelten") CO2-Beseitigungsvorrichtung vorteilhaft auf die oben bereits beschriebenen Ausgestaltungen und besonderen Maßnahmen zurückgegriffen werden kann.

Insbesondere kommt eine großtechnisch unterstützte CO2-Senke in Frage. Die gebildete CO2-Beseitigungsvorrichtung kann z. B. eine flächenmäßige Ausdehnung von mehr als 10¹ km², insbesondere mehr als 10² km² besitzen.

Nachfolgend werden beispielhaft einige vorteilhafte Ausgestaltungen von erfindungsgemäßen Kraftwerken bzw. Betriebsverfahren hierfür erläutert. Es versteht sich, dass viele der erläuterten Ausgestaltungsdetails (soweit die CO2-Beseitigungsvorrichtung als solche betreffend) auch bei isolierter Verwendung der CO2-Beseitigungsvorrichtung, und auch bei Verwendung in Kombination mit andersartigen Verbrennungsanlagen, vorteilhaft einsetzbar sind:
- Überdachte Wald-, Agrar- oder Grünfläche ("landpflanzenbasierte Lösung"): Hier kommt eine Leichtbauüberdachung in Frage, die z. B. nach Art einer Traglufthalle gestaltet sein kann (Eine Folie wird durch einen leicht erhöhten Innendruck vom Boden abgehoben). Durch eine geeignete Auswahl und Gestaltung der Folie (z. B. lichtdurchlässiger Kunststoff) kann das einfallende Sonnenlicht möglichst ungeschwächt in die CO2-Senke eintreten. Als alternative Bauweise sind auch übliche Hallen in einfacher (bevorzugt abgedichteter) Bauart denkbar, z. B. als Stahl- oder Stahlbetontragwerk mit einem ausreichenden Anteil lichtdurchlässiger Elemente, z. B. aus Glas, Plexiglas oder anderen lichtdurchlässigen Kunststoffen. Die Einbringung von CO2 erfolgt bevorzugterweise in gasförmiger Form durch Einleitung des gereinigten Rauchgases in den Innenraum der Vorrichtung, oder in Form einer wässrigen Lösung. Die wässrige Lösung kann beispielsweise erzeugt werden, indem Wasser in einen intensiven Kontakt mit dem CO2-haltigen Rauchgas gebracht wird (Eindüsung feiner Wassertröpfchen in das Rauchgas). Zur Verbesserung der Durchführung der Photosynthese und des Wachstums können den Organismen Nährstoffe und Düngemittel geeigneter Art und Menge zugeführt werden. Zur Bekämpfung etwaiger Pflanzenkrankheiten oder von Schädlingsbefall können jeweils geeignete Pflanzenschutzmittel eingebracht werden. Weiterhin weisen die oben genannten Flächen bevorzugterweise zumindest teilweise einen geeigneten Boden für das Wachstum der Organismen auf.
- Große Wasserfläche bzw. großes Wasserreservoir ("wasserpflanzenbasierte Lösung"): Hier wird die Photosynthese durch Organismen durchgeführt, welche sich zumindest größtenteils im Wasser, am Grund oder an Ufern befinden. Die Trennung der erhöhten CO2-Konzentration von der Umgebung kann wie bei überdachten Wald- oder Grünflächen (siehe oben) erfolgen. Weiter kann, falls das CO2 bereits im Wasser gelöst ist, und falls die erreichte Photosynthesegeschwindigkeit die (begrenzte) Übertrittsgeschwindigkeit des CO2 vom Wasser zur Luft übersteigt, auf eine gesonderte aufwendige Umhüllung bzw. Abdeckung/Trennung verzichtet werden. Vorteilhafterweise wird die Trennung der erhöhten CO2-Konzentration durch eine auf der Wasseroberfläche (weitgehend) aufliegende, für Sonnenlicht durchflutbare flächige Komponente (z. B. Folienanordnung oder dergleichen) erzielt. Durch die direkte Auflage wird eine Windbeanspruchung vermieden. Das CO2 kann beispielsweise durch Verdüsen von Wassertröpfchen im Rauchgas gelöst werden. Das CO2-aufgereicherte Wasser kann dann an verschiedenen Orten des Wasserreservoirs eingepumpt werden, um eine zweckmäßige Verteilung zu erreichen. Alternativ kann dem Wasserreservoir ein mit CO2 angereichertes Medium zugeführt werden. In einer weiteren Alternative kann Rauchgas oder ein CO2-haltiges Gasgemisch mit dem Wasser in Kontakt gebracht werden (z. B. durch Gaspolster über dem Wasser, Eindüsen von Gasblasen in das Wasser). Um eine gute Exposition aller Organismen zu erreichen, kann der Inhalt des Wasserreservoirs kontinuierlich umgewälzt oder verwirbelt werden. Dies unterstützt auch den Stoffaustausch der Photosynthese. Die Umwälz- oder Bewegungsgeschwindigkeit sollte dabei so begrenzt werden, dass die Organismen nicht geschädigt werden. Zur Verbesserung der Photosynthese können den Organismen Nährstoffe und Düngemittel geeigneter Art und Menge zugeführt werden. Auch können jeweils geeignete Schutzmittel (z. B. Pflanzenschutzmittel) eingebracht werden.
- Großtechnische CO2-Senken der oben als CO2-Beseitigungsvorrichtungen bereits beschriebenen Art: Ihre wesentliche Funktion besteht wie bei den anderen CO2-Senken darin, CO2 aus dem Rauchgas auf photosynthetischem Weg zu beseitigen. Ihnen liegt jeweils das Bestreben zugrunde, die Effektivität der Photosynthese mittels großtechnischer Einrichtungen zu erhöhen und den Ressourcenverbrauch (Landverbrauch) zu reduzieren.
- Die CO2-Senken können vorteilhafterweise in Form einzelner Abschnitte oder Zellen ausgeführt werden, um Störungen und Beeinträchtigungen auf einzelne Bereiche begrenzen zu können, und um außerdem die Stoffumsetzung optimieren zu können. Die Versorgung der Zellen mit Rauchgas/CO2 oder CO2-haltigem Medium (z. B. CO2-aufgereichertes Wasser) kann dabei in Form einer Reihen-, Parallelschaltung oder alternierend/intermittierend erfolgen. Reihenschaltung: Rauchgas/CO2 /Medium wird von einer Zelle in die nächste geleitet; die CO2-Konzentration nimmt von Zelle zu Zelle ab. Eine Optimierung der Zellen und/oder Spezialisierung der Organismen auf die jeweilige CO2-Konzentration ist möglich. Parallelschaltung: Alle Zellen werden in gleichem Maße mit CO2 beaufschlagt; alle Zellen besitzen (etwa) die gleiche CO2-Konzentration und die gleiche CO2-Beseitigungsaufgabe. Alternierende/intermittierende Beaufschlagung: Den Zellen wird eine gewisse Menge CO2 zugeführt, und sie führen die CO2-Beseitigungsaufgabe über einen Zeitraum durch, bevor eine erneute Einspeisung erfolgt. In der einspeisungsfreien Zeit einer Zelle wird in andere vorhandene Zellen eingespeist (oder Rauchgas/CO2 wird eingespeichert und/oder anderen Einrichtungen zugeführt und/oder an die Umgebung abgegeben - intermittierende Beaufschlagung). Beliebige Kombinationen der Schaltungsarten sind möglich und können aus den biologischen Erfordernissen der eingesetzten photosynthesetreibenden Organismen vorteilhaft sein.
- In industrialisierten bzw. dicht besiedelten geographischen Gebieten besteht kaum ausreichender Platz für Kraftwerksanlagen mit ausreichend dimensionierten CO2-Senken (für einen Kraftwerksblock mit einer Leistung von 1000 MW wären je nach Effektivität und Ausführung der CO2-Senke Flächen zwischen 100 und 10000 km² erforderlich). Daher wird vorgeschlagen, die beschriebenen Kraftwerke mit der CO2-Beseitigungsvorrichtung in eher wenig besiedelten Gebieten mit hoher Sonneneinstrahlung und niedrigen Landkosten (z. B. Wüsten) zu installieren. Bei hohen vorherrschenden Tagestemperaturen und starken Temperaturschwankungen ist hier zur Erreichung einer optimalen Temperatur für die Photosynthese eine Stabilisierung der Temperatur und/oder eine aktive Temperaturführung und/oder optional eine Kühlung zweckmäßig.
- Bei der landpflanzenbasierten Lösung kann dies durch Einbringung ausreichend hoher Wärmekapazität (z. B. große Wassermassen), durch Verdunstungskühlung (hohe Wasseroberfläche, z. B. durch kontinuierliche Verdüsung von Wassertropfen), durch Abdeckung von Teilflächen durch Blenden/Spiegel oder ähnliches, sowie durch aktive Kühlung (Klimaanlagen/Kühlanlagen/Rückkühlanlagen) erreicht werden. Zur Sicherstellung eines ausreichenden Feuchtigkeitsniveaus für die Photosynthese sollte eine ausreichende Luftfeuchtigkeit sichergestellt werden. Erforderlichenfalls können zur weiteren Befeuchtung Wassertröpfchen eingedüst werden, weiterhin kann eine Beregnung und/oder Bewässerung vorgesehen werden. Es ist auch denkbar, die Organismen oder Teile davon zeitweise oder dauerhaft unter Wasser zu bringen.
- Bei der wasserpflanzenbasierten Lösung trägt bereits die Wärmekapazität des Wassers zum Ausgleich der täglichen Temperaturschwankungen bei. Soweit erforderlich können Details wie bei der landpflanzenbasierten Lösung zusätzlich verwendet werden.
- Für überdachte/abgedeckte Bereiche der CO2-Senken ist ein kontrollierter Luftaustausch für die geregelte Abfuhr des erzeugten O2 vorteilhaft (z. B. mittels eines oder mehrerer Austauschkanäle). Zur Verbesserung der Effektivität können photolumineszente Stoffe zur Wandlung von für die Photosynthese ungünstigen Bestandteilen der Sonnenstrahlung in photosynthetisch aktive Strahlung verwendet werden. Die photolumineszenten Stoffe können so ausgewählt werden, dass sich eine möglichst günstige Wandlung ergibt (z. B. Wandlung von Lichtquanten aus dem UV-Bereich in weniger energiereiche Lichtquanten im sichtbaren Spektrum, welche einen höheren Photosynthese-Wirkungsgrad aufweisen).
- Zur Aufrechterhaltung des Gleichgewichtes zwischen Erzeugung von Biomasse und ihrer Entnahme kann ein im Wesentlichen kontinuierlicher Abzug von Biomasse vorteilhaft sein.
- Bei der landpflanzenbasierten Lösung kann die Biomasse mittels technischer Einrichtungen entnommen (geerntet/gemäht/gefällt/gerodet), erforderlichenfalls transportiert, zerkleinert, aufbereitet oder weiterverarbeitet werden.
- Bei der wasserpflanzenbasierten Lösung kann die Biomasse durch technische Einrichtungen zur Entnahme, Trocknung, Weiterverarbeitung entnommen und weiterverarbeitet werden. Durch eine im Wesentlichen kontinuierliche Entnahme wird auch eine Verschlammung bzw. ein Zusetzen des Wasserbeckens bzw. der die Organismen aufnehmenden Einrichtung vermieden.
- Die entnommene Biomasse kann verschiedenen Nutzungen zugeführt werden, beispielsweise: Nahrungs- oder Futtermittel, Düngemittel, Grundstoff für Kraftstoffe, erneuter Kraftwerksbrennstoff. Im Falle einer der erstgenannten Verwendungen könnte durch die Schaffung großer hochwertiger Flächen für Land- oder Wasserpflanzen ein großer zusätzlicher Ertrag erwirtschaftet werden. Im Falle der letztgenannten Verwendung (Kraftwerksbrennstoff) ergibt sich ein weiteres immenses Nutzenpotenzial. Es müsste für den Betrieb des Kraftwerkes kein oder nur noch wenig fossiler Brennstoff verbrannt werden, vielmehr können im wahrsten Sinne nachwachsende Rohstoffe (die durch die geschaffene Anlage selbst mit Hilfe des einfallenden Sonnenlichtes erzeugt werden) als Kraftwerksbrennstoff verwendet werden. Dabei handelt es sich um die gezielte Nutzung der auf die CO2-Senke einfallenden Sonnenenergie; diese wird durch Photosynthese in chemische Energie in Form von Biomasse umgesetzt, und kann im Kraftwerksprozess mit einem Wirkungsgrad von beispielsweise 45% in elektrische Energie umgewandelt werden.
- In Hinblick auf den Stoffstrom Biomasse/Brennstoff kann vor einer Verwendung der Biomasse als Kraftwerksbrennstoff ein geeigneter Aufbereitungs- bzw. Trocknungsprozess vorgeschaltet werden.
Für die "landpflanzenbasierte Lösung" kommen z. B. Aufbereitungsmethoden in Betracht, wie sie im Wesentlichen aus dem Stand der Technik bekannt sind. Für die Trocknung kommt, soweit überhaupt erforderlich, bevorzugt eine Trocknung in einem umschlossenen Trocknungsbereich zum Einsatz. Dazu wird die Biomasse z. B. in einem Trocknungsbereich ausgebreitet und der Sonneneinstrahlung ausgesetzt. Die Abfuhr des infolge Verdunstung entstehenden Wasserdampfes erfolgt z. B. durch trockenes Rauchgas, welches (weitgehend abgekühlt) den Trocknungsbereich überstreicht und Wasserdampf bis zu einer Sättigung aufnehmen und abführen kann.

Für die "wasserpflanzenbasierte Lösung" kann als erster Schritt (nach dem Abzug der Biomasse aus dem photosynthetisch aktiven Bereich) z. B. eine Eindickung/Entwässerung der Biomasse mit Hilfe von bekannten Verfahren der mechanischen Verfahrenstechnik (z. B. Pressen etc.) erfolgen. Hierzu kommen Einrichtungen wie "Drainbelts", Siebbandpressen und/oder Excenterschnecken zum Einsatz. Um die Handhabung der entwässerten Biomasse zu verbessern, kann (z. B. mit Hilfe von Doppelwellenmischern) eine Zumischung von bereits getrockneter Biomasse erfolgen.

Darüber hinaus ist eine weitere Trocknung vorteilhaft, welche zum weit überwiegenden Teil durch eine solare Wärmezufuhr in einem umschlossenen Trocknungsbereich erfolgt. Dazu wird die Biomasse z. B. in einem Trocknungsbereich (in mechanisch entwässertem Zustand) ausgebreitet und der Sonneneinstrahlung ausgesetzt. Die Abfuhr des infolge Verdunstung entstehenden Wasserdampfes erfolgt z. B. durch trockenes Rauchgas, welches (weitgehend abgekühlt) den Trocknungsbereich überstreicht und Wasserdampf bis zu einer Sättigung aufnehmen und abführen kann. Das nun feuchte Rauchgas strömt der CO2-Beseitigungsvorrichtung bzw. einem Austauschkanal zu, und trägt die Feuchtigkeit in die Vorrichtung bzw. den Austauschkanal zurück.

Sollte das Rauchgas infolge der Verbrennung feuchter Brennstoffe und/oder der Verbrennung von Brennstoffen mit hohem Wasserstoffanteil (Bildung von H2O) nicht trocken genug für eine ausreichende Abfuhr von Wasserdampf sein, kann diese Transportkapazität durch Entnahme und Rezirkulation von teilgereinigtem Rauchgas aus der Vorrichtung (z. B. aus dem Austauschkanal) erfolgen. Durch die im Vergleich zur Einrichtung vorliegende erhöhte Temperatur in der Trocknungseinrichtung kann dieses Gas weiteren Wasserdampf aufnehmen, selbst wenn es der Einrichtung im Wesentlichen gesättigt entnommen wird.
- Für den Wasserhaushalt der Gesamtanlage ist es als wichtig zu betrachten, dass bei etwaigen Trocknungsvorgängen der erzeugten Biomasse anfallendes Wasser aufgefangen und dem Kreislauf wieder zugeführt wird. Das Gleiche gilt für anfallenden Wasserdampf, welcher kondensiert und ebenfalls wieder zugeführt werden sollte. Weiterhin kann anfallender Dampf im Rauchgas (z. B. aus Verbrennung von gewonnener Biomasse oder von wasserstoffhaltigen Brennstoffen) der Gesamtanlage durch Kondensation oder durch direkte Einleitung in die CO2-Senken zugänglich gemacht werden.
- Die Abluft der CO2-Senken kann vorteilhafterweise der Kraftwerksanlage als Verbrennungsluft ganz oder teilweise wieder zugeführt werden. Dies bietet den Vorteil, dass auch ein etwas erhöhter CO2-Anteil in der Abluft zulässig sein kann, und dass der Teil der CO2-Beseitigung, der aufgrund geringer CO2-Konzentration mit geringerer Geschwindigkeit abläuft, eingespart werden kann. Damit ergibt sich eine Einsparung der erforderlichen Gesamtfläche.
- In einer vorteilhaften Ausgestaltung der Kraftwerksanlage ist diese über eine Hochspannungs-Gleichstrom-Übertragung (HGÜ), eine für weite Entfernungen besonders geeignete Übertragungseinrichtung, mit den Verbrauchern verbunden.

Zusammenfassend kann auf Basis des erfindungsgemäßen Kraftwerkes bzw. Betriebsverfahrens hierfür eine nachhaltige Lösung des Energie-Problems erfolgen, indem insbesondere in sonnenreichen Breiten einfallende Energie mit Hilfe wettbewerbsfähiger Technologien großtechnisch in elektrische Energie umgewandelt werden kann. Eine nachhaltige, überaus wettbewerbsfähige Versorgung mit CO2-neutraler elektrischer Energie wird möglich.

Eine bekannte Möglichkeit zur Beseitigung des CO2 besteht wie eingangs bereits erläutert in der Speicherung in großen unterirdischen Formationen. Alternativ zur Speicherung von CO2 ("CCS") können CO2-Emissionsrechte auch mit Hilfe des "Clean Development Mechanism" (CDM) durch Emissionsreduzierung in Entwicklungsländern und durch Transfer von umweltfreundlicher Technologie in Entwicklungsländer erworben werden. Weiterhin wird unter CDM die Schaffung von CO2-Senken (z. B. Aufforstung) durch Emissionsrechte vergütet.

Vor diesem Hintergrund ist offensichtlich, dass ein geeignetes Geschäftsmodell zum Betrieb einer Vorrichtung für die Beseitigung von CO2, insbesondere einer großtechnischen CO2-Senke, ausgesprochen attraktiv wäre und wirtschaftlich äußerst hohes Potenzial bieten würde. Weiterhin würde durch ein solches Geschäftsmodell ein erheblicher Nutzen für die Umwelt gestiftet und der globalen Erwärmung entgegengewirkt.

Daher ist ein Verfahren zur wirtschaftlichen Nutzung einer CO2-Beseitigunginteressant, umfassend die Errichtung und den Betrieb einer CO2-Senke, insbesondere in einer geographischen Lage mit hoher Sonneneinstrahlung und/oder niedrigen Landkosten (z. B. in einer Wüste).

Das Verfahren kann folgende (nicht zwangs-läufig sequenzielle) Schritte umfassen:
- Planung und Errichtung einer CO2-Senke, insbesondere einer CO2-Beseitigungsvorrichtung der oben bereits beschriebenen Art
- Generierung bzw. Annahme von Aufträgen zur Beseitigung von CO2
- Zufuhr von CO2 in die CO2-Senke
- Betrieb der CO2-Senke, bevorzugt umfassend ein Messen zum Nachweis der CO2-Beseitigungsleistung
- Geltendmachung der CO2-Beseitigungsleistung. Eine "Geltendmachung" kann z. B. die Einforderung entsprechender gesetzlicher Prämien darstellen, aber auch z. B. eine aufgrund der CO2-Beseitigungsleistung gerechtfertigte Minderung oder Nichtzahlung ansonsten fälliger gesetzlicher Abgaben.

Das Verfahren kann ferner einen oder mehrere der folgenden Schritte umfassen:
- Vermarktung der im Betrieb der CO2-Senke gewonnenen Biomasse
- Optimierung der CO2-Senke

Der neuen technisch-wirtschaftlichen Lösung liegt die Idee zugrunde, eine effektive und kostengünstige Beseitigung des CO2 durch eine bevorzugt großtechnisch unterstützte Anwendung der Photosynthese zu verwirklichen. Dabei können die biologischen Prozesse der Photosynthese durch technische Einrichtungen positiv beeinflusst werden, so dass Effektivität und Ressourcenverbrauch (Landverbrauch) optimiert werden können und ein überaus positives Geschäftsmodell erreicht werden kann. Zu bedenken ist hierbei, dass insbesondere für die Betreiber von Großkraftwerken oder anderer Verbrennungsanlagen die Beseitigung von abgeschiedenem CO2 ein in naher Zukunft konkret anstehendes Problem darstellt. Sequestrierung als Stand der Technik ist für die Beseitigung von CO2 kostenintensiv und kann durch die neue Lösung unterboten werden.

Nachfolgend werden für die einzelnen der oben genannten Schritte des Nutzungsverfahrens jeweils weitere bevorzugte Weiterbildungen erläutert.
- Planung und Errichtung der CO2-Senke:
   Dieser Schritt kann z. B. einen oder mehrere der folgenden Maßnahmen beeinhalten:
   - Beschaffung von Landnutzungsrechten und Betriebsgenehmigungen in geographischen Breiten mit hoher Sonneneinstrahlung und niedrigen Landkosten. Dies bevorzugt in ausreichender Größe, etwa für die Beseitigung zumindest einer produzierten CO2-Menge eines 100 MW-, insbesondere 1000 MW-Kraftwerkes. Dafür sind je nach Ausführung der CO2-Senke etwa 10 bis über 1000 km² erforderlich.
   - Errichtung einer CO2-Beseitigungsvorrichtung der oben bereits beschriebenen Art als CO2-Senke, mit Schaffung einer biologischen "Erstausstattung". Eine solche Anlage kann mit beliebigen photosynthesetreibenden Organismen ausgestattet werden, solange diese hinsichtlich hoher Photosyntheseleistung und hoher Biomasseerzeugung geeignet sind und für diese Organismen vorteilhafte Umweltbedingungen geschaffen werden können. Es kommen im Wesentlichen Module von CO2-Beseitigungsvorrichtungen mit den oben bereits beschriebenen Besonderheiten und Wirkungsweisen in Frage (z. B. "wasserpflanzenbasierte Lösung" oder "landpflanzenbasierte Lösung"), welche einzeln oder in artgleichen oder artverschieden Kombinationen verwendet werden können.
- Generierung bzw. Annahme von Aufträgen zur Beseitigung von CO2:
   Dieser Schritt kann z. B. durch "Aufkauf" von CO2 zur Beseitigung (Auftragsart "A"), oder Annahme eines Auftrages zur Beseitigung von CO2 gemäß rechtlicher Besimmungen einer Regierung, wie z. B. CDM (Auftragsart "B"), ggf. auch ohne tatsächlichen Antransport des CO2 realisiert werden.

Die Beauftragung kann auch, falls es sich um einen Bestandteil einer Firma oder Firmengruppe handelt, firmenintern erfolgen. Maßgeblich ist in diesem Fall, dass sich durch die CO2-Beseitigung ein monetärer oder anders quantifizierbarer Nutzen für die Firma oder Firmengruppe einstellt. Die Beauftragung kann sowohl direkt als auch indirekt, z. B. über Zwischenhändler oder Agenten erfolgen.
- Zufuhr von CO2 in die CO2-Senke:
   Je nach Art des Auftrages kann dies einen Transport des CO2 zur CO2-Senke, die Vereinnahmung und Zuführung des CO2 zur CO2-Senke umfassen. Beispielsweise wenn die Beseitigung von CO2 gemäß CDM Vertragsbestandteil ist, so kann CO2 alternativ oder zusätzlich auch unmittelbar am Ort der CO2-Senke aus der Atmosphäre entnommen und der CO2-Senke zugeführt werden.
- Betrieb der CO2-Senke:
   Die CO2-Senke und deren Betrieb können insbesondere zur Umsetzung von CO2 und H2O zu O2 und Biomasse ausgelegt sein. Der Schritt des Betriebes der CO2-Senke kann z. B. einen oder mehrere der folgenden Maßnahmen umfassen:
   - Schaffung und Aufrechterhaltung der technisch-biologischen Voraussetzungen für einen kontinuierlichen Betrieb. Hierzu kann z. B. vorgesehen sein: Einstellung einer geeigneten CO2-Konzentration (für "A") oder eines geeigneten Durchsatzes bzw. einer geeigneten Austauschrate (für "B"); Aufrechterhaltung eines kontinuierlichen oder intermittierenden Stoffaustausches von CO2 und O2; Aufrechterhaltung einer vorteilhaften Temperaturführung der Anlage zur Verbesserung bzw. Optimierung der Photosynthese; Sicherstellung eines verlustarmen Wasserhaushaltes (sorgsame Kapselung der Anlage, Trocknung der Biomasse und/oder Wasserrückführung); Düngung, Nährstoffzuführung, biologische Zustandsüberwachung und Zuführung von Pflanzenschutzmitteln nach Bedarf; Entnahme und zweckmäßige Aufbereitung, und ggf. Weiterverarbeitung erzeugter Biomasse; Steuerung und Überwachung, Inspektion, Wartung, Instandsetzung der Anlage.
   - Schaffung und Aufrechterhaltung der personellen und personenbezogenen Voraussetzungen für einen kontinuierlichen Betrieb. Hierzu kann z. B. vorgesehen sein: Mitarbeiter für die Steuerung, Überwachung und Wartung der biologischtechnischen Anlagen; Mitarbeiter für die Entnahme, Aufbereitung und ggf. Weiterverarbeitung der Biomasse und für deren Transport, Handhabung und Zuführung von Betriebs- und Hilfsstoffen; Mitarbeiter für die wissenschaftlichbiologische Überwachung und Führung der Anlage; Geeignete gefährdungbezogene Arbeitsschutzmaßnahmen; Schaffung eines Schutzes gegen unbefugtes Eindringen in die Anlage oder in Teile davon; Schaffung geeigneter Unterkünfte und Infrastruktur.
   - Schaffung und Aufrechterhaltung der wirtschaftlichen Voraussetzungen für einen kontinuierlichen Betrieb. Hierzu kann z. B. vorgesehen sein: Schaffung erforderlicher Nachweismöglichkeiten für den erfolgten CO2-Umsatz und der Möglichkeiten einer externen Überprüfung; Qualifizierung und/oder Zertifizierung der Unternehmung für CO2-Abscheidung bzw. -beseitigung; Minimierung oder Vermeidung ungewollter CO2-Leckagen; Auswahl geeigneter Biomasse zur Erzeugung, unter Abwägung der Parameter Photosyntheseleistung vs. Flächenbedarf, Robustheit gegenüber den spezifischen Umgebungsbedingungen, erzielbarer Marktpreis und Absatzmarkt; Beschaffung und Bereithaltung erforderlicher Hilfs- und Betriebsstoffe sowie technischer Ausrüstungsgegenstände und/oder Ersatzteile.
- Geltendmachung der CO2-Beseitigungsleistung, und gegebenenfalls Nachweis einer solchen Geltendmachung:
   Dieser Schritt kann z. B. eine in regelmäßigen Zeitabständen erfolgende Rechnungslegung umfassen.
- Vermarktung der im Betrieb der CO2-Senke gewonnenen Biomasse:
   Dieser Schritt kann z. B. einen Vertrieb der Biomasse z. B. als Nahrungs- oder Futtermittel, Düngemittel, Grundstoff für Biokraftstoffe und/oder Kraftwerksbrennstoff umfassen.
- Optimierung der CO2-Senke:
   Bei diesem Schritt können, bevorzugt kontinuierlich, eine oder mehrere der folgenden Optimierungsarten vorgesehen sein:
   - Wirtschaftliche Optimierung, wie z. B.: Optimierung der CO2-Beseitigungserlöse; Optimierung der Organismenauswahl (Art, Menge, Markt); Optimierung der Vermarktungserlöse der Biomasse oder Biomasseprodukte.
   - Technische Optimierung, wie z. B.: Optimierung der technischen Einrichtungen und Abläufe; Optimierung der Produktivität, Optimierung des Ressourceneinsatzes (z. B. elektrischer Eigenbedarf, Wasserverbrauch); Optimierung der Biomasseentnahme, -aufbereitung, -weiterverarbeitung.
   - Biologische Optimierung, wie z. B.: Einsatz der photosynthesetreibenden Organismen (Art, Menge); Optimierung der Prozessparameter und Randbedingungen; Optimierung der biologisch-technischen Anlagen in biologischer Hinsicht; Durchführung von Labor- und Feldversuchen zu Photosyntheseleistung und Biomasseerzeugung; Durchführung von voreilenden Versuchen (Vorhersage der biologischen Entwicklung) und Szenario-Versuchen; Erstellung und Aufbewahrung biologischer Proben von bewährten bzw. besonders geeigneten Stämmen von Organismen (für Wiedereinsatz oder Neuausbringung).

Mit den beschriebenen optionalen Weiterbildungen kann ein besonders erfolgreiches, ökologisch wie ökonomisch nachhaltiges Geschäftsmodell erreicht werden.

Vorteilhaft kann das Geschäftsmodell den Erwerb von CO2 oder CO2-Äquivalenten zu Entsorgungszwecken umfassen, und adressiert damit insbesondere ein anstehendes, konkretes Problem von z. B. Kraftwerksbetreibern. Es zeigt eine großtechnisch durchführbare Beseitigungsmöglichkeit für CO2 auf. Damit ergibt sich ein Marktpotenzial, welches in mehrstelliger (EUR-) Millionenhöhe einzuschätzen ist.

In einer bevorzugten Weiterbildung des Verfahrens zur wirtschaftlichen Nutzung einer CO2-Beseitigung wird vorgeschlagen, den Betrieb der CO2-Senke zur Beseitigung von unmittelbar aus einer Verbrennungsanlage (z. B. Großfeuerungsanlage oder Kraftwerk) stammenden CO2 durchzuführen, wobei die CO2-Senke und die Verbrennungsanlage bevorzugt "räumlich miteinander kombiniert" werden, also die CO2-Senke z. B. unmittelbar benachbart zu dem Kraftwerk errichtet wird bzw. das Kraftwerk und die CO2-Senke eine bauliche Einheit bilden.

Hinsichtlich Aufbau und Betriebsweise der CO2-Senke können hierbei sämtliche der oben bereits beschriebenen Ausgestaltungen und Weiterbildungen vorgesehen sein. Insbesondere kann die Errichtung und der Betrieb eines Großkraftwerkes mit einer verbundenen großtechnischen CO2-Senke in Breiten mit hoher Sonneneinstrahlung und niedrigen Landkosten vorgenommen werden.

Dieser Weiterbildung liegt die zentrale Idee zugrunde, dass ein Kraftwerk oder eine andere Verbrennungsanlage in hervorragender Weise in einen integrierten Kreislauf der CO2-Erzeugung und seiner Beseitigung direkt eingebunden werden kann. Somit kann eine aufwändige Abscheidung von CO2 entfallen, da z. B. ein CO2-haltiges Rauchgas direkt (bevorzugt jedoch nach so genannter Rauchgasreinigung) der CO2-Senke zugeführt werden kann. Gleichzeitig mit der CO2-Beseitigung erfolgt die Schaffung neuer Biomasse als Brennstoff und neuen Sauerstoffes zur Verbrennung.

Anstelle einer technischen Rauchgasreinigung kann diese ebenfalls auf biologischem Wege erfolgen. Es ist bekannt, dass Stickoxide (NOx) von bestimmten Organismen abgebaut werden können. Dies kann durch Beigabe betreffender Organismen in bestimmten Bereichen oder in der gesamten CO2-Beseitigungseinrichtung bewirkt werden.

In Hinblick auf den Stoffstrom Biomasse/Brennstoff sollte vor einer Verwendung der Biomasse als Brennstoff ein geeigneter Aufbereitungs- bzw. Trocknungsprozess vorgeschaltet werden, z. B. wie oben bereits beschrieben (z. B. mit Eindickung/Entwässerung der Biomasse, weitere Trocknung durch solare Wärmezufuhr, Abfuhr entstehenden Wasserdampfes z. B. mittels (trockenem) Rauchgas etc.)

In einer bevorzugten Weiterbildung wird eine gegenüber der Atmosphäre erhöhte CO2-Konzentration in dem zwischen Verbrennungsanlage und CO2-Beseitigungseinrichtung zirkulierenden Stoffstrom eingestellt.

Die vorliegende Erfindung stellt insbesondere auch die Grundlage für ein Geschäftsmodell zum Betrieb eines wettbewerbsfähigen Großkraftwerkes ohne wesentlichen Ausstoß von CO2 in die Atmosphäre bereit. Dieses Geschäftsmodell zum kostengünstigen Betrieb eines CO2-freien Großkraftwerkes besitzt ein immenses Geschäftspotenzial.

Die bisher verfolgte Abscheidung von CO2 aus dem Rauchgas und seine Sequestrierung als Stand der Technik sind sehr kostenintensiv und wirkungsgradschädlich und können durch die Weiterbildung der Erfindung weit unterboten werden.

Die Weiterbildung kann folgende (nicht zwangsläufig sequenzielle) Schritte bzw. Besonderheiten umfassen:
- Planung und Errichtung eines Kraftwerkes (oder einer anderen Verbrennungsanlage) mit einer verbundenen CO2-Senke, insbesondere mit einer CO2-Beseitigungsvorrichtung der oben bereits beschriebenen Art
- Beschaffung von Landnutzungsrechten und Betriebsgenehmigungen
- Errichtung und Inbetriebsetzung des Kraftwerkes und der CO2-Senke und Schaffung eines Brennstoffvorrates sowie einer biologischen "Erstausstattung", sowie Schaffung einer Leitungsverbindung zu den Abnehmern der elektrischen Leistung (vorzugsweise Hochspannungs-Gleichstrom-Übertragung ("HGÜ")).
- Betrieb des Kraftwerkes zur Erzeugung elektrischer Leistung, Rauchgasreinigung, Zuführung des gereinigten Rauchgases zur CO2-Senke bzw. den darin verwendeten photosynthesetreibenden Organismen. Das Kraftwerk kann z. B. zunächst mit dem angelegten Brennstoffvorrat betrieben, um mit dem entstehenden CO2 eine hinreichende Menge von Biomasse für den späteren Brennstoffkreislauf erzeugen zu können. Die erforderlichen bzw. zweckmäßigen Schritte und Maßnahmen für die Schaffung und die Aufrechterhaltung eines kontinuierlichen Kraftwerksbetriebes an sich sind dem Fachmann wohlbekannt und können vorteilhaft auf den hier vorgesehenen Betrieb angewendet werden. Abweichend vom üblichen Kraftwerksbetrieb kommen folgende Aspekte zum Tragen:
   - Bevorzugterweise wird aus der CO2-Senke entnommene und geeignet aufbereitete Biomasse als Brennstoff für das Kraftwerk verwendet. Damit kann vorteilhaft ein Brennstoffkreislauf realisiert werden, welcher seine Energie aus dem auf die CO2-Senke einfallenden Sonnenlicht erhält.
   - Weiter wird bevorzugterweise Luft aus der CO2-Senke (mit einem hinreichend niedrigen CO2-Anteil) zumindest teilweise als Verbrennungsluft verwendet.
   - Der Betrieb der CO2-Senke erfolgt bevorzugt zur Umsetzung von CO2 und H2O zu O2 und Biomasse.
- Schaffung und Aufrechterhaltung der technisch-biologischen Voraussetzungen für den kontinuierlichen Betrieb, z. B. etwa durch Einstellung einer geeigneten CO2-Konzentration oder eines geeigneten Durchsatzes bzw. einer geeigneten Austauschrate für den Kraftwerksbetrieb; Aufrechterhaltung eines kontinuierlichen oder alternierenden/intermittierenden Stoffaustausches von CO2 und O2; Aufrechterhaltung einer hinsichtlich der Photosynthese vorteilhaften Temperaturführung; Sicherstellung eines verlustarmen Wasserhaushaltes (z. B. sorgsame Kapselung, Biomasse-Trocknung, Wasserrückführung, Kondensation des Wasseranteils abgegebener Rauchgasanteile); Zuführung von Hilfsstoffen nach Bedarf, biologische Zustandsüberwachung; Entnahme und zweckmäßige Aufbereitung erzeugter Biomasse; Steuerung, Überwachung, Inspektion, Wartung, Instandsetzung der Anlage, Minimierung oder Vermeidung ungewollter Leckagen.
- Schaffung und Aufrechterhaltung der personellen und personenbezogenen Voraussetzungen für den kontinuierlichen Betrieb, z. B. wie oben bereits beschrieben
- Schaffung und Aufrechterhaltung der wirtschaftlichen Voraussetzungen für den kontinuierlichen Betrieb, z. B. wie oben bereits beschrieben, wobei z. B. eine Auswahl geeigneter Biomasse auch den Gesichtspunkten der Eignung als Kraftwerksbrennstoff und des erforderlichen Aufwandes für Entnahme und Aufbereitung als Kraftwerksbrennstoff getroffen werden kann. Bereitstellung von Zusatzbrennstoff nach Bedarf.
- Rechnungslegung für die Stromerzeugung
- Vermarktung etwaiger im Überschuss gewonnenen Biomasse, z. B. wie oben bereits beschrieben
- Bevorzugt kontinuierliche wirtschaftlich-technischbiologische Optimierung der Anlage
   Die wirtschaftliche Optimierung kann umfassen: Optimierung der CO2-Beseitigungsleistung oder der Biomasseerzeugung; Optimierung der Organismenauswahl (Art, Menge, Eignung als Kraftwerksbrennstoff); Optimierung der Vermarktungserlöse etwaiger im Überschuss gewonnener Biomasse oder Biomasseprodukte.
   Die technische Optimierung kann umfassen: Optimierung des Kraftwerkswirkungsgrades und der Effizienz der Gesamtanlage; Optimierung der technischen Einrichtungen und Abläufe; Optimierung der Produktivität, Optimierung des Ressourceneinsatzes (z. B. elektrischer Eigenbedarf, Wasserverbrauch); Optimierung der Biomasseentnahme und - aufbereitung.
   Die biologische Optimierung kann umfassen: Einsatz der photosynthesetreibenden Organismen (Art, Menge); Optimierung der Prozessparameter und Randbedingungen; Optimierung der biologisch-technischen Anlagen in biologischer Hinsicht; Durchführung von Labor- und Feldversuchen zu Photosyntheseleistung und Biomasseerzeugung; Durchführung von voreilenden Versuchen (Vorhersage der biologischen Entwicklung) und Szenario-Versuchen; Erstellung und Aufbewahrung biologischer Proben von bewährten oder besonders geeigneten Stämmen von Organismen (für Wiedereinsatz oder Neuausbringung).

Mit den beschriebenen optionalen Weiterbildungen kann ein ökologisch wie ökonomisch besonders nachhaltiges, überaus attraktives Geschäftsmodell erreicht werden.

Vorteilhaft kann auch das Geschäftsmodell eine einzigartige Möglichkeit für eine nachhaltige Lösung des Energieproblems bieten, indem neue wirtschaftlich attraktive Möglichkeiten aufgezeigt werden, wie die in sonnenreichen Breiten einfallende Energie mit Hilfe wettbewerbsfähiger Technologien großtechnisch in elektrische Energie umgewandelt werden kann. Eine nachhaltige, überaus wettbewerbsfähige Versorgung mit CO2-neutraler elektrischer Energie wird möglich. Das neue Geschäftsmodell bietet immense Vorteile gegenüber den derzeit diskutierten Lösungen zum Betrieb CO2-freier Kraftwerke, welche mit gravierenden Leistungseinbußen und Mehrkosten verbunden sind.

Das Geschäftsmodell kann auch Großfeuerungsanlagen betreffen. In diesem Fall wird die Schaffung von Leitungsverbindungen durch Schaffung von Absatz/Transportkanälen ersetzt. Weiterhin erfolgt die Rechnungslegung für das erzeugte Produkt.

Die Erfindung wird nachfolgend anhand von Ausführungsbeispielen mit Bezug auf die beigefügten Zeichnungen weiter beschrieben. Es stellen jeweils stark schematisiert dar:
- Fig. 1: eine CO2-Beseitigungsvorrichtung ("CO2-Senke") gemäß eines ersten Ausführungsbeispiels,
- Fig. 2: ein Detail aus Fig. 1,
- Fig. 3: eine der Fig. 1 ähnliche Darstellung zur Veranschaulichung von Möglichkeiten zur Abfuhr von erzeugter Biomasse,
- Fig. 4: eine bei der Vorrichtung von Fig. 1 verwendbare Biomasse-Weiterverarbeitungseinrichtung,
- Fig. 5: eine bei der Vorrichtung von Fig. 1 verwendbare Luftaustauscheinrichtung,
- Fig. 6: eine CO2-Beseitigungsvorrichtung gemäß eines zweiten Ausführungsbeispiels,
- Fig. 7: eine bei der Vorrichtung von Fig. 6 verwendbare Temperaturführungseinrichtung,
- Fig. 8: eine Darstellung zur Veranschaulichung von Möglichkeiten zur Anordnung von photosynthesetreibenden Organismen bei der Vorrichtung von Fig. 6,
- Fig. 9: den prinzipiellen Aufbau eines Kohlekraftwerkes zum Betrieb in Kombination mit einer CO2-Beseitigungsvorrichtung (Luft-Rauchgasseite),
- Fig. 10: den Aufbau einer für das Kraftwerk von Fig. 9 verwendbaren CO2-Beseitigungsvorrichtung,
- Fig. 11: eine für das Kraftwerk von Fig. 9 verwendbare Luftzufuhreinrichtung,
- Fig. 12: ein in der Vorrichtung von Fig. 10 verwendbares CO2-Beseitigungsmodul,
- Fig. 13: ein in der Vorrichtung von Fig. 10 verwendbares CO2-Beseitigungsmodul,
- Fig. 14: eine für die Vorrichtung von Fig. 10 verwendbare Biomasse-Weiterverarbeitungseinrichtung gemäß einer ersten Ausführungsvariante,
- Fig. 15: eine für die Vorrichtung von Fig. 10 verwendbare Biomasse-Weiterverarbeitungseinrichtung gemäß einer zweiten Ausführungsvariante,
- Fig. 16: eine für die Vorrichtung von Fig. 10 verwendbare Biomasse-Weiterverarbeitungseinrichtung gemäß einer dritten Ausführungsvariante,
- Fig. 17: eine für CO2-Beseitigungsmodule gemäß Fig. 11 verwendbare Temperaturführungseinrichtung,
- Fig. 18: eine CO2-Beseitigungsvorrichtung gemäß eines weiteren Ausführungsbeispiels,
- Fig. 19: eine Anordnung mehrerer CO2-Senken in Verbindung mit einem Gasaustauschkanal gemäß eines Ausführungsbeispiels,
- Fig. 20: ein Ablaufdiagramm für ein Verfahren zur wirtschaftlichen Nutzung einer CO2-Beseitigung gemäß eines Ausführungsbeispiels, und
- Fig. 21: ein Ablaufdiagramm eines Verfahrens zur wirtschaftlichen Nutzung einer CO2-Beseitigung gemäß eines weiteren Ausführungsbeispiels.

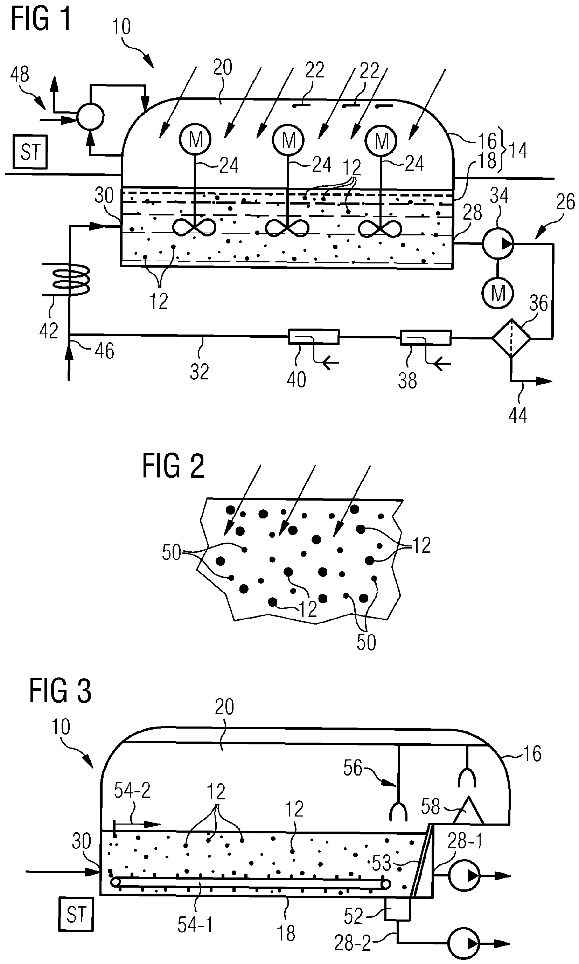

Fig. 1 zeigt den prinzipiellen Aufbau einer Vorrichtung 10 zur Beseitigung von CO2, umfassend photosynthesetreibende Organismen 12 zur photosynthetischen Umsetzung von CO2, welche im dargestellten Ausführungsbeispiel von "Wasserpflanzen" oder ähnlichen im Wasser lebenden Organismen gebildet sind (z. B. auch Algen oder Bakterien). Eine derartige Ausführung wird daher nachfolgend auch als "wasserpflanzenbasierte Lösung" bezeichnet.

Die Vorrichtung 10 umfasst ferner eine Umhüllung 14, die im dargestellten Ausführungsbeispiel von einem mit Wasser befüllten Becken 18 zur Aufnahme der Organismen 12 und einer lichtdurchlässigen Überdachung 16 gebildet ist.

Die Umhüllung 16 dient zur Unterbringung der Organismen in einem Innenraum 20 und zur Aufrechterhaltung vorbestimmter Umgebungsbedingungen für die Organismen 12 im Innenraum 20.

Aufgrund der Lichtdurchlässigkeit der Überdachung 16 kann von oben auf die Vorrichtung 10 auftreffendes Sonnenlicht (vgl. Pfeile) in den Innenraum 20 und somit zu den photosynthesetreibenden Organismen 12 vordringen.

Der Überdachung 16 sind ansteuerbare Blendeneinrichtungen 22 zugeordnet, mittels welchen das Ausmaß der Sonnenlichtbestrahlung der Organismen 12 variabel einstellbar bzw. begrenzbar ist.

Die Ansteuerung der Blendeneinrichtungen 22 erfolgt durch eine Steuereinrichtung, welche bevorzugt als eine programmgesteuerte elektronische Steuereinrichtung ausgebildet ist, welche die Blendeneinrichtungen 22 sowie nachfolgend noch beschriebene weitere steuerbare Komponenten der Vorrichtung 10 ansteuert.

Ein in der Steuereinrichtung ST ablaufendes Steuerprogramm berücksichtigt hierbei eine Vielzahl von Betriebsparametern der Vorrichtung 10, insbesondere von Parametern der Organismen 12 und/oder der Umgebung der Organismen 12 (z. B. Temperatur und Luftfeuchtigkeit in der Innenatmosphäre des Innenraumes 20 (überhalb des Wasserspiegels) sowie Temperatur des Wassers im Wasserbecken 18, CO2-Konzentrationen überhalb und unterhalb des Wasserspiegels etc.).

Derartige Betriebsparameter werden mit Hilfe einer Vielzahl von geeignet ausgebildeten und angeordneten Sensoren erfasst und der Steuereinrichtung ST zugeführt.

Die Vorrichtung 10 umfasst des weiteren steuerbare Rührwerke 24 zur Verwirbelung des im Becken 18 befindlichen Wassers sowie eine Umwälzeinrichtung 26 zur Umwälzung des Wassers.

Die Umwälzeinrichtung 26 umfasst eine zwischen einem Wasserabfluss 28 und einem Wasserzufluss 30 angeordnete Wasserleitungsanordnung 32, durch welche hindurch Wasser aus dem Becken 18 gepumpt wird. Im dargestellten Ausführungsbeispiel sind entlang dieser Leitungsanordnung 32 eine steuerbare Pumpe 34, ein Filter 36, eine CO2-Zumischeinrichtung 38, eine weitere Zumischeinrichtung 40 und ein Wärmetauscher 42 angeordnet. Deren Betrieb wird durch die Steuereinrichtung bedarfsgerecht angesteuert.

Der Filter 36 dient zum Ausfiltern von Organismen aus dem Wasser, um diese als "Biomasse" weiter zu verwenden. Ein entsprechender Biomasse-Abfuhranschluss ist mit 44 bezeichnet.

Die CO2-Zumischeinrichtung 38 dient zur gesteuerten Zumischung von zu beseitigendem CO2 in den Wasserkreislauf. Mittels der weiteren Zumischeinrichtung 40 können neue und/oder aufbereitete Organismen in den Wasserkreislauf eingebracht werden. Außerdem können an dieser Stelle Hilfsstoffe wie z. B. Nährstoffe, Dünger etc. zugemischt werden.

Der Wärmetauscher 42 ermöglicht vorteilhaft eine bedarfsweise Temperierung des Wassers, wobei je nach Installationsumgebung der Vorrichtung 10 sowohl ein Heizen als auch ein Kühlen in Betracht kommt.

Der Wärmetauscher 42 ist Teil eines Wärmemanagementsystems der Vorrichtung 10 zur gesteuerten Temperierung des Wassers sowie der Atmosphäre im Innenraum 20. Dieses System kann z. B. aktive Heiz- bzw. Kühleinrichtungen, aber auch teilweise passiv, etwa auf Basis eines Wärmespeichers arbeitende Einrichtungen zur Abgabe (bzw. Aufnahme) von Wärme umfassen.

Die Wasserleitungsanordnung 32 umfasst außerdem einen Wasserzufluss 46, an welchem Wasser in den Kreislauf zurückgeführt werden kann, welches bei einer Weiterverarbeitung der am Anschluss 44 abgeführten Biomasse anfällt.

Die Vorrichtung 10 umfasst ferner eine Luftaustauscheinrichtung 48 zum kontrollierten und gesteuerten Austausch von Luft zwischen der Luft im Innenraum 20 (Innenraumatmosphäre) und der Luft in der Umgebung der Vorrichtung 10 (Atmosphäre).

Eine weitere vorteilhafte Besonderheit der dargestellten Vorrichtung 10 ist die Verwendung von photolumineszenten Stoffen 50, welche z. B. in granularer Form dem als Trägermedium für die Organismen 12 dienenden Wasser beigemischt sind.

Fig. 2 veranschaulicht die im Wasser des Beckens 18 vorliegende Mischung aus Organismen 12 und photolumineszenten Stoffen 50. Durch die Anwesenheit der Stoffe 50 wird die Effizienz der CO2-Umsetzung vorteilhaft verbessert.

Im dargestellten Beispiel sind photolumineszente Stoffe z. B. zur Wandlung von kurzwelligen Bestandteilen der Sonnenstrahlung (z. B. aus dem Bereich 200 bis 360 nm) in eine langwelligere, hier für die Photosynthese günstigere Strahlung (z. B. in den Bereich 360 bis 700 nm) geeignet gewählt.

Fig. 3 veranschaulicht weitere bzw. abgewandelte Möglichkeiten für die Abfuhr von Biomasse aus der Vorrichtung 10. Der Einfachheit der Darstellung von Fig. 3 halber sind einige der in Fig. 1 eingezeichneten Vorrichtungskomponenten in Fig. 3 weggelassen.

Zunächst erkennt man in Fig. 3 mit 30 und 28-1 bezeichnet wieder einen Wasserzufluss bzw. Wasserabfluss, welche in der in Fig. 1 dargestellten Weise zur Wasserumwälzung nutzbar sind.

Wie oben mit Bezug auf die Fig. 1 erläutert, können Komponenten der Umwälzeinrichtung 26 gleichzeitig als Komponenten einer Biomasse-Abfuhreinrichtung genutzt werden, indem aus einer aus dem Wasserbecken 18 stammenden Wasserströmung Biomasse herausgefiltert wird.

Des Weiteren ist in Fig. 3 eine Abschlämmgrube 52 ersichtlich, die zum Abfangen schwererer Bestandteile der Biomasse dient und in der Nähe des Abflusses 28-1 angeordnet ist. Zwischen der Grube 52 und dem Abfluss 28-1 ist ein schräg verlaufender Rechen 53 im Wasserbecken 18 angeordnet, mittels welchem bereits im Becken 18 ein Sieben des Wassers zur Abscheidung von Biomasseanteilen in die Grube 52 erfolgt. Am Boden der Abschlämmgrube 52 ist ein weiterer Wasserabfluss 28-2 vorgesehen, der zur Abfuhr der Biomasse nutzbar ist.

Außerdem kann das Befüllen der Abschlämmgrube 52 durch weitere, im Wasserbecken 18 angeordnete technische Einrichtungen realisiert bzw. gefördert werden, wie z. B. mittels einer am Boden des Wasserbeckens 18 umlaufenden Fördereinrichtung (z. B. dargestellte Abstreiferkette 54-1).

Speziell für an der Wasseroberfläche befindliche Organismen 12 bzw. Umsetzungsprodukte kann eine entlang der Wasseroberfläche verfahrbare Abstreifer- bzw. Siebeinrichtung 54-2 vorgesehen sein.

Insbesondere zur Abfuhr größerer Mengen an Biomasse, die sich vor dem Rechen 53 ansammeln, kann auch ein Hebezeug 56 wie in Fig. 3 symbolisiert eingesetzt werden, um Biomasse 58 aus dem Wasserbecken 18 herauszufördern, um diese einer späteren Weiterverarbeitung und/oder Weiterverwendung zuzuführen.

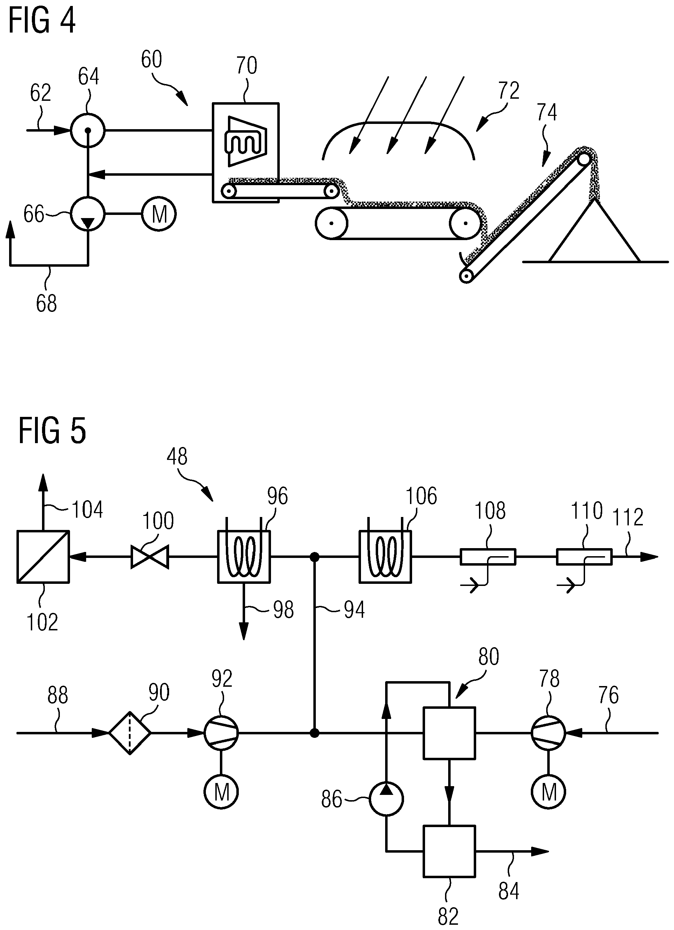

Fig. 4 veranschaulicht mögliche Biomasse-Weiterverarbeitungsschritte anhand einer sich z. B. an den Biomasse-Abfuhranschluss 44 der Vorrichtung 10 anschließenden Biomasse-Weiterverarbeitungseinrichtung 60.

Die Einrichtung 60 umfasst einen Biomasse-Zufuhranschluss 62, welcher z. B. mit dem Abfuhranschluss 44 (Fig. 1) verbunden ist.

Die zugeführte Biomasse wird im dargestellten Beispiel zunächst einer Zentrifuge 64 (alternativ z. B. Schwerkraftsichter) zugeführt, um Wasser aus der Biomasse abzuscheiden und über eine Pumpe 66 an einem Wasserabflussanschluss 68 bereitzustellen, welcher z. B. mit dem Wasserzufluss 46 in Fig. 1 verbunden ist.

Sodann wird die bereits grob entwässerte Biomasse einer Presse, z. B. der symbolisierten Siebbandpresse 70 zugeführt, um durch mechanisches Pressen der Biomasse weiters Wasser zu entziehen und über die Pumpe 66 am Wasserabfluss 68 bereitzustellen.

Sodann wird die nurmehr feuchte Biomasse einer weiteren Trocknungsstation 72 zugeführt, in welcher z. B. eine thermische Trocknung durchgeführt wird. Dies z. B. durch Sonnenlichteinstrahlung (Pfeile) oder andere Fremdwärme.

Schließlich wird die trockene Biomasse sodann über ein Förderband 74 als Schüttgut bereitgestellt (z. B. für einen kommerziellen Vertrieb).

Es versteht sich, dass für die Weiterverarbeitung der Biomasse auch vielfältige andere, an sich bekannte Möglichkeiten zur Verfügung stehen, auf die hier vorteilhaft zurückgegriffen werden kann.

Die Rückspeisung des aus dem Trocknungsprozess (hier am Wasserabfluss 68) bereitgestellten Wassers in das Wasserreservoir (hier in den in Fig. 1 dargestellten Umwälz-Wasserkreislauf), ist insbesondere bei Verwendung der Vorrichtung 10 in geographischen Gebieten mit Wassermangel sehr vorteilhaft. Alternativ oder zusätzlich kann dieses Wasser jedoch auch anderen Zwecken dienen, etwa zu Kühlungszwecken.

Ganz allgemein erfolgt eine Biomasseaufbereitung bevorzugt durch Schritte der Eindickung und der Entwässerung, optional Trockensubstanzzumischung. Aufgrund der hohen Massenströme sind Lösungen mit umlaufenden Siebbändern für die Eindickung vorteilhaft. Die Eindickung erfolgt vorteilhaft in räumlicher Nähe der CO2-beseitigenden Vorrichtungsinnenräume, um Leistungsbedarf und Investitionen für Transport bzw. Förderung der Biomasse zu begrenzen. Zur Verfahrensunterstützung kann ein Flockungsmittel zugesetzt werden.

Eine Entwässerung der Biomasse kann vorteilhaft mit Anlagen für hohe Durchsätze, z. B. Siebbandpressen oder Bogenpressen oder dergleichen erfolgen. Beispielsweise kann das Aufgabegut zwischen Siebbändern einen zunehmenden Druck und nachfolgend einer Scherpresszone ausgesetzt werden, in der alternierende Walkbewegung für eine möglichst wirksame Entwässerung sorgen. Flockungsmittel kann optional verwendet werden. Erreichbare Biomasseanteile z. B. 30%.

Zur Vereinfachung der Handhabbarkeit kann der entwässerten Biomasse eine Trockensubstanz, insbesondere bereits weiter getrocknete Biomasse aus derselben Biomasse-Weiterverarbeitung, zugeführt bzw. zugemischt werden (Rückführung von Biomassetrocknung). Insbesondere kann die Biomasse z. B. zu stückigen oder festen Biomasseteilchen (z. B. "Pellets") geformt werden (z. B. durch eine Schneckenpresse).

Der Transport zur Biomassetrocknung kann z. B. mittels Förderbändern erfolgen.

Fig. 5 zeigt etwas detaillierter den Aufbau der bei der Vorrichtung 10 von Fig. 1 verwendeten Luftaustauscheinrichtung 48.

Dieser Einrichtung 48 wird über einen Luftzufuhranschluss 76 Luft aus der Innenraumatmosphäre der Vorrichtung 10 zugeführt.

Diese gelangt über ein Gebläse 78 zu einem CO2-Wäscher 80, in welchem das CO2 z. B. mittels einer Kalkmilchdusche ausgewaschen wird und an einer Kalkmilch-Aufbereitungseinheit 82 an einem CO2-Abgabeanschluss 84 bereitgestellt wird, der bevorzugt derart mit der Vorrichtung 10 verbunden ist, dass das ausgewaschene CO2 wieder den photosynthesetreibenden Organismen 12 zugeführt wird.

Der CO2-Abgabeanschluss 84 kann hierfür z. B. mit der in Fig. 1 eingezeichneten CO2-Zumischeinrichtung 38 verbunden sein. Die Kalkmilch durchströmt die Einrichtungen 80, 82 in einem geschlossenem Kreislauf, getrieben durch eine Pumpe 86.

Die somit aufbereitete Luft strömt zusammen mit Frischluft, die über einen Luftzufuhranschluss 88, einen Filter 90 und ein Gebläse 92 ebenfalls der Einrichtung 48 zugeführt wird, in eine Luftsammelleitung 94.

Ausgehend von der Sammelleitung 94 wird die Luft im dargestellten Beispiel in zweierlei Weise verwendet:

Zum Einen, in Fig. 5 links dargestellt, wird der Luft mittels einer Rückkühlanlage bzw. eines Wasserabscheiders 96 Wasser entzogen (auskondensiert) und an einem Wasserabfuhranschluss 98 bereitgestellt. Dieses Wasser kann z. B. wie das von der Biomasse-Weiterverarbeitungseinrichtung 60 am Anschluss 68 bereitgestellte Wasser einer Wiederverwendung zugeführt werden. Die somit entfeuchtete Luft wird dann über ein steuerbares Ventil 100 und einen Feinfilter 102 (z. B. biologische Sperre, Sterilisation) in die Atmosphäre ausgelassen (Abgabestutzen 104).

Zum Anderen, in Fig. 5 rechts dargestellt, strömt die Luft aus der Sammelleitung 94 über einen Wärmetauscher 106, eine erste Zumischeinrichtung 108 (z. B. zur Zumischen von neuen oder aufbereiteten Organismen) und eine zweite Zumischeinrichtung 110 zur Zumischung von CO2, welches z. B. von der Bereitstellung am CO2-Abgabeanschluss 84 der Luftaustauscheinrichtung 48 oder einer anderen CO2-Quelle stammt (z. B. zu beseitigendes, der Vorrichtung 10 zugeführtes CO2). Die in dieser Weise aufbereitete Luft wird über eine Abgabeleitung 112 in die Innenraumatmosphäre der Vorrichtung 10 und/oder in das Wasser zurückgeleitet.

Mit der beschriebenen CO2-Beseitigungsvorrichtung 10 kann CO2 effektiv durch eine technisch unterstützte Anwendung der Photosynthese erfolgen. Durch eine geeignete Sensorik kann die Steuereinrichtung ST sämtliche steuerbaren Vorrichtungskomponenten gezielt zur Schaffung von optimalen Photosynthesebedingungen ansteuern. Die Vorrichtung 10 kann sehr vorteilhaft insbesondere in geographischen Gebieten mit hoher Sonneneinstrahlung und niedrigen Landkosten installiert werden. Das zu beseitigende CO2 kann hierbei vor Ort z. B. der Atmosphäre entnommen werden, oder unmittelbar aus einer antropogenen CO2-Quelle stammen, sei es eine Quelle am Ort der Vorrichtung 10 oder entfernt davon. In letzterem Falle muss das CO2 angeliefert werden (z. B. Pipeline oder dergleichen).

Bei der nachfolgenden Beschreibung von weiteren Ausführungsbeispielen werden für gleichwirkende Komponenten weitgehend die gleichen Bezugszahlen verwendet, jeweils ergänzt durch einen kleinen Buchstaben zur Unterscheidung der Ausführungsform. Dabei wird im Wesentlichen nur auf die Unterschiede zu dem bzw. den bereits beschriebenen Ausführungsbeispielen eingegangen und im Übrigen hiermit ausdrücklich auf die Beschreibung vorangegangener Ausführungsbeispiele verwiesen.

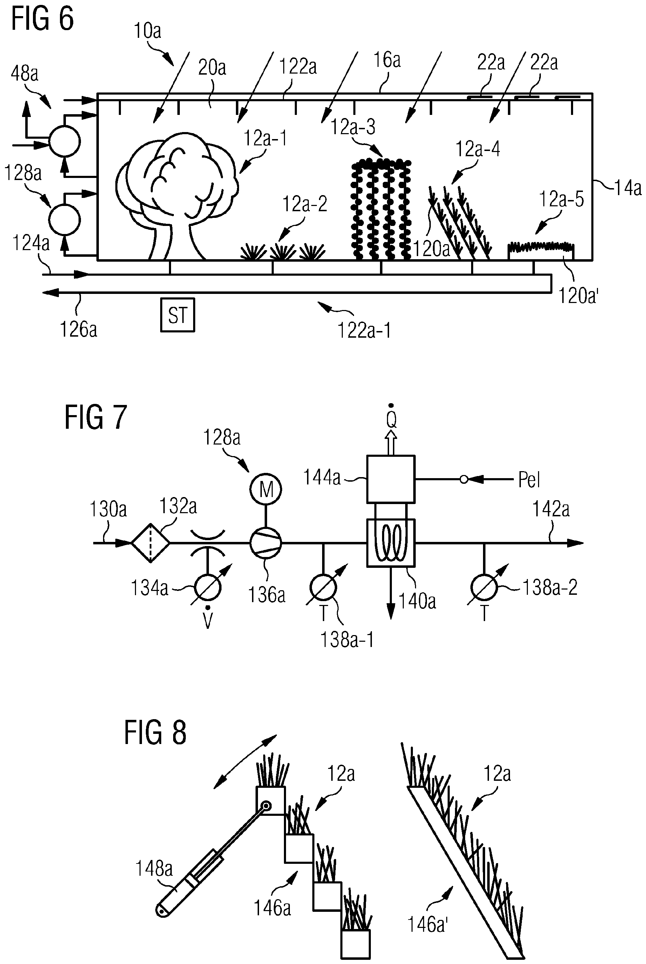

Fig. 6 veranschaulicht eine CO2-Beseitigungsvorrichtung 10a gemäß einer zweiten Ausführungsform, die sich von der zuvor beschriebenen im Wesentlichen lediglich dadurch unterscheidet, dass die für die Photosynthese eingesetzten Organismen 12 keine "Wasserpflanzen" sondern "Landpflanzen" sind, beispielsweise wie in Fig. 6 veranschaulicht Bäume oder Sträucher 12a-1, Gräser oder Bodenpflanzen 12a-2, Rankpflanzen 12a-3, Gräser oder Bodenpflanzen 12a-4 auf Trägereinrichtungen 120a mit geneigten Flächen, Nutzpflanzen 12a-5 in separaten Behältern 120a' etc.

Dementsprechend ist in einem Innenraum 20a der Vorrichtung 10a auch kein Wasserbecken zur Aufnahme der Organismen 12 vorgesehen.

Die Organismen 12a werden jedoch über eine Wasserleitungsanordnung 122a-1 (Zufluss 124a-1 und Rückfluss 126a für Überschusswasser) bewässert. Bevorzugt wird damit auch eine bedarfsgerechte Zufuhr von Hilfsstoffen (z. B. Nährstoffe) bewerkstelligt. Im dargestellten Beispiel ist außerdem eine weitere Wasserleitungsanordnung 122a-2 zur Beregnung bzw. Berieselung der Organismen 12a mit Wasser vorgesehen (Wasserzufluss 124a-2).

Wenngleich in Fig. 6 eine Vielzahl verschiedener Arten von Organismen 12a eingezeichnet sind, so ist in der Praxis zumeist der Einsatz einer Monokultur bevorzugt, es sei denn, dass der Innenraum 20a in mehrere Bereiche mit jeweils verschiedenen CO2-Beseitigungsanforderungen aufgeteilt ist und die einzelnen Bereiche in diesem Fall auch unterschiedliche Arten von Organismen 12a aufnehmen können.

Wie bei der oben beschriebenen Ausführungsform erfolgt auch hier ein Sonnenlichteinfall (Pfeile) durch einen als lichtdurchlässige Überdachung 16a ausgebildeten Teil der Umhüllung 14a. Wie bei der ersten Ausführungsform wirkt die Umhüllung 14a darüber hinaus als Verdampfungsschutz (Wasser) und Leckageschutz (CO2).

Eine in Fig. 6 ersichtliche Luftaustauscheinrichtung 48a kann z. B. wie bereits mit Bezug auf Fig. 5 beschrieben ausgebildet sein.

Die Vorrichtung 10a stellt eine "landpflanzenbasierte Lösung" zur CO2-Beseitigung dar. Wie bei der oben bereits beschriebenen wasserpflanzenbasierten Lösung wird mit der Umhüllung 14a vorteilhaft ein von der umgebenden Atmosphäre getrennter, im Wesentlichen umschlossener, bevorzugt großflächiger Raum geschaffen, in welchem durch geeignete Ansteuerung der Vorrichtungskomponenten vorteilhaft eine gegenüber der Atmosphäre erhöhte CO2-Konzentration aufrechterhalten bzw. eingestellt werden kann, um die Photosynthese zu optimieren.

Wenngleich bei der Vorrichtung 10a z. B. durch verstellbare Blendeneinrichtungen 22a sowie die Wasserberegnung mittels der Wasserleitungsanordnung 122a bereits ein gewisser Schutz vor zu hoher Temperatur im Innenraum 20a geschaffen ist, so mangelt es der Vorrichtung 10a im dargestellten Ausführungsbeispiel an einem "Wärmespeicher" wie dieser bei der Vorrichtung 10 von Fig. 1 durch das wasserbefüllte Becken 18 gebildet ist. Mit dem Wasserbecken 18 wird vorteilhaft eine gewisser Vergleichmäßigung der Innenraumtemperatur erzielt. Das Wasser kann tagsüber Wärme aufnehmen (Kühlung des Innenraumes) und in der Nacht Wärme abgeben (Heizung des Innenraumes) .

Selbstverständlich kann auch bei der Vorrichtung 10a ein derartiger oder ähnlicher Wärmespeicher ausgebildet werden, wobei die entsprechenden Wärmetransfervorgänge passiv und/oder aktiv erfolgen können.

Zum Bewirken aktiver Wärmetransfervorgänge kann vorteilhaft auf an sich bekannte Wärmemanagementmaßnahmen zurückgegriffen werden, wie sie z. B. aus dem Bereich der Wärmespeicher bzw. Temperierungseinrichtungen für Gebäude bekannt sind.

Bei der Vorrichtung 10a von Fig. 6 ist alternativ hierzu eine andere technische Einrichtung zur Temperierung des Innenraumes 20a vorgesehen, nämlich eine Temperaturführungseinrichtung 128a, welche dem Innenraum 20a Luft entzieht, diese bedarfsgerecht temperiert und die temperierte Luft wieder in den Innenraum 20a abgibt. Es ist anzumerken, dass eine derartige Temperaturführungseinrichtung auch bei der oben beschriebenen wasserpflanzenbasierten Lösung eingesetzt werden könnte, und dass eine derartige Einrichtung auch als Teil einer gegebenenfalls vorgesehenen Luftaustauscheinrichtung (z. B. 48 in Fig. 1 bzw. 48a in Fig. 6) in dieser integriert vorgesehen sein könnte.

Fig. 7 zeigt den prinzipiellen Aufbau der Temperaturführungseinrichtung 128a zur Temperierung der Innenraumatmosphäre der Vorrichtung 10a.

An einem Lufteinlass 130a tritt Luft aus dem Innenraum 20a ein und passiert sodann einen Luftfilter 132a zum Filtern der Luft, eine Strömungsmesseinrichtung 134a zum Erfassen einer Luftströmungsrate (Volumenstromrate), ein steuerbares Gebläse 136a zur Einstellung einer gewünschten Luftströmungsrate, einen ersten Temperatursensor 138a-1 zum Messen der Temperatur der aus dem Innenraum 20a abgezogenen Luft, einen Wärmetauscher 140a zum bedarfsweisen Temperieren (also Heizen oder Kühlen) der Luft, und einen zweiten Temperatursensor 138a-2. Die somit in gewünschter Weise, etwa mittels der Steuereinrichtung ST temperierte Luft wird an einem Luftauslass 142a wieder in den Innenraum 20a ausgelassen.

In den meisten Anwendungsfällen wird an dieser Stelle eine Kühlung der Luft erforderlich sein. Zu diesem Zweck kann der Wärmetauscher 140a wie dargstellt mit einer Kühlanlage bzw. Rückkühlanlage 144a verbunden sein, welche mit einer elektrischen Versorgungsleistung Pel versorgt wird und einem umgewälzten Kühlmedium Wärme entzieht und beispielsweise an die Umgebung der Vorrichtung 10a abgibt.

Fig. 8 zeigt zwei prinzipielle Möglichkeiten zur Anordnung der Organismen 12a in der Vorrichtung 10a.

Im linken Teil der Figur ist ein rahmenartiger Träger 146a mit mehreren Pflanzenbehältnissen dargstellt, deren räumliche Positionen durch den Träger vorgegeben wird. Im dargestellten Beispiel verläuft der Träger 146a insgesamt entlang einer schräg gestellten Ebene, wobei die einzelnen Pflanzenbehältnisse terrassenartig am Träger angeordnet sind. Die Behältnisse sind z. B. mit Erde oder dergleichen (abhängig von den eingesetzten Organismen 12a) befüllt.

Im rechten Teil der Figur ist ein demgegenüber modifizierter Träger 146a' dargestellt, der an seiner Oberseite insgesamt eine von den Pflanzen 12a bewachsbare Fläche bereitstellt. Der Träger 146a' kann z. B. im Wesentlichen aus einem porösen, feuchten und mit Nährstoffen ausgestatteten Material gebildet sein.

Bei beiden Trägervarianten kann durch den schrägen Verlauf der Pflanzenanordnungsfläche (nicht horizontal) vorteilhaft eine verbesserte Exposition der Pflanzen 12a mit Sonnenlicht erreicht werden, welches durch die transparente Überdachung 16a in den Innenraum 20a einfällt.

Falls die verwendeten Pflanzenträger stationär sind, so sollten diese demnach in die "Haupteinfallsrichtung" des Sonnenlichts orientiert sein.

In einer bevorzugten Ausführungsform sind die verwendeten Träger (z. B. von der Art der dargestellten Träger 146a bzw. 146a') in ihrem Azimutwinkel und/oder Höhenwinkel gesteuert verstellbar. Damit kann die räumliche Anordnung der Pflanzen 12a stets optimal den jeweils gewünschten Bedingungen angepasst werden, insbesondere z. B. einem im Tagesverlauf wechselnden Sonneneinfall nachgeführt werden.

Ein denkbarer Antrieb für eine Höhenwinkelverstellung (Pfeil in Fig. 8) des Trägers 146a ist in der Figur schematisch als hydraulischer Antrieb 148a symbolisiert. Alternativ oder zusätzlich können derartige Trägereinrichtungen auch in gesteuerter Weise in ihrem Azimutwinkel verstellbar sein.

Es versteht sich, dass die dargestellten Träger in vielfältiger Weise modifiziert und in vielfältiger Weise innerhalb der betreffenden CO2-Beseitigungsvorrichtung angeordnet werden können. Von oben betrachtet kann z. B. eine Anordnung der davon getragenen Organismen entlang gerade, geknickt oder gekrümmt vorgesehenen Verläufen vorgesehen sein (je nach Art der Organismen und/oder den geographischen Gegebenheiten).

Die oben beschriebenen CO2-Beseitigungsvorrichtungen 10 und 10a können zur Beseitigung von angeliefertem CO2 oder direkt aus der Umgebung (Atmosphäre) entnommenem CO2 eingesetzt werden.

Die Erfindung sieht vor, eine solche CO2-Beseitigung unmittelbar mit einer anthropogenen CO2-Erzeugung zu kombinieren. Hierfür kann z. B. eine CO2-Beseitigungsvorrichtung der oben beschriebenen Art mit einem CO2-erzeugenden Kraftwerk, insbesondere Großkraftwerk zur Energieerzeugung, gekoppelt werden, so dass von dem Kraftwerk erzeugtes CO2 zumindest teilweise der CO2-Beseitigungsvorrichtung zugeführt wird und dort zumindest teilweise beseitigt wird. Das Kraftwerk und die CO2-Beseitigungsvorrichtung können z. B. in unmittelbarer Nähe zueinander angeordnet sein bzw. das Kraftwerk kann baulich im Bereich der CO2-Beseitigungsvorrichtung integriert sein.

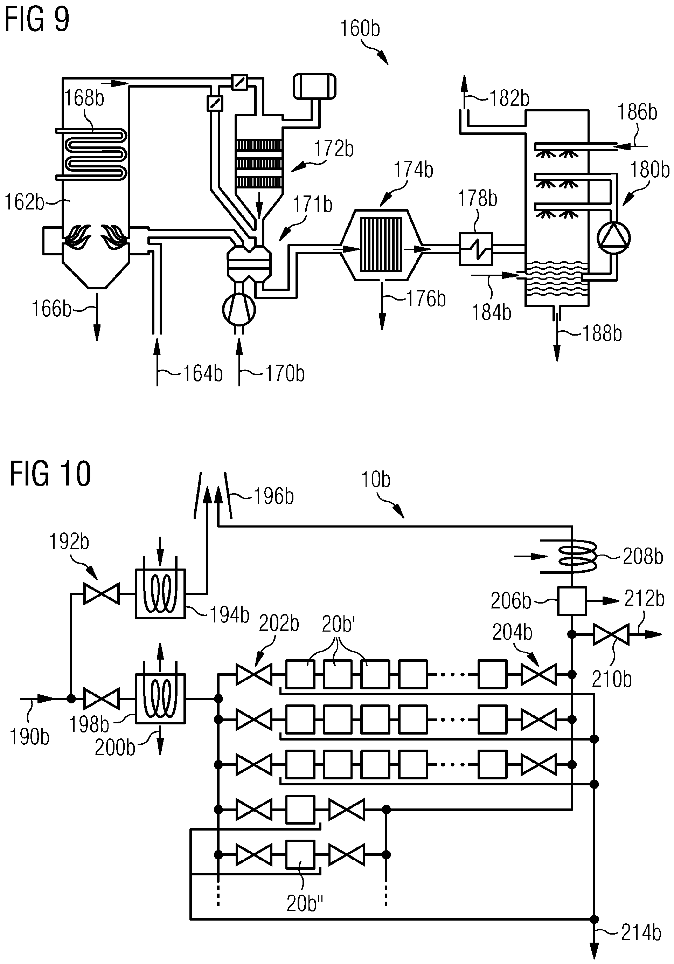

Eine solche vorteilhafte Kopplung zwischen einem Kraftwerk und einer CO2-Beseitigungsvorrichtung wird nachfolgend mit Bezug auf die Fig. 9 bis 17 anhand eines Ausführungsbeispiels erläutert.

Fig. 9 zeigt den Aufbau eines in weiten Teilen konventionellen Kohlekraftwerkes 160b, umfassend einen Brennraum 162b zur Energieerzeugung durch einen CO2-erzeugenden Verbrennungsprozess. Der Brennstoff wie z. B. Kohle wird an einer Brennstoffzufuhr 164b dem Brennraum 162b zugeführt. Bei der Verbrennung entstehende Schlacke wird bei 166b ausgelassen.

Ein Wärmetauscher 168b, der einen Teil eines insgesamt nicht dargestellten Wasser-Dampfkreislaufes bildet, dient zur Abfuhr der entstehenden Verbrennungswärme, die in konventioneller Weise mittels einer Dampfturbine in elektrische Energie gewandelt wird und z. B. in ein Hochspannungsnetz eingespeist wird.

Über einen Zuluftanschluss 170b und einen Luftvorwärmer 171b wird für den Verbrennungsprozess erforderliche Luft zugeführt. Das beim Verbrennungsprozess entstehende Abgas wird im dargestellten Beispiel in konventioneller Weise über eine ammoniakbetriebene Rauchgasentstickungsanlage 172b ("DeNOx") zur Entfernung von Stickstoffmonoxid (NO) und Stickstoffoxiden (NOx)geleitet. Dieser Anlage 172b folgt eine elektrische Gasreinigungseinrichtung ("E-Filter") 174b zur elektrostatischen Abscheidung von Staub bzw. Asche, die bei 176b ausgelassen wird.

Schließlich wird das Abgas noch über einen Wärmetauscher 178b einer Entschwefelungsanlage 180b zur Entfernung von Schwefelverbindungen zugeführt, bevor es bei 182b ausgelassen wird.

Die Entschwefelungsanlage 180b wird bei 184b mit Kalkstein und bei 186b mit Wasser versorgt. Bei 188b wird entstehender Gips abgeführt.

Das Kraftwerk 160b ist zur zumindest teilweisen Beseitigung von CO2 aus dem (bei 182b ausgelassenen) Abgas mit einer CO2-Beseitigungsvorrichtung gekoppelt. Das am Auslass 184b ankommende Abgas wird also nicht wie bei einem konventionellen Kraftwerk z. B.über einen Schornstein direkt in die Atmosphäre ausgelassen.

Fig. 10 zeigt den Aufbau der mit dem Kraftverk verbundenen CO2-Beseitigungsvorrichtung 10b (welche abweichend von diesem Beispiel z. B. auch den Aufbau der oben bereits beschriebenen CO2-Beseitigungsvorrichtungen 10 bzw. 10a besitzen könnte).

Die CO2-Beseitigungsvorrichtung 10b weist einen Abgaszufuhranschluss 190b zur Zufuhr des vom Kraftwerk 160b am Abgasanschluss 182 b bereitgestellten, CO2-reichen Abgases auf.

Mittels einer steuerbaren Ventilanordnung 192b kann das Verhältnis von Abgas, welches über einen Wärmetauscher 194b und einen Schornstein 196b in die Atmosphäre ausgelassen wird, und Abgas, welches über einen Wärmetauscher 198b (mit Wasserrückgewinnung bei 200b) der CO2-Beseitigung zugeführt wird, in gewünschter Weise eingestellt werden.

Wie aus Fig. 10 ersichtlich, wird das zur CO2-Beseitigung vorgesehene Abgas sodann mittels einer eingangsseitigen Ventilanordnung 202b und einer ausgangsseitigen Ventilanordnung 204b in gewünschter Weise (gesteuert) durch eine Parallel- und Reihenanordnung einzelner (voneinander separierter) Innenraumbereiche 20b' bzw. 20b'' geführt. Für diese zellenartige Aufgliederung eines Innenraumes der Vorrichtung 10b gibt es vielfältige Möglichkeiten. Im dargestellten Beispiel sind z. B. drei parallel zueinander angeordnete Reihenanordnungen bestehend aus einer Vielzahl von CO2-Beseitigungsräumen 20b' dargestellt, wobei diesen Reihenanordnungen wiederum zwei einzelne CO2-Beseitigungsräume 20b'' parallel geschaltet sind.

In jedem der Innenraumbereiche 20b' bzw. 20b'' ist eine technische unterstützte CO2-Beseitigung mittels photosynthesetreibender Organismen vorgesehen, wie diese oben bereits ausführlich beschrieben wurde.

Der Vorteil der in Fig. 10 dargestellten Reihenanordnung der Bereiche bzw. Zellen 20b' besitzt den Vorteil, dass das CO2 bzw. CO2-haltige Medium (hier: Abgas eines Kraftwerkes) von einem Bereich in den nächsten Bereich geleitet wird, wobei die CO2-Konzentration von Bereich zu Bereich abnimmt, so dass jeder Bereich individuell hinsichtlich der darin eingesetzten Organismen und/oder Umgebungsbedingungen für diese Organismen für eine hohe CO2-Umsetzungseffizienz angepasst bzw. angepasst betrieben werden kann. Durch die Separation der einzelnen Bereiche können darin individuelle Bedingungen eingestellt (angesteuert) werden.

Die Parallelanordnung einzelner Bereiche bzw. mehrerer Reihenanordnungen besitzt z. B. den Vorteil der Schaffung einer gewissen Redundanz hinsichtlich etwaiger Ausfälle bzw. erforderlicher Wartungsarbeiten in einzelnen Bereichen der Vorrichtung 10b.

Das in seiner CO2-Konzentration durch die CO2-Beseitigung erheblich reduzierte Abgas verlässt die Vorrichtung 10b schließlich über eine Abluftaufbereitungseinrichtung 206b und einen Wärmetauscher 208b. In gesteuerter Weise (mittels eines Ventils 210b) wird im dargestellten Ausführungsbeispiel jedoch ein Teil dieses CO2-gereinigten Abgases an einem Auslass 212b bereitgestellt, der mit dem Zuluftanschluss 170b des Kraftwerkes 160b verbunden ist, um an dieser Stelle wahlweise bzw. in gewünschtem Verhältnis Frischluft und/oder die Abluft der Vorrichtung 10b einzuspeisen.

In Fig. 10 ist auch eine oben bereits beschriebene Biomasse-Abfuhr aus den einzelnen Zellen 20b' und 20b'' symbolisiert. Die Biomasse verlässt die Vorrichtung 10b an einem Biomasse-Abgabebereich 214b. Hinsichtlich der Weiterverwendung bzw. Weiterverarbeitung der Biomasse ist auf die oben bereits gegebenen Erläuterungen zu verweisen.

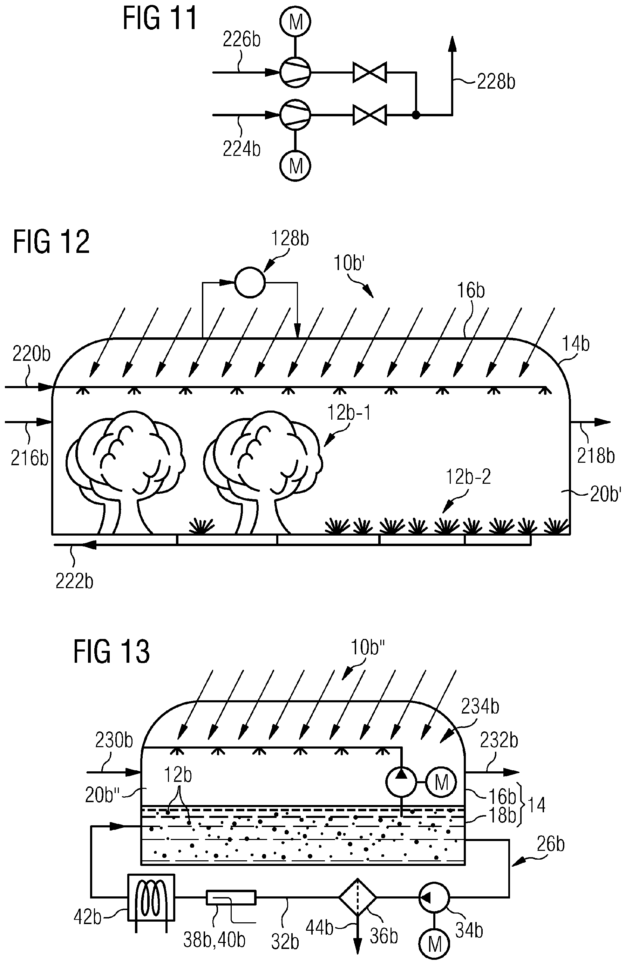

Fig. 11 zeigt eine mögliche Ausführung der steuerbaren Einstellung eines Mischverhältnisses zwischen einem Luftanteil aus der CO2-Beseitigungsvorrichtung 10b (zugeführt an einem Anschluss 224b) und Frischluft (zugeführt an einem Anschluss 226b). Diese beiden Luftströme werden jeweils über ein Gebläse und ein Ventil zu einem Abgabeanschluss 228b geleitet, der mit dem Zuluftanschluss 170b (Fig. 9) verbunden ist.

Fig. 12 veranschaulicht den Aufbau eines Vorrichtungsmoduls 10b' der Vorrichtung 10b (zur Ausbildung eines der Innenraumbereiche 20b' von Fig. 10).

Das dargestellte Beispiel des Moduls 10b' realisiert eine CO2-Senke gemäß der "landpflanzenbasierten Lösung", d. h. enthaltend Landpflanzen 12b-1 und 12b-2 als die photosynthesetreibenden Organismen.

Zur Einbindung des dargestellten CO2-Beseitigungsmoduls 10b' in die Vorrichtung 10b ist ein Abgaszufuhranschluss 216b und ein Abgasabfuhranschluss 218b vorgesehen. Die Bewässerung der Organismen 12b (und bedarfsweise Versorgung mit Hilfsstoffen) erfolgt über eine Wasserzufuhr 220b. Überschusswasser wird über eine bodenseitige Wasserabfuhr 222b abgeführt.

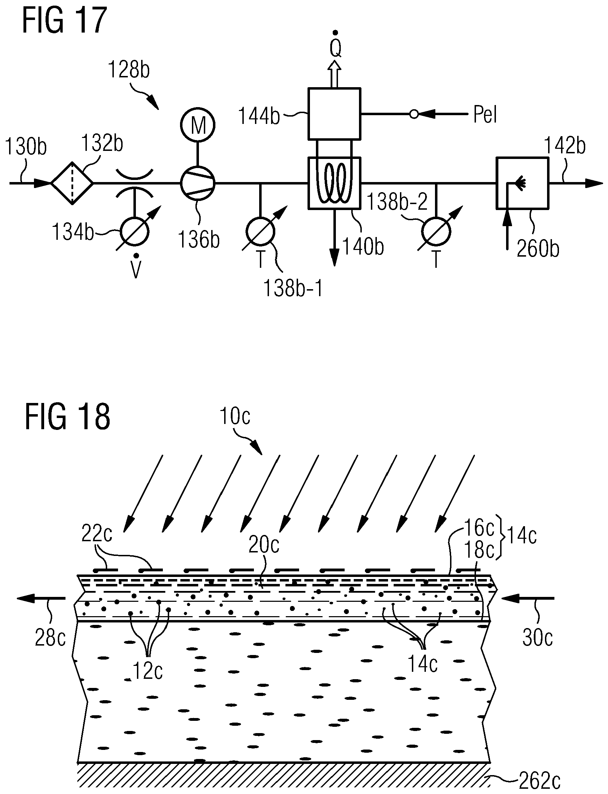

Das Modul 10b' ist mit einer Temperaturführungseinrichtung 128b versehen, welche zur steuerbaren Temperierung des Innenraumes 20b' beiträgt. Der Aufbau dieser Einrichtung 128b ist in Fig. 17 dargestellt.

Fig. 13 zeigt den Aufbau eines CO2-Beseitigungsmoduls 10b'', welches einen der in Fig. 9 dargestellten Innenräume 20b'' ausbildet. Im dargestellten Beispiel handelt es sich um eine "wasserpflanzenbasierte Lösung" mit im Wasser eines Wasserbeckens 18b lebenden Organismen 12b. Zur Integration des Moduls 10b'' sind ein Abgaszufuhranschluss 230b und ein Abgasabfuhranschluss 232b vorgesehen.

Für das wasserbefüllte Becken 18b ist eine Umwälzeinrichtung 26b vorgesehen, beispielsweise wie bereits für das Ausführungsbeispiel gemäß Fig. 1 beschrieben. Eine CO2-Zumischung kann auch an der dargestellten Zumischeinrichtung 38b, 40b erfolgen.

Eine Besonderheit des Moduls 10b'' besteht darin, dass im Innenraum 20b'', überhalb des Wasserspiegels, eine steuerbare Wasserverdüsungseinrichtung 234b zur Erzeugung feiner Wassertröpfchen im Innenraum 20b'' vorgesehen ist.

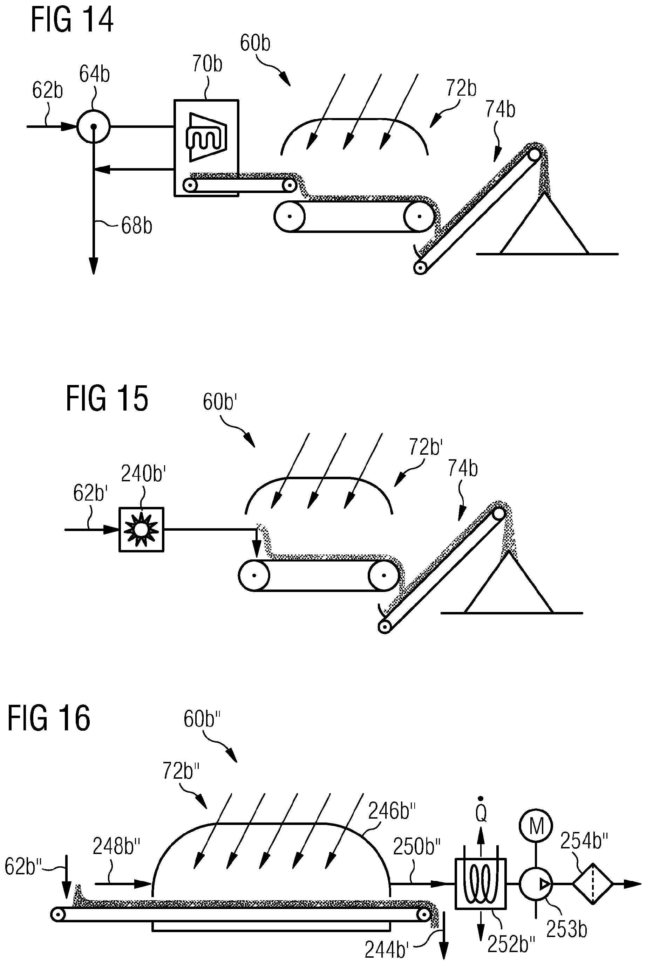

Die Fig. 14, 15 und 16 veranschaulichen drei Ausführungsvarianten einer für die Vorrichtung 10b von Fig. 10 verwendbaren Biomasse-Weiterverarbeitungseinrichtung 60b, 60b' bzw. 60b''.

Fig. 14 zeigt eine insbesondere für Schlämme und stark wasserhaltige Biomasse geeignete Weiterverarbeitungseinrichtung 60b, die in Aufbau und Funktion dem oben mit Bezug auf Fig. 4 beschriebenen Beispiel entspricht.

Fig. 15 zeigt demgegenüber eine insbesondere für feste Biomasse geeignete Weiterverarbeitungseinrichtung 60b', bei welcher die zugeführte Biomasse zunächst über eine Zerkleinerungsstation 240b' und dann über eine Trocknungsstation 72b' (soweit erforderlich) und ein Förderband 74b' geführt wird.

Fig. 16 zeigt schließlich eine Variante mit einer Weiterverarbeitungseinrichtung 60b'' bzw. einem Teil davon, nämlich einer Trocknungsstation 72b'' zur thermischen Trocknung der bei 62b'' zugeführten und bei 244b'' abgeführten Biomasse mittels Sonneneinstrahlung (Pfeile).

Eine Besonderheit der dargestellten Biomasse-Trocknung besteht darin, dass diese in einer (sonnenlichtdurchlässigen) Umhüllung 246b'' durchgeführt wird, um bei der Trocknung anfallendes Wasser sammeln (und in der betreffenden CO2-Beseitigungsvorrichtung weiterverwenden) zu können.

Hierfür wird der Trocknungsstation 72b'' an einem Zufuhranschluss 248b'' Luft zugeführt, welche nach Aufnahme von Wasser aus der Biomasse die Umhüllung 246b'' an einem Luftabgabeanschluss 250b'' wieder verlässt, so dass mittels eines Wärmetauschers 252b'' vorteilhaft Wasser zur Weiterverwendung gewonnen (auskondensiert) werden kann. Die somit getrocknete Luft wird über ein Gebläse 253b'' und einen Filter 254b'' z. B. in die Atmosphäre entlassen (oder in anderer Weise weiterverwendet). Bei dieser Trocknungsvariante unter einer Umhüllung bzw. Abdeckung können vorteilhaft Wasserverluste im Rahmen der Biomasse-Weiterverarbeitung reduziert werden.

Bei einer Biomasse-Verarbeitung, wie beispielhaft anhand der Fig. 14 bis 16 erläutert, kann vorgesehen sein, dass aus einem späteren Verfahrensstadium dieser Verarbeitung entstehende, relativ trockene Biomasse (z. B. Pellets) teilweise in die Biomasse-Verarbeitung zurückgeführt werden, also z. B. in einem bestimmten Anteil der noch wasserhaltigeren Biomasse in einem vorausgegangenen Verfahrensstadium beigemischt wird.

Bei der Trocknung von Biomasse kann insbesondere warmes oder auch abgekühltes Rauchgas aus einem Verbrennungsprozess verwendet werden.

Eine zur Trocknung von Biomasse durch Ausbreitung derselben vorgesehene Trocknungsfläche kann z. B. mindestens 1%, insbesondere 2% der für die Photosynthese genutzten Fläche der Vorrichtung betragen. Maximal beträgt diese Trocknungsfläche bevorzugt 10%, insbesondere 5% dieser Photosynthesefläche.

In einer Ausführungsform ist vorgesehen, dass die Biomasse durch warmes Rauchgas vorgetrocknet wird (z. B. mindestens 1%, insbesondere mindestens 3% der Trocknungsleistung, maximal z. B. 25%, insbesondere 10% der Trocknungsleistung). Die Biomasse wird bevorzugt einer oder mehreren umschlossenen Trocknungseinrichtungen zugeführt, wobei eine Trocknung durch Sonnenlicht und/oder durchströmendes Gas wie z. B. Rauchgas erfolgen kann.

Fig. 17 zeigt die bei dem CO2-Beseitigungsmodul 10b' verwendete Temperaturführungseinrichtung 128b, deren Aufbau und Funktion im Wesentlichen denen der in Fig. 7 dargestellten Einrichtung 128a entsprechen.

Im Unterschied zur Einrichtung 128a (Fig. 7) ist bei der Temperaturführungseinrichtung 128b jedoch ausgangsseitig vor einem Luftauslass 142b noch eine Zumischeinrichtung 260b zum Zumischen von beispielsweise Wasser und/oder anderen Stoffen (z. B. Hilfsstoffe wie Pflanzenschutzmittel etc.) vorgesehen.

Fig. 18 veranschaulicht eine CO2-Beseitigungsvorrichtung 10c gemäß einer weiteren Ausführungsform.

Die Vorrichtung 10c umfasst photosynthesetreibende Organismen 12c, die in einer Umhüllung 14c untergebracht sind.

Die Umhüllung 14c besteht im Wesentlichen aus großflächig sich erstreckenden Folien bzw. Folienanordnungen 16c und 18c, welche einerseits als Überdachung und andererseits als untere Begrenzung des dazwischen befindlichen, von Wasser (enthaltend die Organismen 12c) durchströmten Bereiches 20c dienen. Aufgrund der Transparenz der Überdachung 16c dringt das Sonnenlicht (vgl. Pfeile) in den Innenraum 20c der Vorrichtung 10c ein. Die Pfeile 30c und 28c bezeichnen einen Wasserzufluss bzw. Wasserabfluss, welche beispielsweise mit einer nicht dargestellten Umwälzeinrichtung verbunden sein können, wie dies z. B. bereits bei den weiter oben beschriebenen Ausführungsbeispielen (vgl. z. B. Fig. 1) erläutert wurde.

Unterhalb der Folienanordnung 18c befindet sich ein größeres Wasservolumen (künstlich oder natürlich, Untergrund 262c, gegebenenfalls abgedichtet), welches somit einen Wärmespeicher darstellt, der in unmittelbarer, wärmeübertragender Verbindung mit dem photosynthetisch aktiven, die Organismen 12c enthaltenden Bereich 20c zwischen den Folienanordnungen 16c, 18c steht.

Im dargestellten Ausführungsbeispiel liegt dieser photosynthetisch aktive Bereich im Wesentlichen vollflächig auf dem Wärmespeicher auf ("schwimmend"), so dass vorteilhaft Umgebungstemperaturschwankungen (z. B. im Tageszyklus) erheblich gedämpft werden.

Abweichend von der Darstellung in Fig. 18 könnte auch vorgesehen sein, dass einzelne Bereiche der Umhüllung 14c nicht direkt an das darin enthaltene Organismen-besetzte Wasser angrenzen sondern einen oder mehrere Hohlräume zur Zufuhr von CO2 und/oder Abfuhr von O2 bilden.

Die Wassertiefe im aktiven Bereich kann z. B. im Bereich von 0,01 bis 0,5 m, bevorzugt 0,02 bis 0,3 m liegen. Die Gesamtwassertiefe (aktiver Bereich und Wärmespeicher) kann z. B. im Bereich von 0,2 bis 3 m, bevorzugt 0,4 bis 1,5 m liegen. Insbesondere der Wärmespeicher kann auch eine über seine Fläche betrachtet variierende Wassertiefe aufweisen. Die vorstehend lediglich beispielhaft angegebenen Bereiche gelten dann für die über die Vorrichtungsfläche gemittelten Wassertiefen.

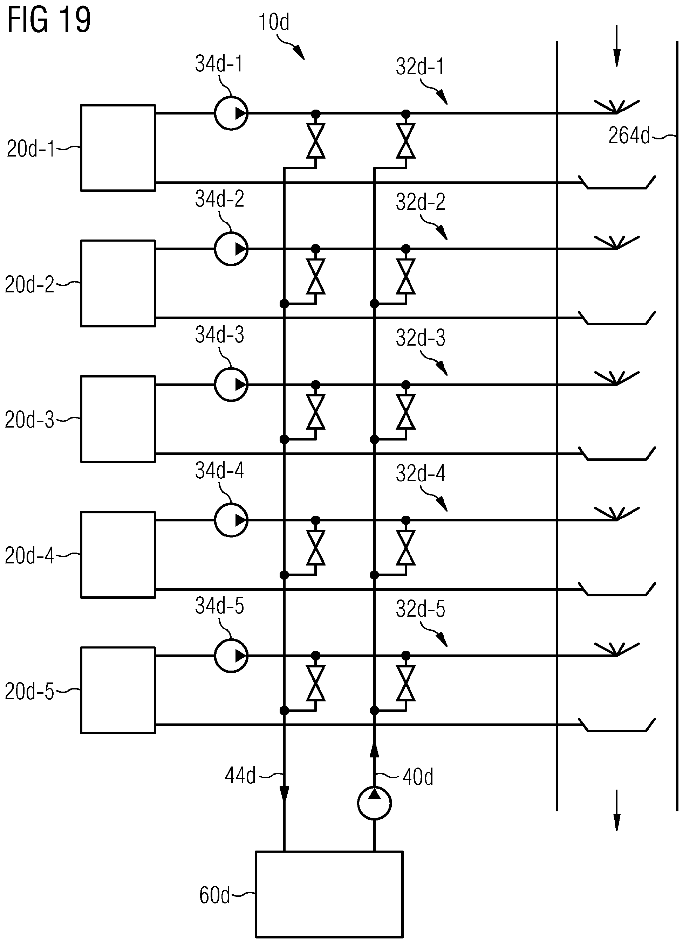

Fig. 19 veranschaulicht eine weitere Ausführungsform einer zur Kombination mit einer Verbrennungsanlage, wie insbesondere einem Kraftwerk, vorgesehenen CO2-Beseitigungsvorrichtung 10d, umfassend eine Anordnung mehrerer als CO2-Senken dienender Vorrichtungsinnenräume 20d-1 bis 20d-5, die jeweils zur photosynthetisch realisierten CO2-Beseitigung dienen und beispielsweise einen Aufbau besitzen, wie er für die oben bereits beschriebenen Ausführungsbeispiele einer "wasserpflanzenbasierten Lösung" erläutert wurde.

Jeder der Innenraumbereiche 20d ist über einen Wasserkreislauf 32d an einen gemeinsamen Gasaustauschkanal 264d angebunden, welcher in Richtung der Pfeile vom Abgas einer Verbrennungsanlage (hier: Kraftwerk) durchströmt wird.

Um im Abgas enthaltenes CO2 den einzelnen Innenraumbereichen 20d zuzuführen, wird mittels einer jeweiligen Pumpe 34d Wasser aus dem jeweiligen Bereich 20d in den Gasaustauschkanal 264d gepumpt und dort verdüst. CO2 aus dem Abgas löst sich sodann in dem verdüsten Wasser, welches wie in der Figur symbolisiert wieder aufgefangen und in den betreffenden Innenraumbereich 20d zurückgeführt wird. Aufgrund der im Verlauf des Gasaustauschkanals aufeinanderfolgenden Anordnung der einzelnen Wasserverdüsungs- und Wasserauffangeinrichtungen ergibt sich in diesem Kanalverlauf eine kontinuierliche Abreicherung von CO2 durch das In-Lösung-Gehen von CO2 im eingedüsten Wasser und eine kontinuierliche Anreicherung von O2 aus den CO2-Senken 20d. Somit entsteht am Ende des Gasaustauschkanals 264d ein O2-angereichertes Gasgemisch.

Vorteilhaft können die einzelnen CO2-Senken bzw. Innenraumbereiche 20d-1 der Vorrichtung 10d somit ihre jeweilige CO2-Beseitigungsaufgabe auf unterschiedlichem CO2-Niveau erfüllen. Die Organismen in den einzelnen photosynthetisch aktiven Bereichen 20d-1 bis 20d-5 können z. B. in ihrer Art und/oder Konzentration der im betreffenden Bereich vorgesehenen CO2-Konzentration optimal angepasst werden.

Im dargestellten Ausführungsbeispiel ist außerdem eine Biomasse-Aufbereitung 60d zur Aufbereitung von Biomasse der einzelnen Wasserkreisläufe 32d vorgesehen, welche über einen Biomasse-Abfuhranschluss 44d, einen Biomasse-Zumischanschluss 40d und eine entsprechende steuerbare Ventilanordnung mit den einzelnen Wasserkreisläufen verbunden ist.

Wenngleich in der schematischen Darstellung von Fig. 19 der Gasaustauschkanal 264d in Vertikalrichtung verlaufend eingezeichnet ist, so ist in der Praxis ein longidudinaler, horizontaler Verlauf, etwa durch im Wesentlichen die gesamte Vorrichtung 10d hindurch, bevorzugt. Wasser-Verdüsungs- und Auffangeinrichtungen sind jedoch übereinander angeordnet, so dass eingedüstes Wasser auch wieder aufgefangen wird.

Der Gasaustauschkanal besitzt bevorzugt über im Wesentlichen seine gesamte Länge einen einheitlichen Innenquerschnitt (optimierter Strömungswiderstand).

Der aus den Wasserkreisläufen abgezweigte Wasseranteil zur Biomasseaufbereitung (60d) kann z. B. mindestens 0,01, insbesondere 0,05 Tonnen H2O pro Kilogramm produzierter Biomasse betragen. Andererseits beträgt der maximale Wasseranteil bevorzugt höchstens 5, insbesondere 1 t H2O pro Kilogramm produzierter Biomasse. Die eingedüste Wassermenge wird bevorzugt so bemessen, dass in Anbetracht der O2/CO2-Konzentrationen und der Löslichkeitskoeffizienten ein ausreichender Stofftransport gewährleistet wird. Typische Menge: mindestens 0,05, insbesondere 0,2 Tonnen H2O pro Kilogramm CO2. Maximal bevorzugt 30, insbesondere 10 Tonnen H2O pro Kilogramm CO2.

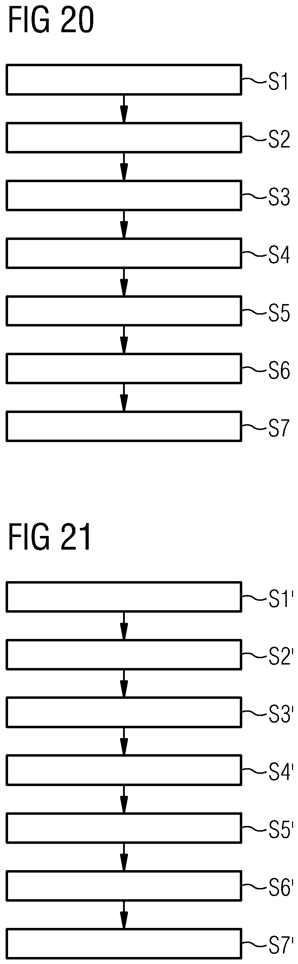

Die beschriebenen CO2-Beseitigungsvorrichtungen und CO2-Beseitigungsverfahren, einschließlich deren Kombination mit einer anthropogenen CO2-Quelle wie z. B. einem Verbrennungskraftwerk, ermöglichen sogar interessante Verfahren bzw. Geschäftsmodelle zur wirtschaftlichen Nutzung der CO2-Beseitigung. Ausführungsbeispiele derartiger Nutzungsverfahren werden nachfolgend mit Bezug auf die Fig. 20 und 21 erläutert.

Fig. 20 ist ein Ablaufdiagramm eines Verfahrens zur wirtschaftlichen Nutzung einer CO2-Beseitigung mittels einer CO2-Beseitigungsvorrichtung.

Das Verfahren beginnt bei einem Schritt S1 mit der Planung und Errichtung der CO2-Beseitigungsvorrichtung.

Sodann werden in diesem Beispiel folgende Schritte durchgeführt:
Schritt S2: Annahme von Aufträgen zur CO2-Beseitigung und/oder Erwerb zur Beseitigung.
Schritt S3: Einbringen von CO2 in die CO2-Beseitigungsvorrichtung, gegebenenfalls nach vorherigem Antransport.
Schritt S4: Betrieb der CO2-Beseitigungsvorrichtung zur Umsetzung von CO2, insbesondere zur Umsetzung von CO2 und Wasser in Sauerstoff und Biomasse.
Schritt S5: Geltendmachung der CO2-Beseitigungsleistung, beispielsweise durch regelmäßige Rechnungslegung.
Schritt S6 (optional): Vermarktung der im Betrieb der CO2-Beseitigungsvorrichtung gewonnenen Biomasse, beispielsweise durch kommerziellen Vertrieb.
Schritt S7 (optional): Optimierung der CO2-Beseitigungsanlage, insbesondere in wirtschaftlicher und/oder technischer und/oder biologischer Hinsicht, bevorzugt in kontinuierlicher Weise.
Fig. 21 zeigt ein Ablaufdiagramm eines Verfahrens, welches dahingehend modifiziert ist, dass der Betrieb der CO2-Vorrichtung zur Beseitigung von unmittelbar aus einem Kraftwerk oder einer Großfeuerungsanlage stammenden CO2 durchgeführt wird.

Dieses Verfahren umfasst folgende Schritte:
Schritt S1': Planung und Errichtung eines Kraftwerkes (insbesondere Großkraftwerk) mit einer daran angekoppelten bzw. damit verbundenen CO2-Beseitigungsvorrichtung.
Schritt S2': Betrieb des Kraftwerkes zur Erzeugung elektrischer Leistung und wenigstens teilweise Einleitung von aus dem Abgas stammenden CO2 in die CO2-Beseitigungsvorrichtung. Schritt S3': Betrieb der CO2-Beseitigungsvorrichtung zur Umsetzung von CO2, insbesondere von CO2 und Wasser in O2 und Biomasse.
Schritt S4' (optional): Rückführung wenigstens eines Teils der erzeugten Biomasse und/oder wenigstens eines Teils des erzeugten Sauerstoffes als Brennstoff bzw. Verbrennungsluft zum Kraftwerk.
Schritt S5': Geltendmachung der Stromerzeugungsleistung, insbesondere durch regelmäßige Rechnungslegung.
Schritt S6' (optional): Vermarktung von gegebenenfalls im Überschuss produzierter Biomasse, beispielsweise durch kommerziellen Vertrieb der Biomasse.
Schritt S7' (optional): Optimierung des Kraftwerkes und/oder der CO2-Beseitigungsvorrichtung, insbesondere in wirtschaftlicher und/oder technischer und/oder biologischer Hinsicht, bevorzugt in kontinuierlicher Weise.

## Patentansprüche

1. Verbrennungsanlage (160), insbesondere Kraftwerk, umfassend
- einen Brennraum (162), insbesondere zur Energieerzeugung durch einen CO2-erzeugenden Verbrennungsprozess, und
- eine CO2-Beseitigungsvorrichtung (10) zur wenigstens teilweisen Beseitigung von CO2 aus dem Abgas des Brennraums (162), umfassend eine Vielzahl photosynthesetreibender Organismen (12), zur Umsetzung von CO2, eine Umhüllung (14) zur Unterbringung der Organismen (12) in einem photosynthetisch aktiven Bereich in einem Innenraum (20) der Umhüllung und zur Aufrechterhaltung vorbestimmter Umgebungsbedingungen für die Organismen (12) im Innenraum (20), Mittel (16, 22) zur Einleitung von Sonnenlicht in den Innenraum (20), eine steuerbare CO2-Zufuhreinrichtung (26, 38) zur gesteuerten Zufuhr von CO2 in den Innenraum, eine steuerbare Produkt-Abfuhreinrichtung (26, 36, 48) zur gesteuerten Abfuhr von Produkten der CO2-Umsetzung,
wobei der Brennraum (162) und der photosynthetisch aktive Bereich in einen Gaskreislauf eingebunden sind, in dem eine Menge von CO2 von dem Brennraum (162) zum photosynthetisch aktiven Bereich und zurück zum Brennraum (162) umläuft,
**dadurch gekennzeichnet, dass** eine Luftzufuhreinrichtung eine Versorgung des Brennraums (162) mit einem zugeführten Gasgemisch vorsieht, welches einen gegenüber der Atmosphäre erhöhten CO2-Gehalt aufweist, um für die Umsetzung von CO2 in der CO2-Beseitigungsvorrichtung (10) eine CO2-Konzentration im Bereich von 5% bis 35% vorzusehen.

2. Verbrennungsanlage (160) nach Anspruch 1, wobei die Luftzufuhreinrichtung steuerbar zur Versorgung des Brennraums (162) mit einem Gasgemisch aus Frischluft und Abluft aus dem photosynthetisch aktiven Bereich vorgesehen ist.

3. Verbrennungsanlage (160) nach einem der Ansprüche 1 bis 2, wobei aus dem Innenraum (20) abgeführte Biomasse und ein dem Verbrennungsprozess zugeführter Brennstoff bzw. Brennstoffanteil Stofferscheinungsformen in einem im Wesentlichen geschlossenen Stoffkreislauf darstellen.

4. Verfahren zum Betreiben einer Verbrennungsanlage (160), insbesondere eines Kraftwerkes (160) zur Energieerzeugung durch einen CO2-erzeugenden Verbrennungsprozess,
umfassend den Schritt einer wenigstens teilweisen Beseitigung von CO2 aus dem Abgas eines Brennraums (162) der Verbrennungsanlage (160) unter Verwendung einer CO2-Beseitigungsvorrichtung (10) umfassend eine Vielzahl photosynthesetreibender Organismen (12), zur Umsetzung von CO2, eine Umhüllung (14) zur Unterbringung der Organismen (12) in einem photosynthetisch aktiven Bereich in einem Innenraum (20) der Umhüllung und zur Aufrechterhaltung vorbestimmter Umgebungsbedingungen für die Organismen (12) im Innenraum (20), Mittel (16, 22) zur Einleitung von Sonnenlicht in den Innenraum (20), eine steuerbare CO2-Zufuhreinrichtung (26, 38) zur gesteuerten Zufuhr von CO2 in den Innenraum, eine steuerbare Produkt-Abfuhreinrichtung (26, 36, 48) zur gesteuerten Abfuhr von Produkten der CO2-Umsetzung,
wobei der Brennraum (162) und der photosynthetisch aktive Bereich in einen Gaskreislauf eingebunden sind, in dem eine Menge von CO2 von dem Brennraum (162) zum photosynthetisch aktiven Bereich und zurück zum Brennraum (162) umläuft,
**dadurch gekennzeichnet, dass** der Brennraum (162) mit einem Gasgemisch versorgt wird, welches einen gegenüber der Atmosphäre erhöhten CO2-Gehalt aufweist, um für die Umsetzung von CO2 in der CO2-Beseitigungsvorrichtung (10) eine CO2-Konzentration im Bereich von 5% bis 35% vorzusehen.

5. Verfahren nach Anspruch 4, umfassend den Schritt einer gesteuerten Versorgung des Brennraumes (162) mit einem Gasgemisch aus Frischluft und Abluft aus dem photosynthetisch aktiven Bereich.

6. Verfahren nach einem der Ansprüche 4 bis 5, wobei Biomasse aus dem Innenraum (20) abgeführt und als Brennstoff bzw. Brennstoffanteil dem Verbrennungsprozess im Brennraum (162) zugeführt wird.

## Claims

1. Incinerator plant (160), particularly power plant, comprising
- a combustion chamber (162), particularly for energy production by means of a CO2-generating incineration process, and
- a CO2 elimination device (10) for the at least partial elimination of CO2 from the waste gas of the combustion chamber (162), comprising a multiplicity of photosynthetic organisms (12), for the conversion of CO2, a shell (14) for the accommodation of the organisms (12) in a photosynthetically active region in an interior (20) of the shell and for the maintenance of predetermined ambient conditions for the organisms (12) in the interior (20), means (16, 22) for the introduction of sunlight into the interior (20), a controllable CO2 feeding mechanism (26, 38) for the controlled feeding of CO2 into the interior, a controllable product removal mechanism (26, 36, 48) for the controlled removal of products of the CO2 conversion,
wherein the combustion chamber (162) and the photosynthetically active region are integrated into a gas loop in which a quantity of CO2 circulates from the combustion chamber (162) to the photosynthetically active region and back to the combustion chamber (162),
**characterized in that** an air feeding mechanism provides for supply of the combustion chamber (162) with a fed gas mixture comprising a CO2 content elevated with respect to the atmosphere in order to provide a CO2 concentration in the range from 5% to 35% for the conversion of CO2 in the CO2 elimination device (10).

2. Incinerator plant (160) according to Claim 1, wherein the air feeding mechanism is provided in a controllable manner for the supply of the combustion chamber (162) with a gas mixture composed of fresh air and waste air from the photosynthetically active region.

3. Incinerator plant (160) according to either of Claims 1 and 2, wherein biomass removed from the interior (20) and a fuel or fuel fraction fed to the incineration process are the outward appearances of material in a substantially closed material loop.

4. Method for operating an incinerator plant (160), particularly a power plant (160) for energy production by means of a CO2-generating incineration process,
comprising the step of an at least partial elimination of CO2 from the waste gas of a combustion chamber (162) of the incinerator plant (160) using a CO2 elimination device (10) comprising a multiplicity of photosynthetic organisms (12), for the conversion of CO2, a shell (14) for the accommodation of the organisms (12) in a photosynthetically active region in an interior (20) of the shell and for the maintenance of predetermined ambient conditions for the organisms (12) in the interior (20), means (16, 22) for the introduction of sunlight into the interior (20), a controllable CO2 feeding mechanism (26, 38) for the controlled feeding of CO2 into the interior, a controllable product removal mechanism (26, 36, 48) for the controlled removal of products of the CO2 conversion,
wherein the combustion chamber (162) and the photosynthetically active region are integrated into a gas loop in which a quantity of CO2 circulates from the combustion chamber (162) to the photosynthetically active region and back to the combustion chamber (162),
**characterized in that** the combustion chamber (162) is supplied with a gas mixture comprising a CO2 content elevated with respect to the atmosphere in order to provide a CO2 concentration in the range from 5% to 35% for the conversion of CO2 in the CO2 elimination device (10).

5. Method according to Claim 4, comprising the step of a controlled supply of the combustion chamber (162) with a gas mixture composed of fresh air and waste air from the photosynthetically active region.

6. Method according to either of Claims 4 and 5, wherein biomass is removed from the interior (20) and is fed as fuel or fuel fraction to the incineration process in the combustion chamber (162).

## Revendications

1. Usine d'incinération (160), en particulier centrale électrique, comprenant :
- une chambre de combustion (162), en particulier pour la production d'énergie via un processus de combustion générant du CO₂ ; et
- un dispositif d'élimination du CO₂ (10) pour l'élimination au moins partielle du CO₂ à partir des gaz d'échappement de la chambre de combustion (162), comprenant une multitude d'organismes (12) entraînant une photosynthèse, pour la conversion du CO₂, une enceinte (14) pour placer les organismes (12) dans une zone active du point de vue de la photosynthèse dans un espace interne (20) de l'enceinte et pour le maintien de conditions ambiantes prédéfinies pour les organismes (12) dans l'espace interne (20), des moyens (16, 22) pour laisser entrer la lumière du soleil dans l'espace interne (20), un mécanisme d'alimentation en CO₂ (26, 38) qui peut être commandé pour l'alimentation commandée de CO₂ dans l'espace interne, un mécanisme d'évacuation de produit (26, 36, 48) qui peut être commandé pour l'évacuation commandée des produits de la conversion du CO₂ ;
dans laquelle la chambre de combustion (162) et la zone active du point de vue de la photosynthèse sont intégrées dans un circuit de gaz, dans lequel circule une quantité de CO₂ depuis la chambre de combustion (162) jusqu'à la zone active du point de vue de la photosynthèse et en retour en direction de la chambre de combustion (162) ; **caractérisée en ce qu'**un mécanisme d'alimentation d'air fournit une alimentation de la chambre de combustion (162) avec un mélange de gaz alimenté qui présente une teneur en CO₂ supérieure par rapport à celle de l'atmosphère, dans le but de fournir, pour la conversion du CO₂ dans le dispositif d'élimination du CO₂ (10), une concentration de CO₂ dans la plage de 5 % à 35 %.

2. Usine d'incinération (160) selon la revendication 1, dans laquelle le mécanisme d'alimentation d'air est prévu pour être commandé à des fins d'alimentation de la chambre de combustion (162) avec un mélange gazeux d'air frais et d'air évacué à partir de la zone active du point de vue de la photosynthèse.

3. Usine d'incinération (160) selon l'une quelconque des revendications 1 à 2, dans lequel, de la biomasse évacuée de l'espace interne (20) et un combustible, respectivement une fraction de combustible alimenté au processus de combustion représentent des formes de substances dans un circuit de substances essentiellement fermé.

4. Procédé pour l'exploitation d'une usine d'incinération (160), en particulier d'une centrale électrique (160) pour la production d'énergie via un processus de combustion générant du CO₂,
comprenant l'étape dans laquelle on procède à une élimination au moins partielle du CO₂ à partir des gaz d'échappement d'une chambre de combustion (162) de l'usine d'incinération (160) en utilisant un dispositif d'élimination du CO₂ (10) comprenant une multitude d'organismes (12) entraînant une photosynthèse, pour la conversion du CO₂, une enceinte (14) pour placer les organismes (12) dans une zone active du point de vue de la photosynthèse, dans un espace interne (20) de l'enceinte et pour le maintien de conditions ambiantes prédéfinies pour les organismes (12) dans l'espace interne (20), des moyens (16, 22) pour laisser entrer la lumière du soleil dans l'espace interne (20), un mécanisme d'alimentation en CO₂ (26, 38) qui peut être commandé pour l'alimentation commandée de CO₂ dans l'espace interne, un mécanisme d'évacuation de produit (26, 36, 48) qui peut être commandé pour l'évacuation commandée des produits de la conversion du CO₂ ;
dans lequel la chambre de combustion (162) et la zone active du point de vue de la photosynthèse sont intégrées dans un circuit de gaz, dans lequel circule une quantité de CO₂ depuis la chambre de combustion (162) jusqu'à la zone active du point de vue de la photosynthèse et en retour en direction de la chambre de combustion (162) ; **caractérisé en ce que** la chambre de combustion (162) est alimentée avec un mélange de gaz qui présente une teneur en CO₂ supérieure par rapport à celle de l'atmosphère, dans le but de fournir, pour la conversion du CO₂ dans le dispositif d'élimination du CO₂ (10) une concentration de CO₂ dans la plage de 5 % à 35 %.

5. Procédé selon la revendication 4, comprenant l'étape dans laquelle on procède à une alimentation commandée de la chambre de combustion (162) avec un mélange gazeux d'air frais et d'air évacué à partir de la zone active du point de vue de la photosynthèse.

6. Procédé selon l'une quelconque des revendications 4 à 5, dans lequel de la biomasse est évacuée hors de l'espace interne (20) et est alimentée à titre de combustible, respectivement de fraction de combustible au processus de combustion dans la chambre de combustion (162).
